# EUROPEAN PATENT APPLICATION

(11) **EP 4 691 365 A2**
(43) Date of publication of application: **11.02.2026**
(21) Application number: 25223283.0
(22) Date of filing: 29.12.2022
(51) Int. Cl.: A61B 5/145

(54) **METHODS AND SYSTEMS FOR SENSING A PLURALITY OF ANALYTES**

(30) Priority: 30.12.2021 US 202163295120 P
(62) Divisional of application: 22862328.6
(71) Applicant: Abbott Diabetes Care Inc., Alameda, California 94502 (US)
(72) Inventor: DALTON, Scott D., Berkeley, CA 94708 (US); KUNICH, Theodore J., Pleasanton, CA 94588 (US); CHOW, Eric, San Francisco, CA 94127 (US)
(74) Representative: Mathys & Squire

(57) **Abstract**

A device includes a multiple analyte sensor, a transimpedance amplifier, and a differential amplifier. The multiple analyte sensor includes a first working electrode, a second working electrode, a counter electrode, and a reference electrode. Each of the first working electrode and the second working electrode is configured to receive a signal indicative of a presence of a respective analyte. The counter electrode is a sum of the received signal of each of the first working electrode and the second working electrode. The transimpedance amplifier is configured to receive a first signal of the received signals from the first working electrode and a second signal of the received signals from the second working electrode. The transimpedance amplifier converts the received first signal and the received second signal to an output including a variable bias offset. The differential amplifier is configured to subtract the variable bias offset from the output.

## Description

### BACKGROUND

The disclosed subject matter relates to systems and methods for sensing a plurality of analytes, for example sensors for monitoring levels of analytes.

While some of these analyte sensors are equipped with powerful processors and operate using a permanent power supply, other analyte sensors are designed to operate efficiently, using little power. Active analyte sensors, including those used in monitoring levels of analytes, are one example of such analyte sensors. An analyte sensor can include sensing hardware to detect raw values of signals that have been determined to correlate with levels of an analyte. The analyte sensor can also be configured to perform on-device processing, for example applying algorithms and calibration parameters to the signals, to convert the raw values to levels of the analyte useful for monitoring the condition of the patient or for diagnosis and treatment. Analyte sensors can further determine patterns or trends in the levels of analytes in the patient. Such processed information can be provided to the patient or other interested parties for review. To increase efficiency, components of the analyte sensor can be dedicated to particular functions of the processing, but increased specialization of the components can also increase the cost of the components used in the analyte sensor.

At least in part because analyte sensors can be worn by a patient, attached, for example to the patient's skin, the analyte sensor can be designed to operate for extended periods of time using an internal power source. When the internal source is depleted, the analyte sensor can be discarded. Accordingly, there is an opportunity for systems and methods which can be embodied in and implemented by low-power analyte sensors to increase the computational- and power-efficiency of the devices while reducing the cost of production of the analyte sensors and maintaining the ability of the analyte sensors to perform expected functions, such as secure wireless communication, simple application and activation, and compatibility with a variety of receiving devices.

### SUMMARY

The purpose and advantages of the disclosed subject matter will be set forth in and apparent from the description that follows, as well as will be learned by practice of the disclosed subject matter. Additional advantages of the disclosed subject matter will be realized and attained by the methods and systems particularly pointed out in the written description and claims hereof, as well as from the drawings.

To achieve these and other advantages and in accordance with the purpose of the disclosed subject matter, as embodied and broadly described, the disclosed subject matter includes an embedded system used by an analyte sensor and methods of operation thereof.

In accordance with the disclosed subject matter, for purpose of illustration and not limitation, a device can include a multiple analyte sensor, a transimpedance amplifier, and a differential amplifier, and a processor. The device can be embodied as analyte sensing hardware. The multiple analyte sensor may be embodied as a dual analyte sensor. The multiple analyte sensor can include a first working electrode, a second working electrode, a counter electrode, and a reference electrode. Each of the first working electrode and the second working electrode can be configured to receive a signal indicative of a presence of a respective analyte. The analyte can include one or more of ketone, glucose, or lactate. The counter electrode can be a sum of the received signal of each of the first working electrode and the second working electrode. The transimpedance amplifier can be configured to receive a first signal of the received signals from the first working electrode and a second signal of the received signals from the second working electrode. The transimpedance amplifier can convert the received first signal and the received second signal to an output including a variable bias offset. The received signals can be current signals. The transimpedance amplifier can be configured to convert the first current signal and the second current signal into an output voltage. The differential amplifier can be configured to subtract the variable bias offset from the output to generate a modified output including a variable residual. The processor can be configured to generate data indicative of an analyte value from the modified output. The processor can use a calibration function configured to adjust the generated data based on the variable residual. The calibration function can be configured to adjust the generated data to remove the variable residual. The variable bias offset can be determined based on a first analyte measured by the first working electrode or a second analyte measured by the second working electrode. The differential amplifier can be further configured to calibrate out a bias dependent residual and a common-mode op-amp characteristic from the output to generate the modified output. The bias dependent residual can be determined based on a two-point offset calibration for one of the first working electrode or the second working electrode. The common-mode op-amp characteristic can be determined based on a three-point calibration.

According to aspects of the disclosed subject matter, the device can further include an analog to digital converter, a communication module, and a negative poise bias amplifier. The analog to digital converter can be configured to convert the modified output to a digital output. The subtraction of the bias offset from the output can reduce the modified output into a range of the analog to digital converter. The communication module can be configured to process the digital output into measurement results. The communication module can be separate from the device. The communication module can be further configured to provide the measurement results to a receiving device for display via wireless communication. The negative poise bias amplifier can be configured to subtract a predetermined voltage from the reference electrode to generate a first working electrode bias to bias the first working electrode. The predetermined voltage can be determined based on a first analyte measured by the first working electrode or a second analyte measured by the second working electrode. The negative poise bias amplifier can be further configured to receive a reference signal from the reference electrode and a negative poise reference. The device can be configured to detect blood ketone levels, blood glucose levels, blood lactate levels, or other analyte levels.

In accordance with the disclosed subject matter, for purpose of illustration and not limitation, a device can include a multiple analyte sensor and an application specific integrated circuit. The device may be embodied as analyte sensing hardware. The multiple analyte sensor may be embodied as a dual analyte sensor. The multiple analyte sensor can include a first working electrode, a second working electrode, a counter electrode, and a reference electrode. Each of the first working electrode and the second working electrode can be configured to receive a signal indicative of a presence of a respective analyte. The application specific integrated circuit can be configured to receive a first signal of the received signals from the first working electrode and a second signal of the received signals from the second working electrode. The application specific integrated circuit can set a first independent bias voltage for the first working electrode and a second independent bias voltage for the second working electrode. The application specific integrated circuit can generate data indicative of an analyte using the first independent bias voltage and the second independent bias voltage. The application specific integrated circuit can be configured to sense one or more signals below a threshold current specific to a particular analyte.

In accordance with the disclosed subject matter, for purpose of illustration and not limitation, a device can include a multiple analyte sensor, a first application specific integrated circuit, a second application specific integrated circuit, and a serial-peripheral interface (SPI) interface. The device can be embodied as analyte sensing hardware. The multiple analyte sensor may be embodied as a dual analyte sensor. The multiple analyte sensor can include a first working electrode, a second working electrode, a counter electrode, and a reference electrode. Each of the first working electrode and the second working electrode can be configured to receive a signal indicative of a presence of a respective analyte. The first application specific integrated circuit can be configured to receive a first signal of the received signals from the first working electrode. The first application specific integrated circuit can set a first independent bias voltage for the first working electrode. The first application specific integrated circuit can further be configured to generate data indicative of a first analyte using the first independent bias voltage. The second application specific integrated circuit can be configured to receive a second signal of the received signals from the second working electrode. The second application specific integrated circuit can set a second independent bias voltage for the second working electrode. The second application specific integrated circuit can further be configured to generate data indicative of a second analyte using the second independent bias voltage. The SPI interface can be configured to individually receive communication from either of the first application specific integrated circuit or the second application specific integrated circuit. The SPI interface can include one or more communication lines that are shared between the first application specific integrated circuit and the second application specific integrated circuit.

The accompanying drawings, which are incorporated in and constitute part of this specification, are included to illustrate and provide a further understanding of the methods and systems of the disclosed subject matter. Together with the description, the drawings explain the principles of the disclosed subject matter.

### BRIEF DESCRIPTION OF THE DRAWINGS

The details of the subject matter set forth herein, both as to its structure and operation, may be apparent by study of the accompanying figures, in which like reference numerals refer to like parts.
FIG. 1A is a system overview of a sensor applicator, reader device, monitoring system, network, and remote system.
FIG. 1B is a diagram illustrating an operating environment of an example analyte monitoring system for use with the techniques described herein.
FIG. 2A is a block diagram depicting an example embodiment of a reader device.
FIG. 2B is a block diagram illustrating an example data receiving device for communicating with the sensor according to exemplary embodiments of the disclosed subject matter.
FIGS. 2C and 2D are block diagrams depicting example embodiments of sensor control devices.
FIG. 2E is a block diagram illustrating an example analyte sensor according to exemplary embodiments of the disclosed subject matter.
FIG. 3A is a proximal perspective view depicting an example embodiment of a user preparing a tray for an assembly.
FIG. 3B is a side view depicting an example embodiment of a user preparing an applicator device for an assembly.
FIG. 3C is a proximal perspective view depicting an example embodiment of a user inserting an applicator device into a tray during an assembly.
FIG. 3D is a proximal perspective view depicting an example embodiment of a user removing an applicator device from a tray during an assembly.
FIG. 3E is a proximal perspective view depicting an example embodiment of a patient applying a sensor using an applicator device.
FIG. 3F is a proximal perspective view depicting an example embodiment of a patient with an applied sensor and a used applicator device.
FIG. 4A is a side view depicting an example embodiment of an applicator device coupled with a cap.
FIG. 4B is a side perspective view depicting an example embodiment of an applicator device and cap decoupled.
FIG. 4C is a perspective view depicting an example embodiment of a distal end of an applicator device and electronics housing.
FIG. 4D is a top perspective view of an exemplary applicator device in accordance with the disclosed subject matter.
FIG. 4E is a bottom perspective view of the applicator device of FIG. 4D.
FIG. 4F is an exploded view of the applicator device of FIG. 4D.
FIG. 4G is a side cutaway view of the applicator device of FIG. 4D.
FIG. 5 is a proximal perspective view depicting an example embodiment of a tray with sterilization lid coupled.
FIG. 6A is a proximal perspective cutaway view depicting an example embodiment of a tray with sensor delivery components.
FIG. 6B is a proximal perspective view depicting sensor delivery components.
FIGS. 7A and 7B are isometric exploded top and bottom views, respectively, of an exemplary sensor control device.
FIG. 8A-8C are assembly and cross-sectional views of an on-body device including an integrated connector for the sensor assembly.
FIGS. 9A and 9B are side and cross-sectional side views, respectively, of an example embodiment of the sensor applicator of FIG. 1A with the cap of FIG. 2C coupled thereto.
FIGS. 10A and 10B are isometric and side views, respectively, of another example sensor control device.
FIGS. 11A-11C are progressive cross-sectional side views showing assembly of the sensor applicator with the sensor control device of FIGS. 10A-10B.
FIGS. 12A-12C are progressive cross-sectional side views showing assembly and disassembly of an example embodiment of the sensor applicator with the sensor control device of FIGS. 10A-10B.
FIGS. 13A-13F illustrate cross-sectional views depicting an example embodiment of an applicator during a stage of deployment.
FIG. 14 is a graph depicting an example of an in vitro sensitivity of an analyte sensor.
FIG. 15 is a diagram illustrating example operational states of the sensor according to exemplary embodiments of the disclosed subject matter.
FIG. 16 is a diagram illustrating an example operational and data flow for over-the-air programming of a sensor according to the disclosed subject matter.
FIG. 17 is a diagram illustrating an example data flow for secure exchange of data between two devices according to the disclosed subject matter.
FIG. 18 is a diagram illustrating an example sensor and a receiver for communication with the example sensor according to exemplary embodiments of the disclosed subject matter.
FIG. 19 is a diagram illustrating functional blocks of the sensor according to exemplary embodiments of the disclosed subject matter.
FIG. 20 is a diagram illustrating an example interface between components of the sensor according to exemplary embodiments of the disclosed subject matter.
FIG. 21 is a diagram illustrating example functionality of a measurement along a thermistor of the sensor according to exemplary embodiments of the disclosed subject matter.
FIG. 22 is a diagram illustrating example functionality of an analog front end of the sensor according to exemplary embodiments of the disclosed subject matter.
FIG. 23 is a diagram of an example circuit for reading values for an analyte sensor.
FIG. 24 is a diagram of an example circuit for reading values for an analyte sensor.
FIG. 25 is a diagram of an example circuit for a variable floating poise voltage generator.
FIG. 26 is a diagram of an example circuit with a sensor with a floating variable poise voltage circuit in a measurement configuration.
FIG. 27 is a diagram of an example circuit with a sensor with a floating variable poise voltage circuit in a calibration configuration.
FIGS. 28A-28B are a diagram of an example circuit with a multiple analyte sensor.
FIGS. 29A-29B are a diagram of an example circuit with a multiple analyte sensor.
FIG. 30 is a diagram of an example circuit with a multiple analyte sensor.
FIG. 31 is a diagram illustrating an exemplary voltage characteristic of operational amplifiers for use with the disclosed subject matter.
FIGS. 32A-32B are a diagram of an example circuit with a multiple analyte sensor.
FIG. 33 is a diagram of an example circuit with a multiple analyte sensor.
FIG. 34 is a diagram of an example circuit with a multiple analyte sensor.

### DETAILED DESCRIPTION

Before the present subject matter is described in detail, it is to be understood that this disclosure is not limited to the particular embodiments described, as such may, of course, vary. It is also to be understood that the terminology used herein is for the purpose of describing particular embodiments only, and is not intended to be limiting, since the scope of the present disclosure will be limited only by the appended claims.

As used herein and in the appended claims, the singular forms "a," "an," and "the" include plural referents unless the context clearly dictates otherwise.

The publications discussed herein are provided solely for their disclosure prior to the filing date of the present application. Nothing herein is to be construed as an admission that the present disclosure is not entitled to antedate such publication by virtue of prior disclosure. Further, the dates of publication provided may be different from the actual publication dates which may need to be independently confirmed.

Generally, embodiments of the present disclosure include systems, devices, and methods for the use of analyte sensor insertion applicators for use with in vivo analyte monitoring systems. An applicator can be provided to the user in a sterile package with an electronics housing of the sensor control device contained therein. According to some embodiments, a structure separate from the applicator, such as a container, can also be provided to the user as a sterile package with a sensor module and a sharp module contained therein. The user can couple the sensor module to the electronics housing, and can couple the sharp to the applicator with an assembly process that involves the insertion of the applicator into the container in a specified manner. In other embodiments, the applicator, sensor control device, sensor module, and sharp module can be provided in a single package. The applicator can be used to position the sensor control device on a human body with a sensor in contact with the wearer's bodily fluid. The embodiments provided herein are improvements to reduce the likelihood that a sensor is improperly inserted or damaged, or elicits an adverse physiological response. Other improvements and advantages are provided as well. The various configurations of these devices are described in detail by way of the embodiments which are only examples.

Furthermore, many embodiments include in vivo analyte sensors structurally configured so that at least a portion of the sensor is, or can be, positioned in the body of a user to obtain information about at least one analyte of the body. It should be noted, however, that the embodiments disclosed herein can be used with in vivo analyte monitoring systems that incorporate in vitro capability, as well as purely in vitro or ex vivo analyte monitoring systems, including systems that are entirely non-invasive.

Furthermore, for each and every embodiment of a method disclosed herein, systems and devices capable of performing each of those embodiments are covered within the scope of the present disclosure. For example, embodiments of sensor control devices are disclosed and these devices can have one or more sensors, analyte monitoring circuits (e.g., an analog circuit), memories (e.g., for storing instructions), power sources, communication circuits, transmitters, receivers, processors and/or controllers (e.g., for executing instructions) that can perform any and all method steps or facilitate the execution of any and all method steps. These sensor control device embodiments can be used and can be capable of use to implement those steps performed by a sensor control device from any and all of the methods described herein.

Furthermore, the systems and methods presented herein can be used for operations of a sensor used in an analyte monitoring system, such as but not limited to wellness, fitness, dietary, research, information or any purposes involving analyte sensing over time. As used herein, "analyte sensor" or "sensor" can refer to any device capable of receiving sensor information from a user, including for purpose of illustration but not limited to, body temperature sensors, blood pressure sensors, pulse or heart-rate sensors, glucose level sensors, analyte sensors, physical activity sensors, body movement sensors, or any other sensors for collecting physical or biological information. Analytes measured by the analyte sensors can include, by way of example and not limitation, glucose, ketones, lactate, oxygen, hemoglobin A1C, albumin, alcohol, alkaline phosphatase, alanine transaminase, aspartate aminotransferase, bilirubin, blood urea nitrogen, calcium, carbon dioxide, chloride, creatinine, hematocrit, lactate, magnesium, oxygen, pH, phosphorus, potassium, sodium, total protein, uric acid, etc.

As mentioned, a number of embodiments of systems, devices, and methods are described herein that provide for the improved assembly and use of dermal sensor insertion devices for use with in vivo analyte monitoring systems. In particular, several embodiments of the present disclosure are designed to improve the method of sensor insertion with respect to in vivo analyte monitoring systems and, in particular, to prevent the premature retraction of an insertion sharp during a sensor insertion process. Some embodiments, for example, include a dermal sensor insertion mechanism with an increased firing velocity and a delayed sharp retraction. In other embodiments, the sharp retraction mechanism can be motion-actuated such that the sharp is not retracted until the user pulls the applicator away from the skin. Consequently, these embodiments can reduce the likelihood of prematurely withdrawing an insertion sharp during a sensor insertion process; decrease the likelihood of improper sensor insertion; and decrease the likelihood of damaging a sensor during the sensor insertion process, to name a few advantages. Several embodiments of the present disclosure also provide for improved insertion sharp modules to account for the small scale of dermal sensors and the relatively shallow insertion path present in a subject's dermal layer. In addition, several embodiments of the present disclosure are designed to prevent undesirable axial and/or rotational movement of applicator components during sensor insertion. Accordingly, these embodiments can reduce the likelihood of instability of a positioned dermal sensor, irritation at the insertion site, damage to surrounding tissue, and breakage of capillary blood vessels resulting in fouling of the dermal fluid with blood, to name a few advantages. In addition, to mitigate inaccurate sensor readings which can be caused by trauma at the insertion site, several embodiments of the present disclosure can reduce the end-depth penetration of the needle relative to the sensor tip during insertion.

Before describing these aspects of the embodiments in detail, however, it is first desirable to describe examples of devices that can be present within, for example, an in vivo analyte monitoring system, as well as examples of their operation, all of which can be used with the embodiments described herein.

There are various types of in vivo analyte monitoring systems. "Continuous Analyte Monitoring" systems (or "Continuous Glucose Monitoring" systems), for example, can transmit data from a sensor control device to a reader device continuously without prompting, e.g., automatically according to a schedule. "Flash Analyte Monitoring" systems (or "Flash Glucose Monitoring" systems or simply "Flash" systems), as another example, can transfer data from a sensor control device in response to a scan or request for data by a reader device, such as with a Near Field Communication (NFC) or Radio Frequency Identification (RFID) protocol. In vivo analyte monitoring systems can also operate without the need for finger stick calibration.

In vivo analyte monitoring systems can be differentiated from "in vitro" systems that contact a biological sample outside of the body (or "ex vivo") and that typically include a meter device that has a port for receiving an analyte test strip carrying bodily fluid of the user, which can be analyzed to determine the user's blood sugar level.

In vivo monitoring systems can include a sensor that, while positioned in vivo, makes contact with the bodily fluid of the user and senses the analyte levels contained therein. The sensor can be part of the sensor control device that resides on the body of the user and contains the electronics and power supply that enable and control the analyte sensing. The sensor control device, and variations thereof, can also be referred to as a "sensor control unit," an "on-body electronics" device or unit, an "on-body" device or unit, or a "sensor data communication" device or unit, to name a few.

In vivo monitoring systems can also include a device that receives sensed analyte data from the sensor control device and processes and/or displays that sensed analyte data, in any number of forms, to the user. This device, and variations thereof, can be referred to as a "handheld reader device," "reader device" (or simply a "reader"), "handheld electronics" (or simply a "handheld"), a "portable data processing" device or unit, a "data receiver," a "receiver" device or unit (or simply a "receiver"), or a "remote" device or unit, to name a few. Other devices such as personal computers have also been utilized with or incorporated into in vivo and in vitro monitoring systems.

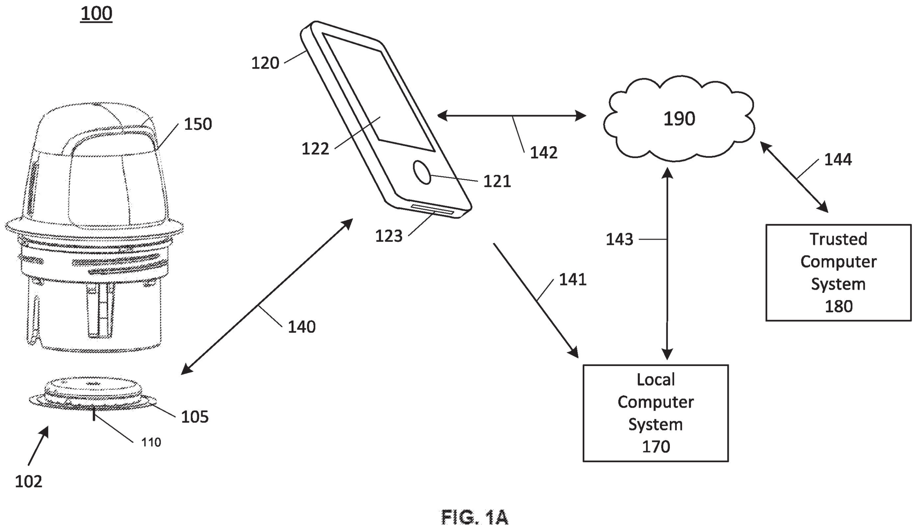

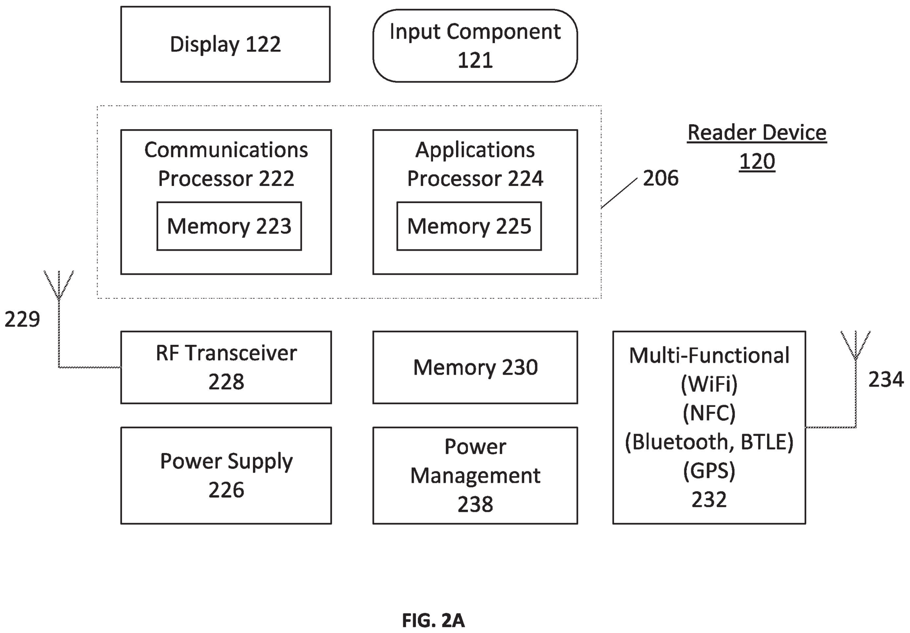

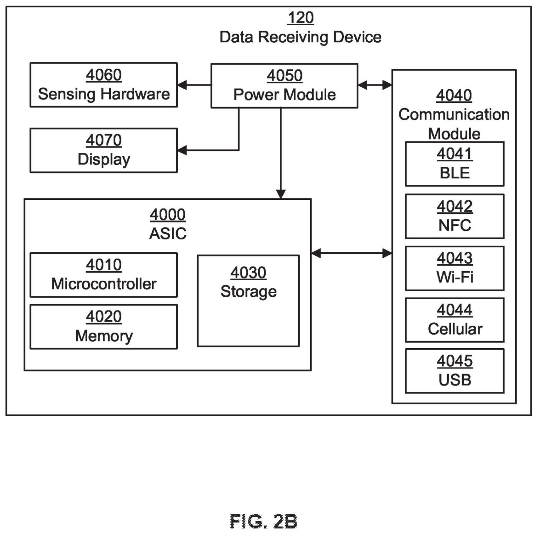

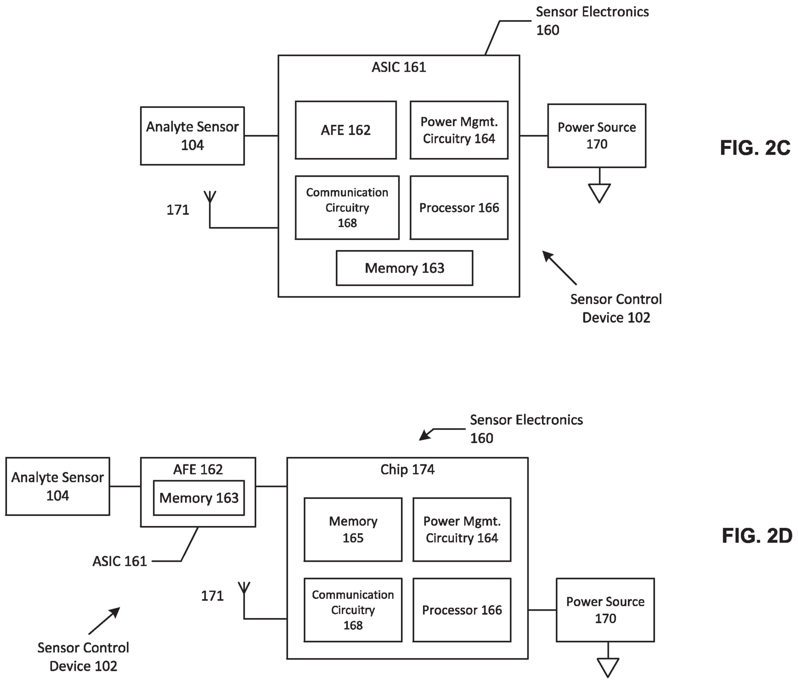

FIG. 1A is a conceptual diagram depicting an example embodiment of an analyte monitoring system 100 that includes a sensor applicator 150, a sensor control device 102, and a reader device 120. Here, sensor applicator 150 can be used to deliver sensor control device 102 to a monitoring location on a user's skin where a sensor 110 is maintained in position for a period of time by an adhesive patch 105. Sensor control device 102 is further described in FIGS. 2B and 2C, and can communicate with reader device 120 via a communication path 144 using a wired or wireless technique. Example wireless protocols include Bluetooth, Bluetooth Low Energy (BLE, BTLE, Bluetooth SMART, etc.), Near Field Communication (NFC) and others. Users can monitor applications installed in memory on reader device 120 using screen 122 and input 121 and the device battery can be recharged using power port 123. More detail about reader device 120 is set forth with respect to FIG. 2A below. Reader device 120 can communicate with local computer system 170 via a communication path 141 using a wired or wireless technique. Local computer system 170 can include one or more of a laptop, desktop, tablet, phablet, smartphone, set-top box, video game console, or other computing device and wireless communication can include any of a number of applicable wireless networking protocols including Bluetooth, Bluetooth Low Energy (BTLE), Wi-Fi or others. Local computer system 170 can communicate via communications path 143 with a network 190 similar to how reader device 120 can communicate via a communications path 142 with network 190, by wired or wireless technique as described previously. Network 190 can be any of a number of networks, such as private networks and public networks, local area or wide area networks, and so forth. A trusted computer system 180 can include a server and can provide authentication services and secured data storage and can communicate via communications path 144 with network 190 by wired or wireless technique.

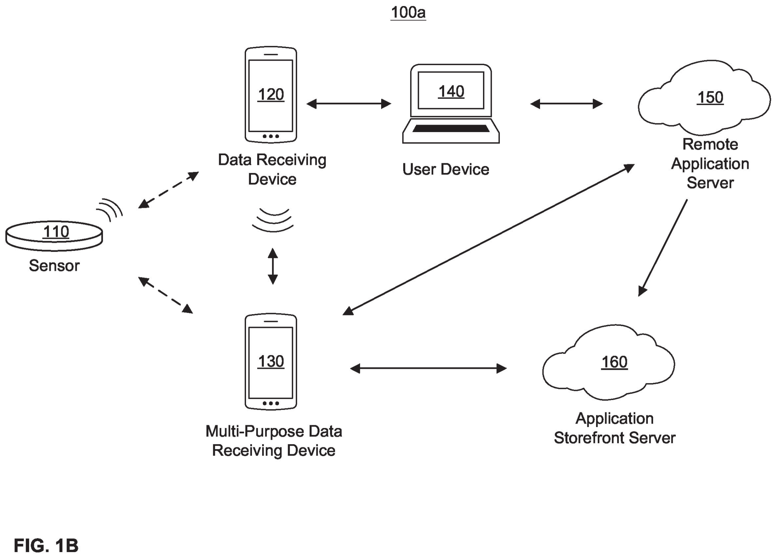

FIG. 1B illustrates an operating environment of an analyte monitoring system 100a capable of embodying the techniques described herein. The analyte monitoring system 100a can include a system of components designed to provide monitoring of parameters, such as analyte levels, of a human or animal body or can provide for other operations based on the configurations of the various components. As embodied herein, the system can include a low-power analyte sensor 110, or simply "sensor" worn by the user or attached to the body for which information is being collected. As embodied herein, the analyte sensor 110 can be a sealed, disposable device with a predetermined active use lifetime (e.g., 1 day, 14 days, 30 days, etc.). Sensors 110 can be applied to the skin of the user body and remain adhered over the duration of the sensor lifetime or can be designed to be selectively removed and remain functional when reapplied. The low-power analyte monitoring system 100a can further include a data reading device 120 or multi-purpose data receiving device 130 configured as described herein to facilitate retrieval and delivery of data, including analyte data, from the analyte sensor 110.

As embodied herein, the analyte monitoring system 100a can include a software or firmware library or application provided, for example via a remote application server 150 or application storefront server 160, to a third-party and incorporated into a multi-purpose hardware device 130 such as a mobile phone, tablet, personal computing device, or other similar computing device capable of communicating with the analyte sensor 110 over a communication link. Multi-purpose hardware can further include embedded devices, including, but not limited to insulin pumps or insulin pens, having an embedded library configured to communicate with the analyte sensor 110. Although the illustrated embodiments of the analyte monitoring system 100a include only one of each of the illustrated devices, this disclosure contemplates the analyte monitoring system 100a incorporate multiples of each components interacting throughout the system. For example and without limitation, as embodied herein, data reading device 120 and/or multi-purpose data receiving device 130 can include multiples of each. As embodied herein, multiple data receiving devices 130 can communicate directly with sensor 110 as described herein. Additionally or alternatively, a data receiving device 130 can communicate with secondary data receiving devices 130 to provide analyte data, or visualization or analysis of the data, for secondary display to the user or other authorized parties.

FIG. 2A is a block diagram depicting an example embodiment of a reader device configured as a smartphone. Here, reader device 120 can include a display 122, input component 121, and a processing core 206 including a communications processor 222 coupled with memory 223 and an applications processor 224 coupled with memory 225. Also included can be separate memory 230, RF transceiver 228 with antenna 229, and power supply 226 with power management module 238. Further included can be a multi-functional transceiver 232 which can communicate over Wi-Fi, NFC, Bluetooth, BTLE, and GPS with an antenna 234. As understood by one of skill in the art, these components are electrically and communicatively coupled in a manner to make a functional device.

For purpose of illustration and not limitation, reference is made to the exemplary embodiment of a data receiving device 120 for use with the disclosed subject matter as shown in FIG. 2B. The data receiving device 120, and the related multi-purpose data receiving device 130, includes components germane to the discussion of the analyte sensor 110 and its operations and additional components can be included. In particular embodiments, the data receiving device 120 and multi-purpose data receiving device 130 can be or include components provided by a third party and are not necessarily restricted to include devices made by the same manufacturer as the sensor 110.

As illustrated in FIG. 2B, the data receiving device 120 includes an ASIC 4000 including a microcontroller 4010, memory 4020, and storage 4030 and communicatively coupled with a communication module 4040. Power for the components of the data receiving device 120 can be delivered by a power module 4050, which as embodied herein can include a rechargeable battery. The data receiving device 120 can further include a display 4070 for facilitating review of analyte data received from an analyte sensor 110 or other device (e.g., user device 140 or remote application server 150). The data receiving device 120 can include separate user interface components (e.g., physical keys, light sensors, microphones, etc.).

The communication module 4040 can include a BLE module 4041 and an NFC module 4042. The data receiving device 120 can be configured to wirelessly couple with the analyte sensor 110 and transmit commands to and receive data from the analyte sensor 110. As embodied herein, the data receiving device 120 can be configured to operate, with respect to the analyte sensor 110 as described herein, as an NFC scanner and a BLE end point via specific modules (e.g., BLE module 4042 or NFC module 4043) of the communication module 4040. For example, the data receiving device 120 can issue commands (e.g., activation commands for a data broadcast mode of the sensor; pairing commands to identify the data receiving device 120) to the analyte sensor 110 using a first module of the communication module 4040 and receive data from and transmit data to the analyte sensor 110 using a second module of the communication module 4040. The data receiving device 120 can be configured for communication with a user device 140 via a Universal Serial Bus (USB) module 4045 of the communication module 4040.

As another example, the communication module 4040 can include, for example, a cellular radio module 4044. The cellular radio module 4044 can include one or more radio transceivers for communicating using broadband cellular networks, including, but not limited to third generation (3G), fourth generation (4G), and fifth generation (5G) networks. Additionally, the communication module 4040 of the data receiving device 120 can include a Wi-Fi radio module 4043 for communication using a wireless local area network according to one or more of the IEEE 802.11 standards (e.g., 802.11a, 802.11b, 802.11g, 802.11n (aka Wi-Fi 4), 802.11ac (aka Wi-Fi 5), 802.11ax (aka Wi-Fi 6)). Using the cellular radio module 4044 or Wi-Fi radio module 4043, the data receiving device 120 can communicate with the remote application server 150 to receive analyte data or provide updates or input received from a user (e.g., through one or more user interfaces). Although not illustrated, the communication module 5040 of the analyte sensor 120 can similarly include a cellular radio module or Wi-Fi radio module.

As embodied herein, the on-board storage 4030 of the data receiving device 120 can store analyte data received from the analyte sensor 110. Further, the data receiving device 120, multi-purpose data receiving device 130, or a user device 140 can be configured to communicate with a remote application server 150 via a wide area network. As embodied herein, the analyte sensor 110 can provide data to the data receiving device 120 or multi-purpose data receiving device 130. The data receiving device 120 can transmit the data to the user computing device 140. The user computing device 140 (or the multi-purpose data receiving device 130) can in turn transmit that data to a remote application server 150 for processing and analysis.

As embodied herein, the data receiving device 120 can further include sensing hardware 4060 similar to, or expanded from, the sensing hardware 5060 of the analyte sensor 110. In particular embodiments, the data receiving device 120 can be configured to operate in coordination with the analyte sensor 110 and based on analyte data received from the analyte sensor 110. As an example, where the analyte sensor 110 glucose sensor, the data receiving device 120 can be or include an insulin pump or insulin injection pen. In coordination, the compatible device 130 can adjust an insulin dosage for a user based on glucose values received from the analyte sensor.

FIGS. 2C and 2D are block diagrams depicting example embodiments of sensor control device 102 having analyte sensor 110 and sensor electronics 160 (including analyte monitoring circuitry) that can have the majority of the processing capability for rendering end-result data suitable for display to the user. In FIG. 2C, a single semiconductor chip 161 is depicted that can be a custom application specific integrated circuit (ASIC). Shown within ASIC 161 are certain high-level functional units, including an analog front end (AFE) 162, power management (or control) circuitry 164, processor 166, and communication circuitry 168 (which can be implemented as a transmitter, receiver, transceiver, passive circuit, or otherwise according to the communication protocol). In this embodiment, both AFE 162 and processor 166 are used as analyte monitoring circuitry, but in other embodiments either circuit can perform the analyte monitoring function. Processor 166 can include one or more processors, microprocessors, controllers, and/or microcontrollers, each of which can be a discrete chip or distributed amongst (and a portion of) a number of different chips.

A memory 163 is also included within ASIC 161 and can be shared by the various functional units present within ASIC 161, or can be distributed amongst two or more of them. Memory 163 can also be a separate chip. Memory 163 can be volatile and/or non-volatile memory. In this embodiment, ASIC 161 is coupled with power source 170, which can be a coin cell battery, or the like. AFE 162 interfaces with in vivo analyte sensor 110 and receives measurement data therefrom and outputs the data to processor 166 in digital form, which in turn processes the data to arrive at the end-result glucose discrete and trend values, etc. This data can then be provided to communication circuitry 168 for sending, by way of antenna 171, to reader device 120 (not shown), for example, where minimal further processing is needed by the resident software application to display the data.

FIG. 2D is similar to FIG. 2C but instead includes two discrete semiconductor chips 162 and 174, which can be packaged together or separately. Here, AFE 162 is resident on ASIC 161. Processor 166 is integrated with power management circuitry 164 and communication circuitry 168 on chip 174. AFE 162 includes memory 163 and chip 174 includes memory 165, which can be isolated or distributed within. In one example embodiment, AFE 162 is combined with power management circuitry 164 and processor 166 on one chip, while communication circuitry 168 is on a separate chip. In another example embodiment, both AFE 162 and communication circuitry 168 are on one chip, and processor 166 and power management circuitry 164 are on another chip. It should be noted that other chip combinations are possible, including three or more chips, each bearing responsibility for the separate functions described, or sharing one or more functions for fail-safe redundancy.

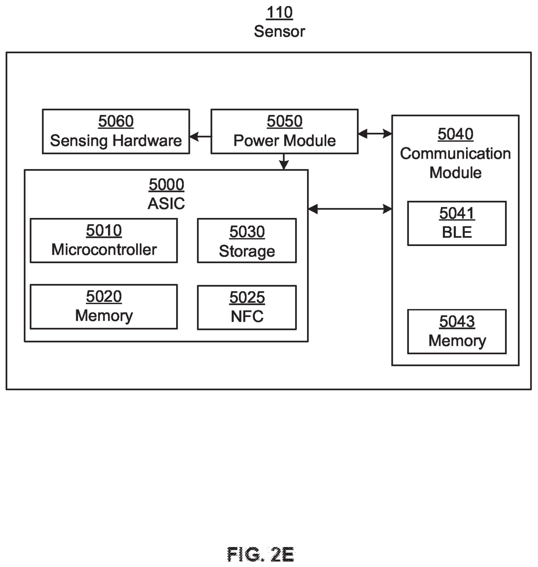

For purpose of illustration and not limitation, reference is made to the exemplary embodiment of an analyte sensor 110 for use with the disclosed subject matter as shown in FIG. 2E. FIG. 2E illustrates a block diagram of an example analyte sensor 110 according to exemplary embodiments compatible with the security architecture and communication schemes described herein.

As embodied herein, the analyte sensor 110 can include an Application-Specific Integrated Circuit ("ASIC") 5000 communicatively coupled with a communication module 5040. The ASIC 5000 can include a microcontroller core 5010, on-board memory 5020, and storage memory 5030. The storage memory 5030 can store data used in an authentication and encryption security architecture. The storage memory 5030 can store programming instructions for the sensor 110. As embodied herein, certain communication chipsets can be embedded in the ASIC 5000 (e.g., an NFC transceiver 5025). The ASIC 5000 can receive power from a power module 5050, such as an on-board battery or from an NFC pulse. The storage memory 5030 of the ASIC 5000 can be programmed to include information such as an identifier for the sensor 110 for identification and tracking purposes. The storage memory 5030 can also be programmed with configuration or calibration parameters for use by the sensor 110 and its various components. The storage memory 5030 can include rewritable or one-time programming (OTP) memory. The storage memory 5030 can be updated using techniques described herein to extend the usefulness of the sensor 110.

As embodied herein, the communication module 5040 of the sensor 100 can be or include one or more modules to support the analyte sensor 110 communicating with other devices of the analyte monitoring system 100. As an example only and not by way of limitation, example communication modules 5040 can include a Bluetooth Low-Energy ("BLE") module 5041 As used throughout this disclosure, Bluetooth Low Energy ("BLE") refers to a short-range communication protocol optimized to make pairing of Bluetooth devices simple for end users. The communication module 5040 can transmit and receive data and commands via interaction with similarly-capable communication modules of a data receiving device 120 or user device 140. The communication module 5040 can include additional or alternative chipsets for use with similar short-range communication schemes, such as a personal area network according to IEEE 802.15 protocols, IEEE 802.11 protocols, infrared communications according to the Infrared Data Association standards (IrDA), etc.

To perform its functionalities, the sensor 100 can further include suitable sensing hardware 5060 appropriate to its function. As embodied herein, the sensing hardware 5060 can include an analyte sensor transcutaneously or subcutaneously positioned in contact with a bodily fluid of a subject. The analyte sensor can generate sensor data containing values corresponding to levels of one or more analytes within the bodily fluid.

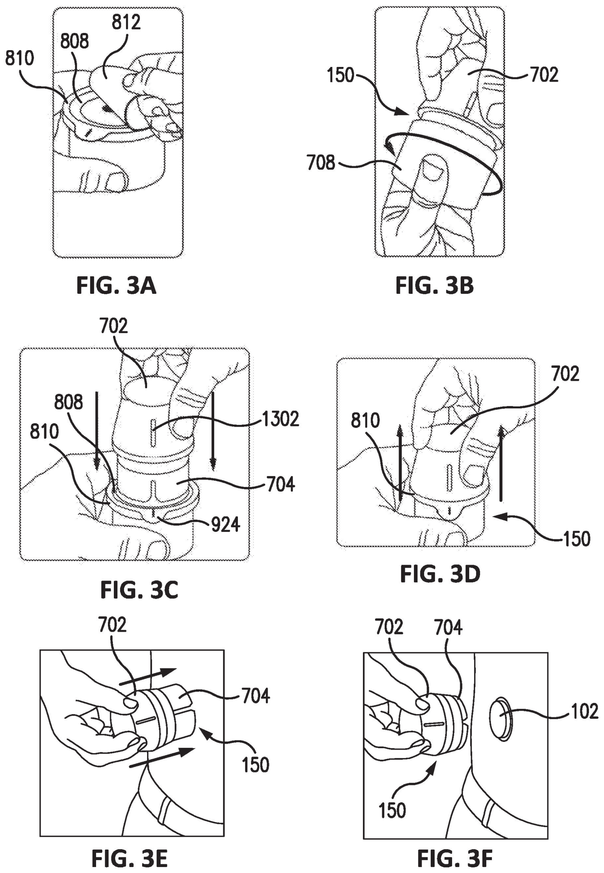

The components of sensor control device 102 can be acquired by a user in multiple packages requiring final assembly by the user before delivery to an appropriate user location. FIGS. 3A-3D depict an example embodiment of an assembly process for sensor control device 102 by a user, including preparation of separate components before coupling the components in order to ready the sensor for delivery. FIGS. 3E-3F depict an example embodiment of delivery of sensor control device 102 to an appropriate user location by selecting the appropriate delivery location and applying device 102 to the location.

FIG. 3A is a proximal perspective view depicting an example embodiment of a user preparing a container 810, configured here as a tray (although other packages can be used), for an assembly process. The user can accomplish this preparation by removing lid 812 from tray 810 to expose platform 808, for instance by peeling a non-adhered portion of lid 812 away from tray 810 such that adhered portions of lid 812 are removed. Removal of lid 812 can be appropriate in various embodiments so long as platform 808 is adequately exposed within tray 810. Lid 812 can then be placed aside.

FIG. 3B is a side view depicting an example embodiment of a user preparing an applicator device 150 for assembly. Applicator device 150 can be provided in a sterile package sealed by a cap 708. Preparation of applicator device 150 can include uncoupling housing 702 from cap 708 to expose sheath 704 (FIG. 3C). This can be accomplished by unscrewing (or otherwise uncoupling) cap 708 from housing 702. Cap 708 can then be placed aside.

FIG. 3C is a proximal perspective view depicting an example embodiment of a user inserting an applicator device 150 into a tray 810 during an assembly. Initially, the user can insert sheath 704 into platform 808 inside tray 810 after aligning housing orienting feature 1302 (or slot or recess) and tray orienting feature 924 (an abutment or detent). Inserting sheath 704 into platform 808 temporarily unlocks sheath 704 relative to housing 702 and also temporarily unlocks platform 808 relative to tray 810. At this stage, removal of applicator device 150 from tray 810 will result in the same state prior to initial insertion of applicator device 150 into tray 810 (i.e., the process can be reversed or aborted at this point and then repeated without consequence).

Sheath 704 can maintain position within platform 808 with respect to housing 702 while housing 702 is distally advanced, coupling with platform 808 to distally advance platform 808 with respect to tray 810. This step unlocks and collapses platform 808 within tray 810. Sheath 704 can contact and disengage locking features (not shown) within tray 810 that unlock sheath 704 with respect to housing 702 and prevent sheath 704 from moving (relatively) while housing 702 continues to distally advance platform 808. At the end of advancement of housing 702 and platform 808, sheath 704 is permanently unlocked relative to housing 702. A sharp and sensor (not shown) within tray 810 can be coupled with an electronics housing (not shown) within housing 702 at the end of the distal advancement of housing 702. Operation and interaction of the applicator device 150 and tray 810 are further described below.

FIG. 3D is a proximal perspective view depicting an example embodiment of a user removing an applicator device 150 from a tray 810 during an assembly. A user can remove applicator 150 from tray 810 by proximally advancing housing 702 with respect to tray 810 or other motions having the same end effect of uncoupling applicator 150 and tray 810. The applicator device 150 is removed with sensor control device 102 (not shown) fully assembled (sharp, sensor, electronics) therein and positioned for delivery.

FIG. 3E is a proximal perspective view depicting an example embodiment of a patient applying sensor control device 102 using applicator device 150 to a target area of skin, for instance, on an abdomen or other appropriate location. Advancing housing 702 distally collapses sheath 704 within housing 702 and applies the sensor to the target location such that an adhesive layer on the bottom side of sensor control device 102 adheres to the skin. The sharp is automatically retracted when housing 702 is fully advanced, while the sensor (not shown) is left in position to measure analyte levels.

FIG. 3F is a proximal perspective view depicting an example embodiment of a patient with sensor control device 102 in an applied position. The user can then remove applicator 150 from the application site.

System 100, described with respect to FIGS. 3A-3F and elsewhere herein, can provide a reduced or eliminated chance of accidental breakage, permanent deformation, or incorrect assembly of applicator components compared to prior art systems. Since applicator housing 702 directly engages platform 808 while sheath 704 unlocks, rather than indirect engagement via sheath 704, relative angularity between sheath 704 and housing 702 will not result in breakage or permanent deformation of the arms or other components. The potential for relatively high forces (such as in conventional devices) during assembly will be reduced, which in turn reduces the chance of unsuccessful user assembly.

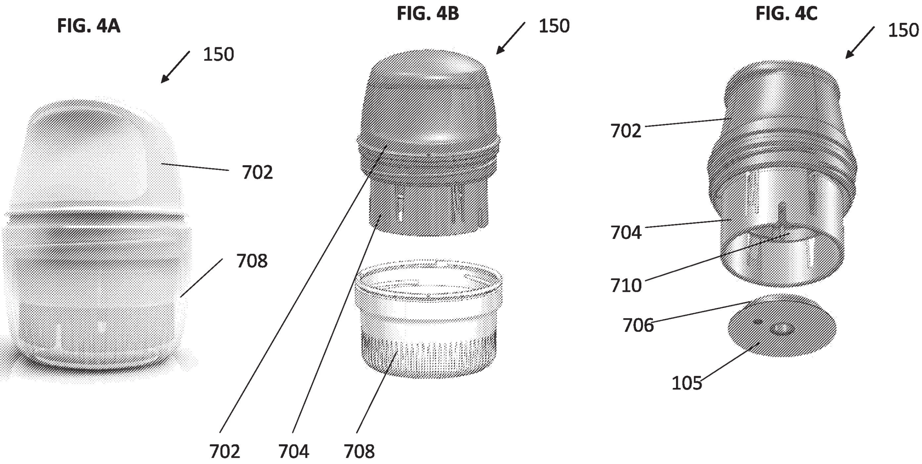

FIG. 4A is a side view depicting an example embodiment of an applicator device 150 coupled with screw cap 708. This is an example of how applicator 150 is shipped to and received by a user, prior to assembly by the user with a sensor. FIG. 4B is a side perspective view depicting applicator 150 and cap 708 after being decoupled. FIG. 4C is a perspective view depicting an example embodiment of a distal end of an applicator device 150 with electronics housing 706 and adhesive patch 105 removed from the position they would have retained within sensor carrier 710 of sheath 704, when cap 708 is in place.

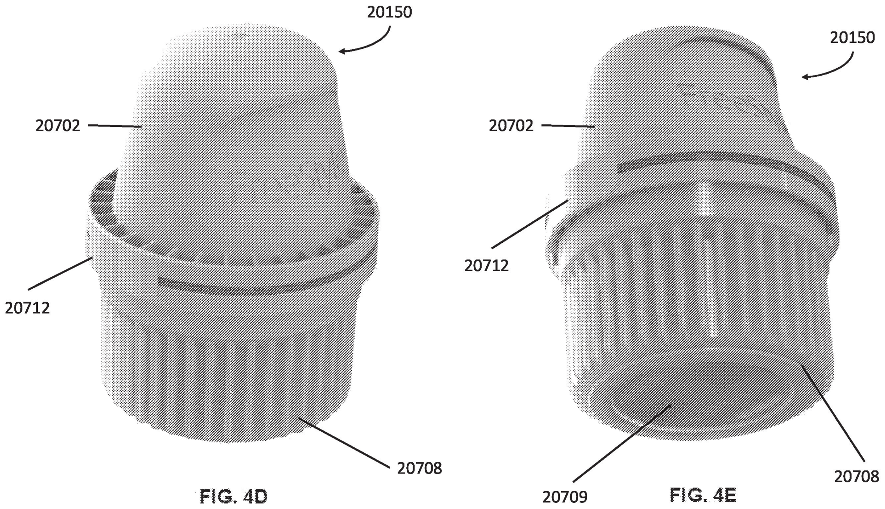

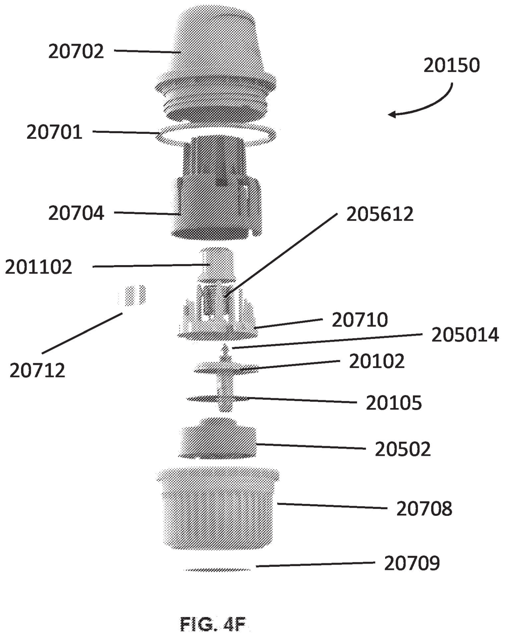

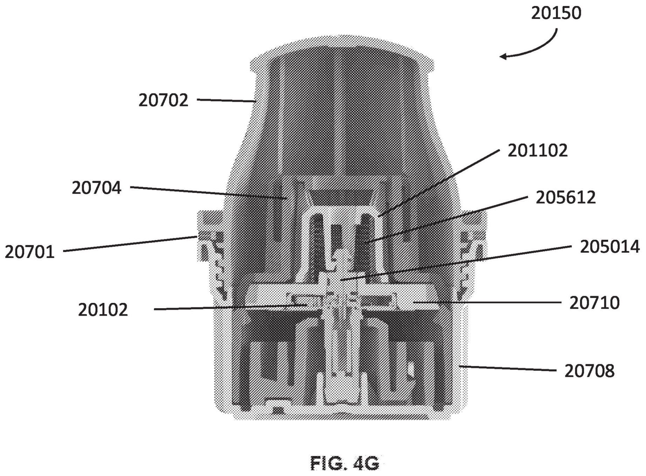

Referring to FIG. 4D-G for purpose of illustration and not limitation, the applicator device 20150 can be provided to a user as a single integrated assembly. FIGs. 4D and 4E provide perspective top and bottom views, respectively, of the applicator device 20150, FIG. 4F provides an exploded view of the applicator device 20150 and FIG. 4G provides a side cut-away view. The perspective views illustrate how applicator 20150 is shipped to and received by a user. The exploded and cut-away views illustrate the components of the applicator device 20150. The applicator device 20150 can include a housing 20702, gasket 20701, sheath 20704, sharp carrier 201102, spring 205612, sensor carrier 20710 (also referred to as a "puck carrier"), sharp hub 205014, sensor control device (also referred to as a "puck") 20102, adhesive patch 20105, desiccant 20502, cap 20708, serial label 20709, and tamper evidence feature 20712. As received by a user, only the housing 20702, cap 20708, tamper evidence feature 20712, and label 20709 are visible. The tamper evidence feature 20712 can be, for example, a sticker coupled to each of the housing 20702 and the cap 20708, and tamper evidence feature 20712 can be damaged, for example, irreparably, by uncoupling housing 20702 and cap 20708, thereby indicating to a user that the housing 20702 and cap 20708 have been previously uncoupled. These features are described in greater detail below.

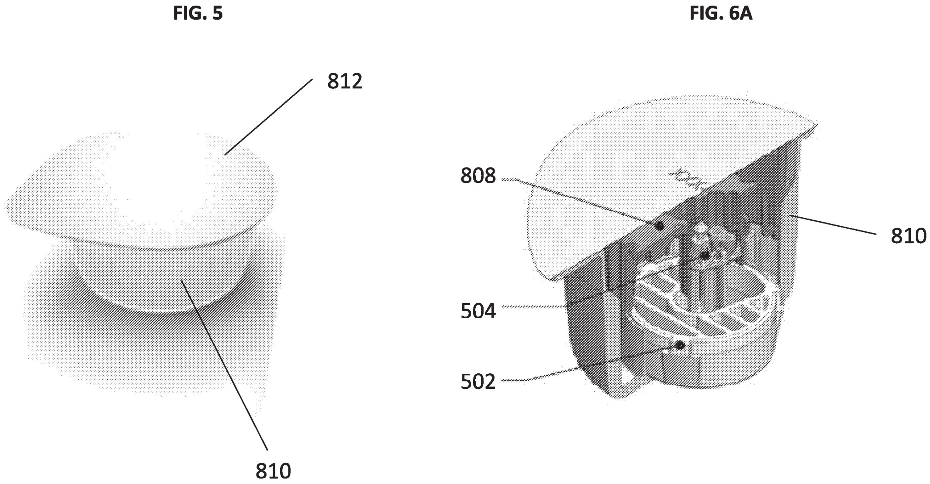

FIG. 5 is a proximal perspective view depicting an example embodiment of a tray 810 with sterilization lid 812 removably coupled thereto, which may be representative of how the package is shipped to and received by a user prior to assembly.

FIG. 6A is a proximal perspective cutaway view depicting sensor delivery components within tray 810. Platform 808 is slidably coupled within tray 810. Desiccant 502 is stationary with respect to tray 810. Sensor module 504 is mounted within tray 810.

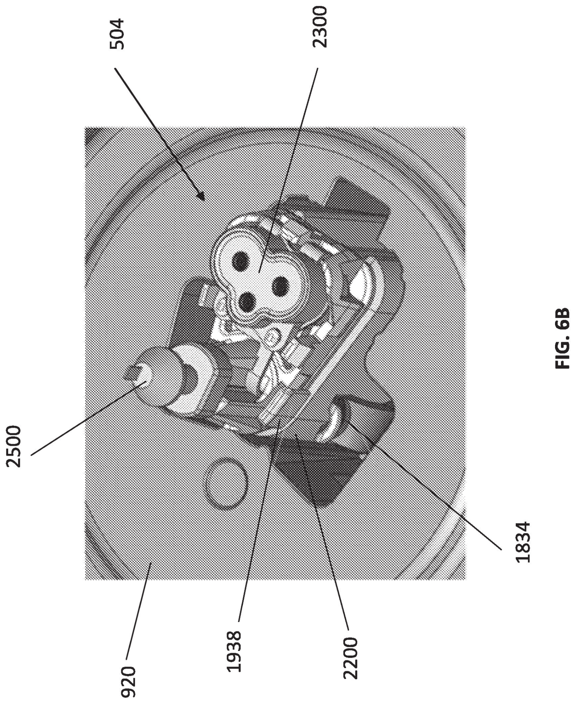

FIG. 6B is a proximal perspective view depicting sensor module 504 in greater detail. Here, retention arm extensions 1834 of platform 808 releasably secure sensor module 504 in position. Module 2200 is coupled with connector 2300, sharp module 2500 and sensor (not shown) such that during assembly they can be removed together as sensor module 504.

Referring briefly again to FIGS. 1 and 3A-3G, for the two-piece architecture system, the sensor tray 202 and the sensor applicator 102 are provided to the user as separate packages, thus requiring the user to open each package and finally assemble the system. In some applications, the discrete, sealed packages allow the sensor tray 202 and the sensor applicator 102 to be sterilized in separate sterilization processes unique to the contents of each package and otherwise incompatible with the contents of the other. More specifically, the sensor tray 202, which includes the plug assembly 207, including the sensor 110 and the sharp 220, may be sterilized using radiation sterilization, such as electron beam (or "e-beam") irradiation. Suitable radiation sterilization processes include, but are not limited to, electron beam (e-beam) irradiation, gamma ray irradiation, X-ray irradiation, or any combination thereof. Radiation sterilization, however, can damage the electrical components arranged within the electronics housing of the sensor control device 102. Consequently, if the sensor applicator 102, which contains the electronics housing of the sensor control device 102, needs to be sterilized, it may be sterilized via another method, such as gaseous chemical sterilization using, for example, ethylene oxide. Gaseous chemical sterilization, however, can damage the enzymes or other chemistry and biologies included on the sensor 110. Because of this sterilization incompatibility, the sensor tray 202 and the sensor applicator 102 are commonly sterilized in separate sterilization processes and subsequently packaged separately, which requires the user to finally assemble the components for use.

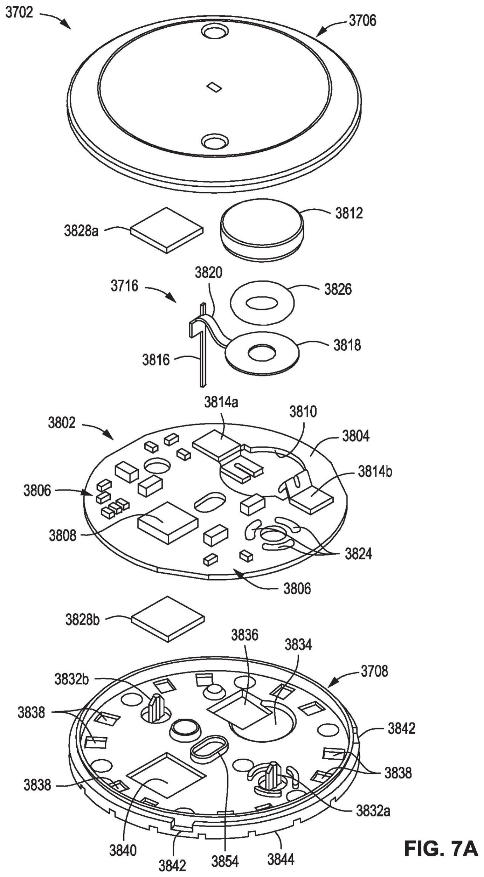

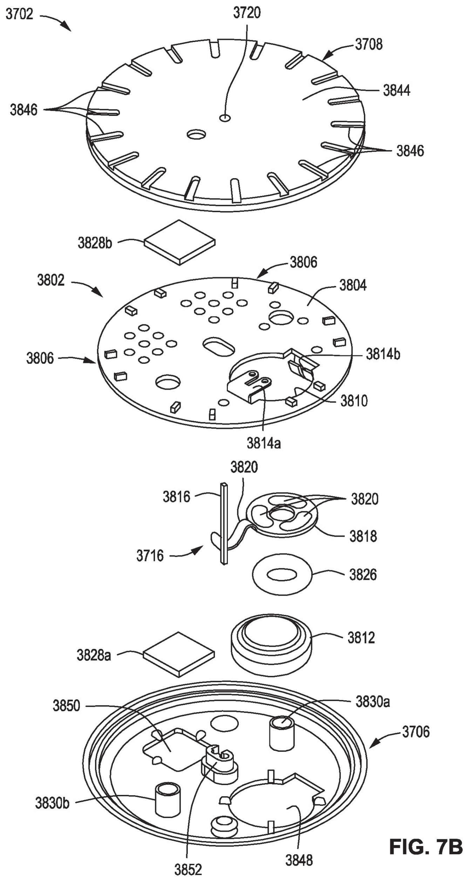

FIGS. 7A and 7B are exploded top and bottom views, respectively, of the sensor control device 3702, according to one or more embodiments. The shell 3706 and the mount 3708 operate as opposing clamshell halves that enclose or otherwise substantially encapsulate the various electronic components of the sensor control device 3702. As illustrated, the sensor control device 3702 may include a printed circuit board assembly (PCBA) 3802 that includes a printed circuit board (PCB) 3804 having a plurality of electronic modules 3806 coupled thereto. Example electronic modules 3806 include, but are not limited to, resistors, transistors, capacitors, inductors, diodes, and switches. Prior sensor control devices commonly stack PCB components on only one side of the PCB. In contrast, the PCB components 3806 in the sensor control device 3702 can be dispersed about the surface area of both sides (i.e., top and bottom surfaces) of the PCB 3804.

Besides the electronic modules 3806, the PCBA 3802 may also include a data processing unit 3808 mounted to the PCB 3804. The data processing unit 3808 may comprise, for example, an application specific integrated circuit (ASIC) configured to implement one or more functions or routines associated with operation of the sensor control device 3702. More specifically, the data processing unit 3808 may be configured to perform data processing functions, where such functions may include but are not limited to, filtering and encoding of data signals, each of which corresponds to a sampled analyte level of the user. The data processing unit 3808 may also include or otherwise communicate with an antenna for communicating with the reader device 106 (FIG. 1).

A battery aperture 3810 may be defined in the PCB 3804 and sized to receive and seat a battery 3812 configured to power the sensor control device 3702. An axial battery contact 3814a and a radial battery contact 3814b may be coupled to the PCB 3804 and extend into the battery aperture 3810 to facilitate transmission of electrical power from the battery 3812 to the PCB 3804. As their names suggest, the axial battery contact 3814a may be configured to provide an axial contact for the battery 3812, while the radial battery contact 3814b may provide a radial contact for the battery 3812. Locating the battery 3812 within the battery aperture 3810 with the battery contacts 3814a,b helps reduce the height H of the sensor control device 3702, which allows the PCB 3804 to be located centrally and its components to be dispersed on both sides (i.e., top and bottom surfaces). This also helps facilitate the chamfer 3718 provided on the electronics housing 3704.

The sensor 3716 may be centrally located relative to the PCB 3804 and include a tail 3816, a flag 3818, and a neck 3820 that interconnects the tail 3816 and the flag 3818. The tail 3816 may be configured to extend through the central aperture 3720 of the mount 3708 to be transcutaneously received beneath a user's skin. Moreover, the tail 3816 may have an enzyme or other chemistry included thereon to help facilitate analyte monitoring.

The flag 3818 may include a generally planar surface having one or more sensor contacts 3822 (three shown in FIG. 7B) arranged thereon. The sensor contact(s) 3822 may be configured to align with and engage a corresponding one or more circuitry contacts 3824 (three shown in FIG. 7A) provided on the PCB 3804. In some embodiments, the sensor contact(s) 3822 may comprise a carbon impregnated polymer printed or otherwise digitally applied to the flag 3818. Prior sensor control devices typically include a connector made of silicone rubber that encapsulates one or more compliant carbon impregnated polymer modules that serve as electrical conductive contacts between the sensor and the PCB. In contrast, the presently disclosed sensor contacts(s) 3822 provide a direct connection between the sensor 3716 and the PCB 3804 connection, which eliminates the need for the prior art connector and advantageously reduces the height H. Moreover, eliminating the compliant carbon impregnated polymer modules eliminates a significant circuit resistance and therefor improves circuit conductivity.

The sensor control device 3702 may further include a compliant member 3826, which may be arranged to interpose the flag 3818 and the inner surface of the shell 3706. More specifically, when the shell 3706 and the mount 3708 are assembled to one another, the compliant member 3826 may be configured to provide a passive biasing load against the flag 3818 that forces the sensor contact(s) 3822 into continuous engagement with the corresponding circuitry contact(s) 3824. In the illustrated embodiment, the compliant member 3826 is an elastomeric O-ring, but could alternatively comprise any other type of biasing device or mechanism, such as a compression spring or the like, without departing from the scope of the disclosure.

The sensor control device 3702 may further include one or more electromagnetic shields, shown as a first shield 3828a and a second shield The shell 3706 may provide or otherwise define a first clocking receptacle 3830a (FIG. 7B) and a second clocking receptacle 3830b (FIG. 7B), and the mount 3708 may provide or otherwise define a first clocking post 3832a (FIG. 7A) and a second clocking post 3832b (FIG. 7A). Mating the first and second clocking receptacles 3830a,b with the first and second clocking posts 3832a,b, respectively, will properly align the shell 3706 to the mount 3708.

Referring specifically to FIG. 7A, the inner surface of the mount 3708 may provide or otherwise define a plurality of pockets or depressions configured to accommodate various component parts of the sensor control device 3702 when the shell 3706 is mated to the mount 3708. For example, the inner surface of the mount 3708 may define a battery locator 3834 configured to accommodate a portion of the battery 3812 when the sensor control device 3702 is assembled. An adjacent contact pocket 3836 may be configured to accommodate a portion of the axial contact 3814a.

Moreover, a plurality of module pockets 3838 may be defined in the inner surface of the mount 3708 to accommodate the various electronic modules 3806 arranged on the bottom of the PCB 3804. Furthermore, a shield locator 3840 may be defined in the inner surface of the mount 3708 to accommodate at least a portion of the second shield 3828b when the sensor control device 3702 is assembled. The battery locator 3834, the contact pocket 3836, the module pockets 3838, and the shield locator 3840 all extend a short distance into the inner surface of the mount 3708 and, as a result, the overall height H of the sensor control device 3702 may be reduced as compared to prior sensor control devices. The module pockets 3838 may also help minimize the diameter of the PCB 3804 by allowing PCB components to be arranged on both sides (i.e., top and bottom surfaces).

Still referring to FIG. 7A, the mount 3708 may further include a plurality of carrier grip features 3842 (two shown) defined about the outer periphery of the mount 3708. The carrier grip features 3842 are axially offset from the bottom 3844 of the mount 3708, where a transfer adhesive (not shown) may be applied during assembly. In contrast to prior sensor control devices, which commonly include conical carrier grip features that intersect with the bottom of the mount, the presently disclosed carrier grip features 3842 are offset from the plane (i.e., the bottom 3844) where the transfer adhesive is applied. This may prove advantageous in helping ensure that the delivery system does not inadvertently stick to the transfer adhesive during assembly. Moreover, the presently disclosed carrier grip features 3842 eliminate the need for a scalloped transfer adhesive, which simplifies the manufacture of the transfer adhesive and eliminates the need to accurately clock the transfer adhesive relative to the mount 3708. This also increases the bond area and, therefore, the bond strength.

Referring to FIG. 7B, the bottom 3844 of the mount 3708 may provide or otherwise define a plurality of grooves 3846, which may be defined at or near the outer periphery of the mount 3708 and equidistantly spaced from each other. A transfer adhesive (not shown) may be coupled to the bottom 3844 and the grooves 3846 may be configured to help convey (transfer) moisture away from the sensor control device 3702 and toward the periphery of the mount 3708 during use. In some embodiments, the spacing of the grooves 3846 may interpose the module pockets 3838 (FIG. 7A) defined on the opposing side (inner surface) of the mount 3708. As will be appreciated, alternating the position of the grooves 3846 and the module pockets 3838 ensures that the opposing features on either side of the mount 3708 do not extend into each other. This may help maximize usage of the material for the mount 3708 and thereby help maintain a minimal height H of the sensor control device 3702. The module pockets 3838 may also significantly reduce mold sink, and improve the flatness of the bottom 3844 that the transfer adhesive bonds to.

Still referring to FIG. 7B, the inner surface of the shell 3706 may also provide or otherwise define a plurality of pockets or depressions configured to accommodate various component parts of the sensor control device 3702 when the shell 3706 is mated to the mount 3708. For example, the inner surface of the shell 3706 may define an opposing battery locator 3848 arrangeable opposite the battery locator 3834 (FIG. 7A) of the mount 3708 and configured to accommodate a portion of the battery 3812 when the sensor control device 3702 is assembled. The opposing battery locator 3848 extends a short distance into the inner surface of the shell 3706, which helps reduce the overall height H of the sensor control device 3702.

A sharp and sensor locator 3852 may also be provided by or otherwise defined on the inner surface of the shell 3706. The sharp and sensor locator 3852 may be configured to receive both the sharp (not shown) and a portion of the sensor 3716. Moreover, the sharp and sensor locator 3852 may be configured to align and/or mate with a corresponding sharp and sensor locator 2054 (FIG. 7A) provided on the inner surface of the mount 3708.

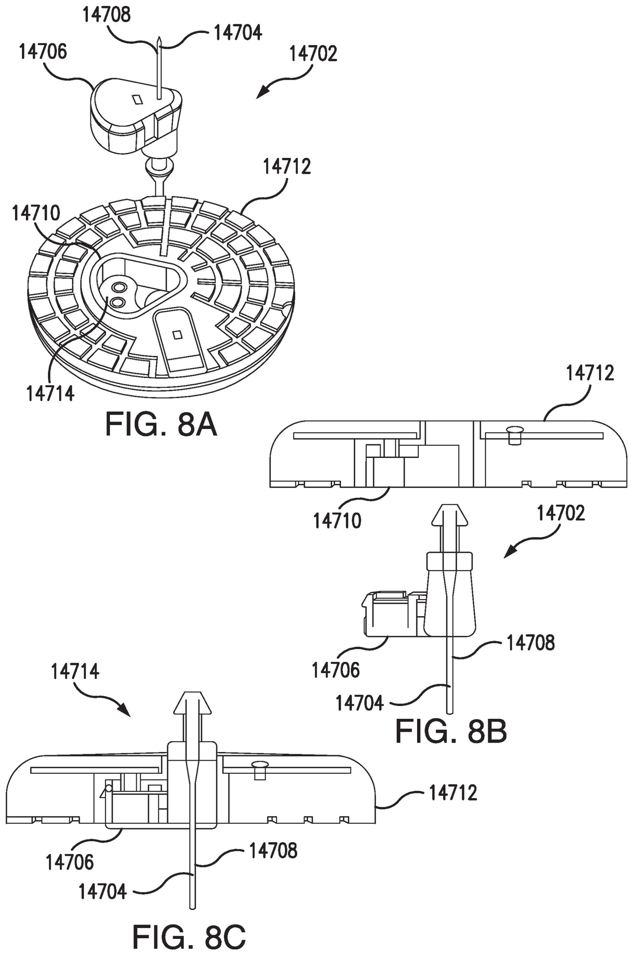

According to embodiments of the present disclosure, an alternative sensor assembly/electronics assembly connection approach is illustrated in FIGS. 8A to 8C. As shown, the sensor assembly 14702 includes sensor 14704, connector support 14706, and sharp 14708. Notably, a recess or receptacle 14710 may be defined in the bottom of the mount of the electronics assembly 14712 and provide a location where the sensor assembly 14702 may be received and coupled to the electronics assembly 14712 , and thereby fully assemble the sensor control device. The profile of the sensor assembly 14702 may match or be shaped in complementary fashion to the receptacle 14710, which includes an elastomeric sealing member 14714 (including conductive material coupled to the circuit board and aligned with the electrical contacts of the sensor 14704). Thus, when the sensor assembly 14702 is snap fit or otherwise adhered to the electronics assembly 14712 by driving the sensor assembly 14702 into the integrally formed recess 14710 in the electronics assembly 14712, the on-body device 14714 depicted in FIG. 8C is formed. This embodiment provides an integrated connector for the sensor assembly 14702 within the electronics assembly 14712.

Additional information regarding sensor assemblies is provided in U.S. Publication No. 2013/0150691 and U.S. Publication No. 2021/0204841, each of which is incorporated by reference herein in its entirety.

According to embodiments of the present disclosure, the sensor control device 102 may be modified to provide a one-piece architecture that may be subjected to sterilization techniques specifically designed for a one-piece architecture sensor control device. A one-piece architecture allows the sensor applicator 150 and the sensor control device 102 to be shipped to the user in a single, sealed package that does not require any final user assembly steps. Rather, the user need only open one package and subsequently deliver the sensor control device 102 to the target monitoring location. The one-piece system architecture described herein may prove advantageous in eliminating component parts, various fabrication process steps, and user assembly steps. As a result, packaging and waste are reduced, and the potential for user error or contamination to the system is mitigated.

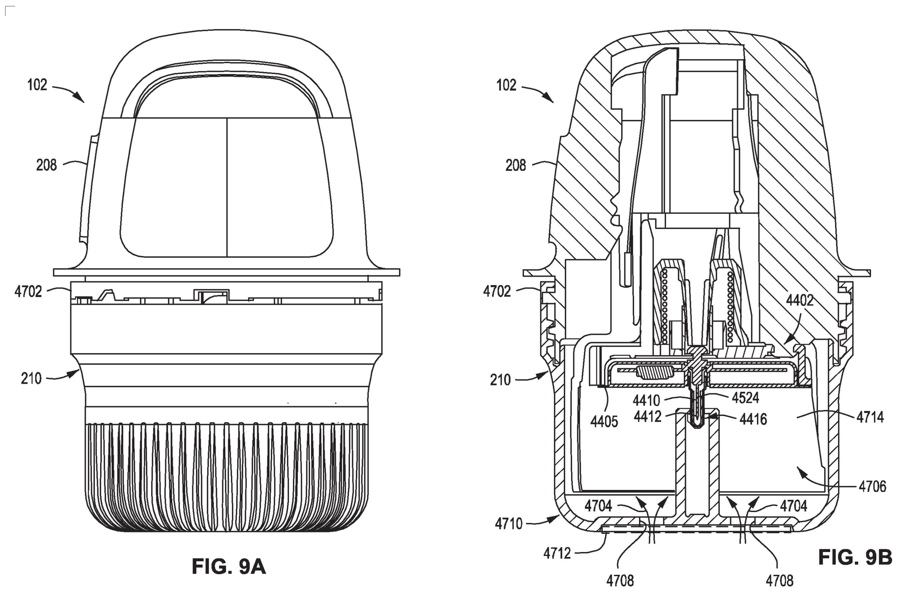

FIGS. 9A and 9B are side and cross-sectional side views, respectively, of an example embodiment of the sensor applicator 102 with the applicator cap 210 coupled thereto. More specifically, FIG. 9A depicts how the sensor applicator 102 might be shipped to and received by a user, and FIG. 9B depicts the sensor control device 4402 arranged within the sensor applicator 102. Accordingly, the fully assembled sensor control device 4402 may already be assembled and installed within the sensor applicator 102 prior to being delivered to the user, thus removing any additional assembly steps that a user would otherwise have to perform.

The fully assembled sensor control device 4402 may be loaded into the sensor applicator 102, and the applicator cap 210 may subsequently be coupled to the sensor applicator 102. In some embodiments, the applicator cap 210 may be threaded to the housing 208 and include a tamper ring 4702. Upon rotating (e.g., unscrewing) the applicator cap 210 relative to the housing 208, the tamper ring 4702 may shear and thereby free the applicator cap 210 from the sensor applicator 102.

According to the present disclosure, while loaded in the sensor applicator 102, the sensor control device 4402 may be subjected to gaseous chemical sterilization 4704 configured to sterilize the electronics housing 4404 and any other exposed portions of the sensor control device 4402. To accomplish this, a chemical may be injected into a sterilization chamber 4706 cooperatively defined by the sensor applicator 102 and the interconnected cap 210. In some applications, the chemical may be injected into the sterilization chamber 4706 via one or more vents 4708 defined in the applicator cap 210 at its proximal end 610. Example chemicals that may be used for the gaseous chemical sterilization 4704 include, but are not limited to, ethylene oxide, vaporized hydrogen peroxide, nitrogen oxide (e.g., nitrous oxide, nitrogen dioxide, etc.), and steam.

Since the distal portions of the sensor 4410 and the sharp 4412 are sealed within the sensor cap 4416, the chemicals used during the gaseous chemical sterilization process do not interact with the enzymes, chemistry, and biologics provided on the tail 4524 and other sensor components, such as membrane coatings that regulate analyte influx.

Once a desired sterility assurance level has been achieved within the sterilization chamber 4706, the gaseous solution may be removed and the sterilization chamber 4706 may be aerated. Aeration may be achieved by a series of vacuums and subsequently circulating a gas (e.g., nitrogen) or filtered air through the sterilization chamber 4706. Once the sterilization chamber 4706 is properly aerated, the vents 4708 may be occluded with a seal 4712 (shown in dashed lines).

In some embodiments, the seal 4712 may comprise two or more layers of different materials. The first layer may be made of a synthetic material (e.g., a flash-spun high-density polyethylene fiber), such as Tyvek^{®} available from DuPont^{®}. Tyvek^{®} is highly durable and puncture resistant and allows the permeation of vapors. The Tyvek^{®} layer can be applied before the gaseous chemical sterilization process, and following the gaseous chemical sterilization process, a foil or other vapor and moisture resistant material layer may be sealed (e.g., heat sealed) over the Tyvek^{®} layer to prevent the ingress of contaminants and moisture into the sterilization chamber 4706. In other embodiments, the seal 4712 may comprise only a single protective layer applied to the applicator cap 210. In such embodiments, the single layer may be gas permeable for the sterilization process, but may also be capable of protection against moisture and other harmful elements once the sterilization process is complete.

With the seal 4712 in place, the applicator cap 210 provides a barrier against outside contamination, and thereby maintains a sterile environment for the assembled sensor control device 4402 until the user removes (unthreads) the applicator cap 210. The applicator cap 210 may also create a dust-free environment during shipping and storage that prevents the adhesive patch 4714 from becoming dirty.

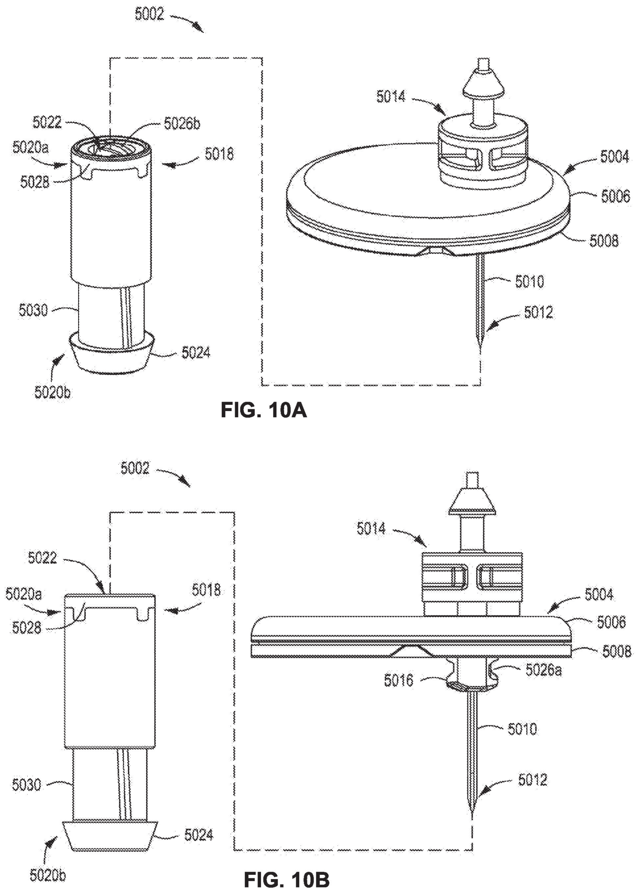

FIGS. 10A and 10B are isometric and side views, respectively, of another example sensor control device 5002, according to one or more embodiments of the present disclosure. The sensor control device 5002 may be similar in some respects to the sensor control device 102 of FIG. 1 and therefore may be best understood with reference thereto. Moreover, the sensor control device 5002 may replace the sensor control device 102 of FIG. 1 and, therefore, may be used in conjunction with the sensor applicator 102 of FIG. 1, which may deliver the sensor control device 5002 to a target monitoring location on a user's skin.

Unlike the sensor control device 102 of FIG. 1, however, the sensor control device 5002 may comprise a one-piece system architecture not requiring a user to open multiple packages and finally assemble the sensor control device 5002 prior to application. Rather, upon receipt by the user, the sensor control device 5002 may already be fully assembled and properly positioned within the sensor applicator 150 (FIG. 1). To use the sensor control device 5002, the user need only open one barrier (e.g., the applicator cap 708 of FIG. 3B) before promptly delivering the sensor control device 5002 to the target monitoring location for use.

As illustrated, the sensor control device 5002 includes an electronics housing 5004 that is generally disc-shaped and may have a circular cross-section. In other embodiments, however, the electronics housing 5004 may exhibit other cross-sectional shapes, such as ovoid or polygonal, without departing from the scope of the disclosure. The electronics housing 5004 may be configured to house or otherwise contain various electrical components used to operate the sensor control device 5002. In at least one embodiment, an adhesive patch (not shown) may be arranged at the bottom of the electronics housing 5004. The adhesive patch may be similar to the adhesive patch 105 of FIG. 1, and may thus help adhere the sensor control device 5002 to the user's skin for use.

As illustrated, the sensor control device 5002 includes an electronics housing 5004 that includes a shell 5006 and a mount 5008 that is matable with the shell 5006. The shell 5006 may be secured to the mount 5008 via a variety of ways, such as a snap fit engagement, an interference fit, sonic welding, one or more mechanical fasteners (e.g., screws), a gasket, an adhesive, or any combination thereof. In some cases, the shell 5006 may be secured to the mount 5008 such that a sealed interface is generated therebetween.

The sensor control device 5002 may further include a sensor 5010 (partially visible) and a sharp 5012 (partially visible), used to help deliver the sensor 5010 transcutaneously under a user's skin during application of the sensor control device 5002. As illustrated, corresponding portions of the sensor 5010 and the sharp 5012 extend distally from the bottom of the electronics housing 5004 (e.g., the mount 5008). The sharp 5012 may include a sharp hub 5014 configured to secure and carry the sharp 5012. As best seen in FIG. 10B, the sharp hub 5014 may include or otherwise define a mating member 5016. To couple the sharp 5012 to the sensor control device 5002, the sharp 5012 may be advanced axially through the electronics housing 5004 until the sharp hub 5014 engages an upper surface of the shell 5006 and the mating member 5016 extends distally from the bottom of the mount 5008. As the sharp 5012 penetrates the electronics housing 5004, the exposed portion of the sensor 5010 may be received within a hollow or recessed (arcuate) portion of the sharp 5012. The remaining portion of the sensor 5010 is arranged within the interior of the electronics housing 5004.

The sensor control device 5002 may further include a sensor cap 5018, shown exploded or detached from the electronics housing 5004 in FIGS. 10A-10B. The sensor cap 5016 may be removably coupled to the sensor control device 5002 (e.g., the electronics housing 5004) at or near the bottom of the mount 5008. The sensor cap 5018 may help provide a sealed barrier that surrounds and protects the exposed portions of the sensor 5010 and the sharp 5012 from gaseous chemical sterilization. As illustrated, the sensor cap 5018 may comprise a generally cylindrical body having a first end 5020a and a second end 5020b opposite the first end 5020a. The first end 5020a may be open to provide access into an inner chamber 5022 defined within the body. In contrast, the second end 5020b may be closed and may provide or otherwise define an engagement feature 5024. As described herein, the engagement feature 5024 may help mate the sensor cap 5018 to the cap (e.g., the applicator cap 708 of FIG. 3B) of a sensor applicator (e.g., the sensor applicator 150 of FIGS. 1 and 3A-3G), and may help remove the sensor cap 5018 from the sensor control device 5002 upon removing the cap from the sensor applicator.

The sensor cap 5018 may be removably coupled to the electronics housing 5004 at or near the bottom of the mount 5008. More specifically, the sensor cap 5018 may be removably coupled to the mating member 5016, which extends distally from the bottom of the mount 5008. In at least one embodiment, for example, the mating member 5016 may define a set of external threads 5026a (FIG. 10B) matable with a set of internal threads 5026b (FIG. 10A) defined by the sensor cap 5018. In some embodiments, the external and internal threads 5026a, b may comprise a flat thread design (e.g., lack of helical curvature), which may prove advantageous in molding the parts. Alternatively, the external and internal threads 5026a,b may comprise a helical threaded engagement. Accordingly, the sensor cap 5018 may be threadably coupled to the sensor control device 5002 at the mating member 5016 of the sharp hub 5014. In other embodiments, the sensor cap 5018 may be removably coupled to the mating member 5016 via other types of engagements including, but not limited to, an interference or friction fit, or a frangible member or substance that may be broken with minimal separation force (e.g., axial or rotational force).

In some embodiments, the sensor cap 5018 may comprise a monolithic (singular) structure extending between the first and second ends 5020a, b. In other embodiments, however, the sensor cap 5018 may comprise two or more component parts. In the illustrated embodiment, for example, the sensor cap 5018 may include a seal ring 5028 positioned at the first end 5020a and a desiccant cap 5030 arranged at the second end 5020b. The seal ring 5028 may be configured to help seal the inner chamber 5022, as described in more detail below. In at least one embodiment, the seal ring 5028 may comprise an elastomeric O-ring. The desiccant cap 5030 may house or comprise a desiccant to help maintain preferred humidity levels within the inner chamber 5022. The desiccant cap 5030 may also define or otherwise provide the engagement feature 5024 of the sensor cap 5018.

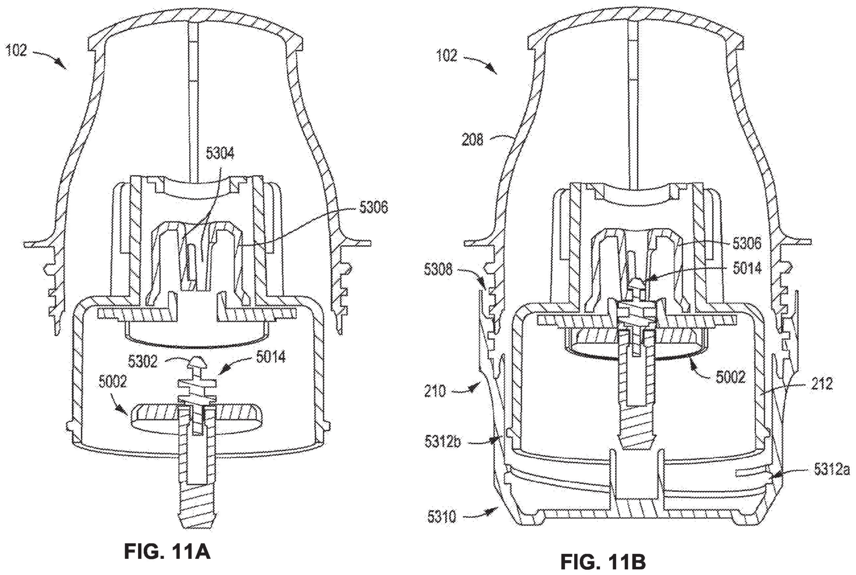

FIGS. 11A-11C are progressive cross-sectional side views showing assembly of the sensor applicator 102 with the sensor control device 5002, according to one or more embodiments. Once the sensor control device 5002 is fully assembled, it may then be loaded into the sensor applicator 102. With reference to FIG. 11A, the sharp hub 5014 may include or otherwise define a hub snap pawl 5302 configured to help couple the sensor control device 5002 to the sensor applicator 102. More specifically, the sensor control device 5002 may be advanced into the interior of the sensor applicator 102 and the hub snap pawl 5302 may be received by corresponding arms 5304 of a sharp carrier 5306 positioned within the sensor applicator 102.

In FIG. 11B, the sensor control device 5002 is shown received by the sharp carrier 5306 and, therefore, secured within the sensor applicator 102. Once the sensor control device 5002 is loaded into the sensor applicator 102, the applicator cap 210 may be coupled to the sensor applicator 102. In some embodiments, the applicator cap 210 and the housing 208 may have opposing, matable sets of threads 5308 that enable the applicator cap 210 to be screwed onto the housing 208 in a clockwise (or counter-clockwise) direction and thereby secure the applicator cap 210 to the sensor applicator 102.

As illustrated, the sheath 212 is also positioned within the sensor applicator 102, and the sensor applicator 102 may include a sheath locking mechanism 5310 configured to ensure that the sheath 212 does not prematurely collapse during a shock event. In the illustrated embodiment, the sheath locking mechanism 5310 may comprise a threaded engagement between the applicator cap 210 and the sheath 212. More specifically, one or more internal threads 5312a may be defined or otherwise provided on the inner surface of the applicator cap 210, and one or more external threads 53 12b may be defined or otherwise provided on the sheath 212. The internal and external threads 53 12a,b may be configured to threadably mate as the applicator cap 210 is threaded to the sensor applicator 102 at the threads 5308. The internal and external threads 5312a,b may have the same thread pitch as the threads 5308 that enable the applicator cap 210 to be screwed onto the housing 208.

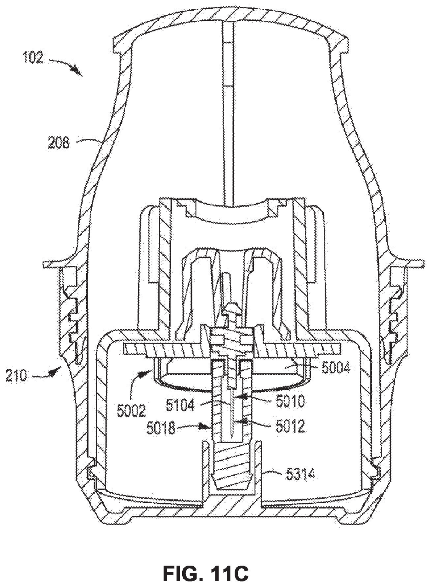

In FIG. 11C, the applicator cap 210 is shown fully threaded (coupled) to the housing 208. As illustrated, the applicator cap 210 may further provide and otherwise define a cap post 5314 centrally located within the interior of the applicator cap 210 and extending proximally from the bottom thereof. The cap post 5314 may be configured to receive at least a portion of the sensor cap 5018 as the applicator cap 210 is screwed onto the housing 208.

With the sensor control device 5002 loaded within the sensor applicator 102 and the applicator cap 210 properly secured, the sensor control device 5002 may then be subjected to a gaseous chemical sterilization configured to sterilize the electronics housing 5004 and any other exposed portions of the sensor control device 5002. Since the distal portions of the sensor 5010 and the sharp 5012 are sealed within the sensor cap 5018, the chemicals used during the gaseous chemical sterilization process are unable to interact with the enzymes, chemistry, and biologies provided on the tail 5104, and other sensor components, such as membrane coatings that regulate analyte influx.

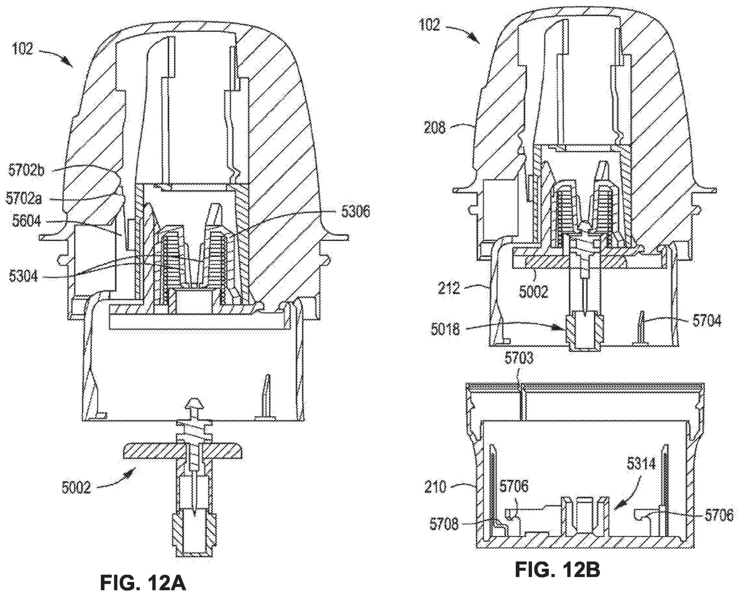

FIGS. 12A-12C are progressive cross-sectional side views showing assembly and disassembly of an alternative embodiment of the sensor applicator 102 with the sensor control device 5002, according to one or more additional embodiments. A fully assembled sensor control device 5002 may be loaded into the sensor applicator 102 by coupling the hub snap pawl 5302 into the arms 5304 of the sharp carrier 5306 positioned within the sensor applicator 102, as generally described above.

In the illustrated embodiment, the sheath arms 5604 of the sheath 212 may be configured to interact with a first detent 5702a and a second detent 5702b defined within the interior of the housing 208. The first detent 5702a may alternately be referred to a "locking" detent, and the second detent 5702b may alternately be referred to as a "firing" detent. When the sensor control device 5002 is initially installed in the sensor applicator 102, the sheath arms 5604 may be received within the first detent 5702a. As discussed below, the sheath 212 may be actuated to move the sheath arms 5604 to the second detent 5702b, which places the sensor applicator 102 in firing position.

In FIG. 12B, the applicator cap 210 is aligned with the housing 208 and advanced toward the housing 208 so that the sheath 212 is received within the applicator cap 210. Instead of rotating the applicator cap 210 relative to the housing 208, the threads of the applicator cap 210 may be snapped onto the corresponding threads of the housing 208 to couple the applicator cap 210 to the housing 208. Axial cuts or slots 5703 (one shown) defined in the applicator cap 210 may allow portions of the applicator cap 210 near its threading to flex outward to be snapped into engagement with the threading of the housing 208. As the applicator cap 210 is snapped to the housing 208, the sensor cap 5018 may correspondingly be snapped into the cap post 5314.

Similar to the embodiment of FIGS. 11A-11C, the sensor applicator 102 may include a sheath locking mechanism configured to ensure that the sheath 212 does not prematurely collapse during a shock event. In the illustrated embodiment, the sheath locking mechanism includes one or more ribs 5704 (one shown) defined near the base of the sheath 212 and configured to interact with one or more ribs 5706 (two shown) and a shoulder 5708 defined near the base of the applicator cap 210. The ribs 5704 may be configured to inter-lock between the ribs 5706 and the shoulder 5708 while attaching the applicator cap 210 to the housing 208. More specifically, once the applicator cap 210 is snapped onto the housing 208, the applicator cap 210 may be rotated (e.g., clockwise), which locates the ribs 5704 of the sheath 212 between the ribs 5706 and the shoulder 5708 of the applicator cap 210 and thereby "locks" the applicator cap 210 in place until the user reverse rotates the applicator cap 210 to remove the applicator cap 210 for use. Engagement of the ribs 5704 between the ribs 5706 and the shoulder 5708 of the applicator cap 210 may also prevent the sheath 212 from collapsing prematurely.

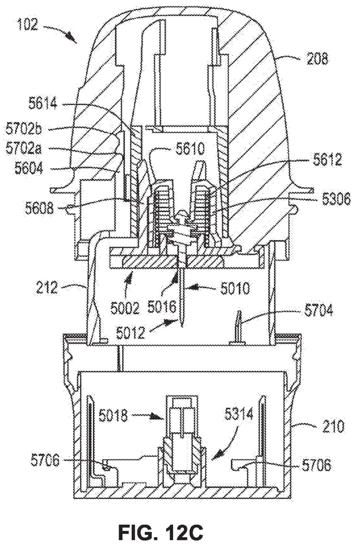

In FIG. 12C, the applicator cap 210 is removed from the housing 208. As with the embodiment of FIGS. 11A-11C, the applicator cap 210 can be removed by reverse rotating the applicator cap 210, which correspondingly rotates the cap post 5314 in the same direction and causes sensor cap 5018 to unthread from the mating member 5016, as generally described above. Moreover, detaching the sensor cap 5018 from the sensor control device 5002 exposes the distal portions of the sensor 5010 and the sharp 5012.

As the applicator cap 210 is unscrewed from the housing 208, the ribs 5704 defined on the sheath 212 may slidingly engage the tops of the ribs 5706 defined on the applicator cap 210. The tops of the ribs 5706 may provide corresponding ramped surfaces that result in an upward displacement of the sheath 212 as the applicator cap 210 is rotated, and moving the sheath 212 upward causes the sheath arms 5604 to flex out of engagement with the first detent 5702a to be received within the second detent 5702b. As the sheath 212 moves to the second detent 5702b, the radial shoulder 5614 moves out of radial engagement with the carrier arm(s) 5608, which allows the passive spring force of the spring 5612 to push upward on the sharp carrier 5306 and force the carrier arm(s) 5608 out of engagement with the groove(s) 5610. As the sharp carrier 5306 moves upward within the housing 208, the mating member 5016 may correspondingly retract until it becomes flush, substantially flush, or sub-flush with the bottom of the sensor control device 5002. At this point, the sensor applicator 102 in firing position. Accordingly, in this embodiment, removing the applicator cap 210 correspondingly causes the mating member 5016 to retract.

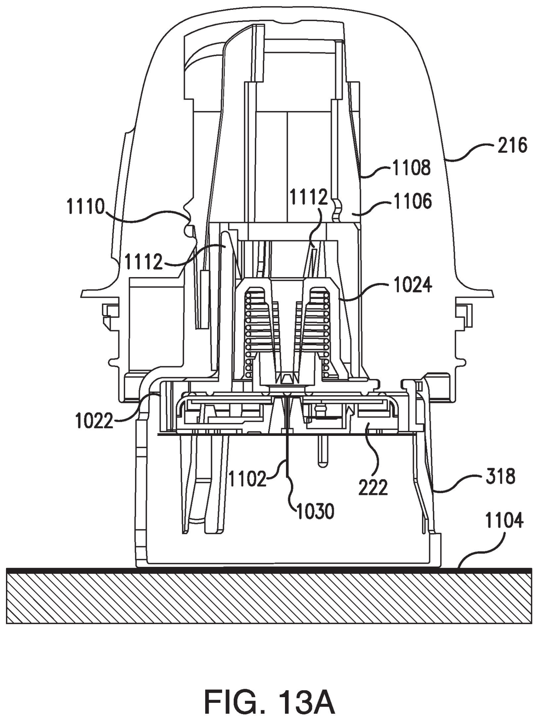

FIGS. 13A-13F illustrate example details of embodiments of the internal device mechanics of "firing" the applicator 216 to apply sensor control device 222 to a user and including retracting sharp 1030 safely back into used applicator 216. All together, these drawings represent an example sequence of driving sharp 1030 (supporting a sensor coupled to sensor control device 222) into the skin of a user, withdrawing the sharp while leaving the sensor behind in operative contact with interstitial fluid of the user, and adhering the sensor control device to the skin of the user with an adhesive. Modification of such activity for use with the alternative applicator assembly embodiments and components can be appreciated in reference to the same by those with skill in the art. Moreover, applicator 216 may be a sensor applicator having one-piece architecture or a two-piece architecture as disclosed herein.

Turning now to FIG. 13A, a sensor 1102 is supported within sharp 1030, just above the skin 1104 of the user. Rails 1106 (optionally three of them) of an upper guide section 1108 may be provided to control applicator 216 motion relative to sheath 318. The sheath 318 is held by detent features 1110 within the applicator 216 such that appropriate downward force along the longitudinal axis of the applicator 216 will cause the resistance provided by the detent features 1110 to be overcome so that sharp 1030 and sensor control device 222 can translate along the longitudinal axis into (and onto) skin 1104 of the user. In addition, catch arms 1112 of sensor carrier 1022 engage the sharp retraction assembly 1024 to maintain the sharp 1030 in a position relative to the sensor control device 222.

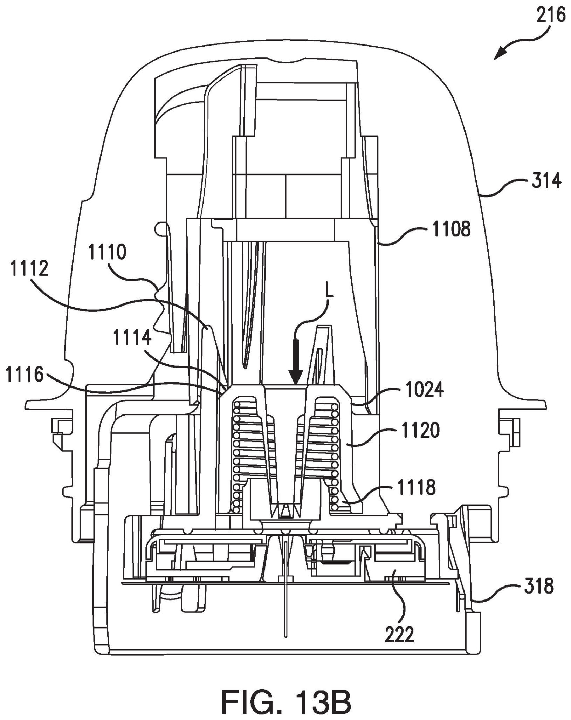

In FIG. 13B, user force is applied to overcome or override detent features 1110 and sheath 318 collapses into housing 314 driving the sensor control device 222 (with associated parts) to translate down as indicated by the arrow L along the longitudinal axis. An inner diameter of the upper guide section 1108 of the sheath 318 constrains the position of carrier arms 1112 through the full stroke of the sensor/sharp insertion process. The retention of the stop surfaces 1114 of carrier arms 1112 against the complimentary faces 1116 of the sharp retraction assembly 1024 maintains the position of the members with return spring 1118 fully energized. According to embodiments, rather than employing user force to drive the sensor control device 222 to translate down as indicated by the arrow L along the longitudinal axis, housing 314 can include a button (for example, not limitation, a push button) which activates a drive spring (for example, not limitation, a coil spring) to drive the sensor control device 222.

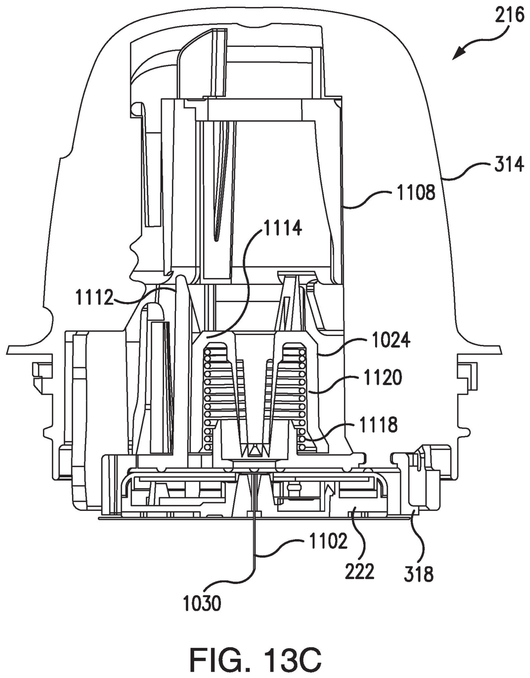

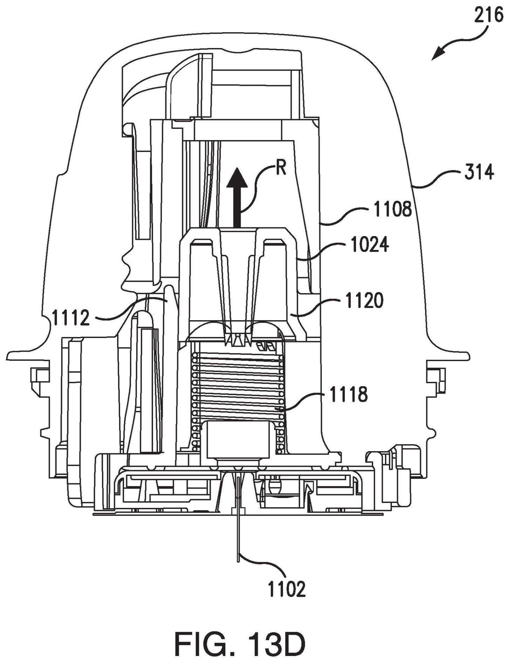

In FIG. 13C, sensor 1102 and sharp 1030 have reached full insertion depth. In so doing, the carrier arms 1112 clear the upper guide section 1108 inner diameter. Then, the compressed force of the coil return spring 1118 drives angled stop surfaces 1114 radially outward, releasing force to drive the sharp carrier 1102 of the sharp retraction assembly 1024 to pull the (slotted or otherwise configured) sharp 1030 out of the user and off of the sensor 1102 as indicated by the arrow R in FIG. 13D.

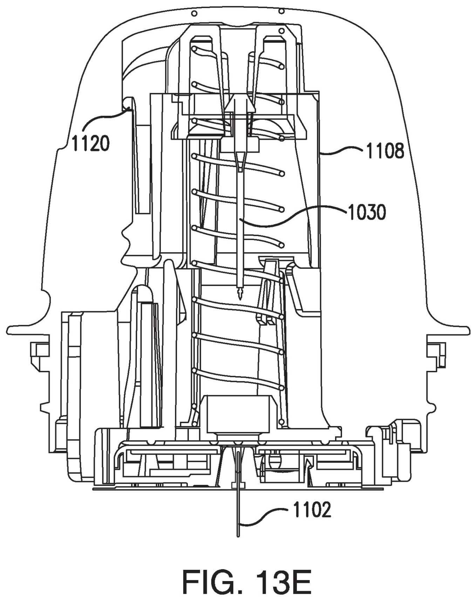

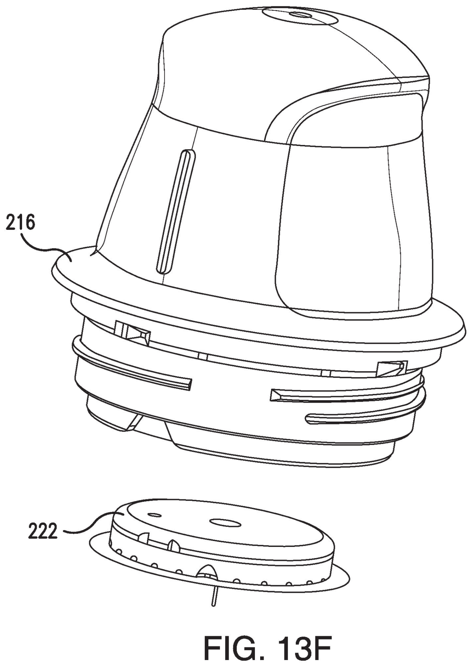

With the sharp 1030 fully retracted as shown in FIG. 13E, the upper guide section 1108 of the sheath 318 is set with a final locking feature 1120. As shown in FIG. 13F, the spent applicator assembly 216 is removed from the insertion site, leaving behind the sensor control device 222, and with the sharp 1030 secured safely inside the applicator assembly 216. The spent applicator assembly 216 is now ready for disposal.

Operation of the applicator 216 when applying the sensor control device 222 is designed to provide the user with a sensation that both the insertion and retraction of the sharp 1030 is performed automatically by the internal mechanisms of the applicator 216. In other words, the present invention avoids the user experiencing the sensation that he is manually driving the sharp 1030 into his skin. Thus, once the user applies sufficient force to overcome the resistance from the detent features of the applicator 216, the resulting actions of the applicator 216 are perceived to be an automated response to the applicator being "triggered." The user does not perceive that he is supplying additional force to drive the sharp 1030 to pierce his skin despite that all the driving force is provided by the user and no additional biasing/driving means are used to insert the sharp 1030. As detailed above in FIG. 13C, the retraction of the sharp 1030 is automated by the coil return spring 1118 of the applicator 216.

With respect to any of the applicator embodiments described herein, as well as any of the components thereof, including but not limited to the sharp, sharp module and sensor module embodiments, those of skill in the art will understand that said embodiments can be dimensioned and configured for use with sensors configured to sense an analyte level in a bodily fluid in the epidermis, dermis, or subcutaneous tissue of a subject. In some embodiments, for example, sharps and distal portions of analyte sensors disclosed herein can both be dimensioned and configured to be positioned at a particular end-depth (i.e., the furthest point of penetration in a tissue or layer of the subject's body, e.g., in the epidermis, dermis, or subcutaneous tissue). With respect to some applicator embodiments, those of skill in the art will appreciate that certain embodiments of sharps can be dimensioned and configured to be positioned at a different end-depth in the subject's body relative to the final end-depth of the analyte sensor. In some embodiments, for example, a sharp can be positioned at a first end-depth in the subject's epidermis prior to retraction, while a distal portion of an analyte sensor can be positioned at a second end-depth in the subject's dermis. In other embodiments, a sharp can be positioned at a first end-depth in the subject's dermis prior to retraction, while a distal portion of an analyte sensor can be positioned at a second end-depth in the subject's subcutaneous tissue. In still other embodiments, a sharp can be positioned at a first end-depth prior to retraction and the analyte sensor can be positioned at a second end-depth, wherein the first end-depth and second end-depths are both in the same layer or tissue of the subject's body.

Additionally, with respect to any of the applicator embodiments described herein, those of skill in the art will understand that an analyte sensor, as well as one or more structural components coupled thereto, including but not limited to one or more spring-mechanisms, can be disposed within the applicator in an off-center position relative to one or more axes of the applicator. In some applicator embodiments, for example, an analyte sensor and a spring mechanism can be disposed in a first off-center position relative to an axis of the applicator on a first side of the applicator, and the sensor electronics can be disposed in a second off-center position relative to the axis of the applicator on a second side of the applicator. In other applicator embodiments, the analyte sensor, spring mechanism, and sensor electronics can be disposed in an off-center position relative to an axis of the applicator on the same side. Those of skill in the art will appreciate that other permutations and configurations in which any or all of the analyte sensor, spring mechanism, sensor electronics, and other components of the applicator are disposed in a centered or off-centered position relative to one or more axes of the applicator are possible and fully within the scope of the present disclosure.

Additional details of suitable devices, systems, methods, components and the operation thereof along with related features are set forth in International Publication No. WO2018/136898 to Rao et. al., International Publication No. WO2019/236850 to Thomas et. al., International Publication No. WO2019/236859 to Thomas et. al., International Publication No. WO2019/236876 to Thomas et. al., and U.S. Patent Publication No. 2020/0196919, filed June 6, 2019, each of which is incorporated by reference in its entirety herein. Further details regarding embodiments of applicators, their components, and variants thereof, are described in U.S. Patent Publication Nos. 2013/0150691, 2016/0331283, and 2018/0235520, all of which are incorporated by reference herein in their entireties and for all purposes. Further details regarding embodiments of sharp modules, sharps, their components, and variants thereof, are described in U.S. Patent Publication No. 2014/0171771, which is incorporated by reference herein in its entirety and for all purposes.

Biochemical sensors can be described by one or more sensing characteristics. A common sensing characteristic is referred to as the biochemical sensor's sensitivity, which is a measure of the sensor's responsiveness to the concentration of the chemical or composition it is designed to detect. For electrochemical sensors, this response can be in the form of an electrical current (amperometric) or electrical charge (coulometric). For other types of sensors, the response can be in a different form, such as a photonic intensity (e.g., optical light). The sensitivity of a biochemical analyte sensor can vary depending on a number of factors, including whether the sensor is in an in vitro state or an in vivo state.

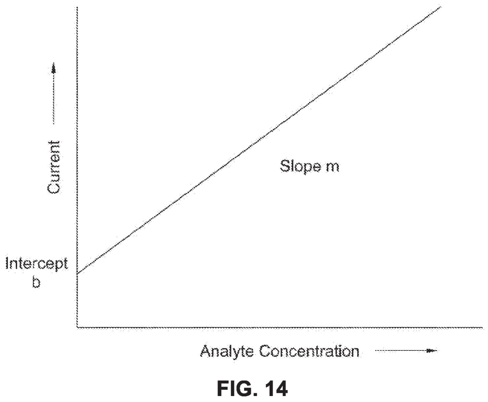

FIG. 14 is a graph depicting the in vitro sensitivity of an amperometric analyte sensor. The in vitro sensitivity can be obtained by in vitro testing the sensor at various analyte concentrations and then performing a regression (e.g., linear or non-linear) or other curve fitting on the resulting data. In this example, the analyte sensor's sensitivity is linear, or substantially linear, and can be modeled according to the equation y=mx+b, where y is the sensor's electrical output current, x is the analyte level (or concentration), m is the slope of the sensitivity and b is the intercept of the sensitivity, where the intercept generally corresponds to a background signal (e.g., noise). For sensors with a linear or substantially linear response, the analyte level that corresponds to a given current can be determined from the slope and intercept of the sensitivity. Sensors with a non-linear sensitivity require additional information to determine the analyte level resulting from the sensor's output current, and those of ordinary skill in the art are familiar with manners by which to model non-linear sensitivities. In certain embodiments of in vivo sensors, the in vitro sensitivity can be the same as the in vivo sensitivity, but in other embodiments a transfer (or conversion) function is used to translate the in vitro sensitivity into the in vivo sensitivity that is applicable to the sensor's intended in vivo use.

Calibration is a technique for improving or maintaining accuracy by adjusting a sensor's measured output to reduce the differences with the sensor's expected output. One or more parameters that describe the sensor's sensing characteristics, like its sensitivity, are established for use in the calibration adjustment.

Certain in vivo analyte monitoring systems require calibration to occur after implantation of the sensor into the user or patient, either by user interaction or by the system itself in an automated fashion. For example, when user interaction is required, the user performs an in vitro measurement (e.g., a blood glucose (BG) measurement using a finger stick and an in vitro test strip) and enters this into the system, while the analyte sensor is implanted. The system then compares the in vitro measurement with the in vivo signal and, using the differential, determines an estimate of the sensor's in vivo sensitivity. The in vivo sensitivity can then be used in an algorithmic process to transform the data collected with the sensor to a value that indicates the user's analyte level. This and other processes that require user action to perform calibration are referred to as "user calibration." Systems can require user calibration due to instability of the sensor's sensitivity, such that the sensitivity drifts or changes over time. Thus, multiple user calibrations (e.g., according to a periodic (e.g., daily) schedule, variable schedule, or on an as-needed basis) can be required to maintain accuracy. While the embodiments described herein can incorporate a degree of user calibration for a particular implementation, generally this is not preferred as it requires the user to perform a painful or otherwise burdensome BG measurement, and can introduce user error.

Some in vivo analyte monitoring systems can regularly adjust the calibration parameters through the use of automated measurements of characteristics of the sensor made by the system itself (e.g., processing circuitry executing software). The repeated adjustment of the sensor's sensitivity based on a variable measured by the system (and not the user) is referred to generally as "system" (or automated) calibration, and can be performed with user calibration, such as an early BG measurement, or without user calibration. Like the case with repeated user calibrations, repeated system calibrations are typically necessitated by drift in the sensor's sensitivity over time. Thus, while the embodiments described herein can be used with a degree of automated system calibration, preferably the sensor's sensitivity is relatively stable over time such that post-implantation calibration is not required.

Some in vivo analyte monitoring systems operate with a sensor that is factory calibrated. Factory calibration refers to the determination or estimation of the one or more calibration parameters prior to distribution to the user or healthcare professional (HCP). The calibration parameter can be determined by the sensor manufacturer (or the manufacturer of the other components of the sensor control device if the two entities are different). Many in vivo sensor manufacturing processes fabricate the sensors in groups or batches referred to as production lots, manufacturing stage lots, or simply lots. A single lot can include thousands of sensors.

Sensors can include a calibration code or parameter which can be derived or determined during one or more sensor manufacturing processes and coded or programmed, as part of the manufacturing process, in the data processing device of the analyte monitoring system or provided on the sensor itself, for example, as a bar code, a laser tag, an RFID tag, or other machine readable information provided on the sensor. User calibration during in vivo use of the sensor can be obviated, or the frequency of in vivo calibrations during sensor wear can be reduced if the code is provided to a receiver (or other data processing device). In embodiments where the calibration code or parameter is provided on the sensor itself, prior to or at the start of the sensor use, the calibration code or parameter can be automatically transmitted or provided to the data processing device in the analyte monitoring system.

Some In vivo analyte monitoring system operate with a sensor that can be one or more of factory calibrated, system calibrated, and/or user calibrated. For example, the sensor can be provided with a calibration code or parameter which can allow for factory calibration. If the information is provided to a receiver (for example, entered by a user), the sensor can operate as a factory calibrated sensor. If the information is not provided to a receiver, the sensor can operate as a user calibrated sensor and/or a system calibrated sensor.

In a further aspect, programming or executable instructions can be provided or stored in the data processing device of the analyte monitoring system, and/or the receiver/controller unit, to provide a time varying adjustment algorithm to the in vivo sensor during use. For example, based on a retrospective statistical analysis of analyte sensors used in vivo and the corresponding glucose level feedback, a predetermined or analytical curve or a database can be generated which is time based, and configured to provide additional adjustment to the one or more in vivo sensor parameters to compensate for potential sensor drift in stability profile, or other factors.

In accordance with the disclosed subject matter, the analyte monitoring system can be configured to compensate or adjust for the sensor sensitivity based on a sensor drift profile. A time varying parameter β(t) can be defined or determined based on analysis of sensor behavior during in vivo use, and a time varying drift profile can be determined. In certain aspects, the compensation or adjustment to the sensor sensitivity can be programmed in the receiver unit, the controller or data processor of the analyte monitoring system such that the compensation or the adjustment or both can be performed automatically and/or iteratively when sensor data is received from the analyte sensor. In accordance with the disclosed subject matter, the adjustment or compensation algorithm can be initiated or executed by the user (rather than self-initiating or executing) such that the adjustment or the compensation to the analyte sensor sensitivity profile is performed or executed upon user initiation or activation of the corresponding function or routine, or upon the user entering the sensor calibration code.

In accordance with the disclosed subject matter, each sensor in the sensor lot (in some instances not including sample sensors used for in vitro testing) can be examined non-destructively to determine or measure its characteristics such as membrane thickness at one or more points of the sensor, and other characteristics including physical characteristics such as the surface area/volume of the active area can be measured or determined. Such measurement or determination can be performed in an automated manner using, for example, optical scanners or other suitable measurement devices or systems, and the determined sensor characteristics for each sensor in the sensor lot is compared to the corresponding mean values based on the sample sensors for possible correction of the calibration parameter or code assigned to each sensor. For example, for a calibration parameter defined as the sensor sensitivity, the sensitivity is approximately inversely proportional to the membrane thickness, such that, for example, a sensor having a measured membrane thickness of approximately 4% greater than the mean membrane thickness for the sampled sensors from the same sensor lot as the sensor, the sensitivity assigned to that sensor in one embodiment is the mean sensitivity determined from the sampled sensors divided by 1.04. Likewise, since the sensitivity is approximately proportional to active area of the sensor, a sensor having measured active area of approximately 3% lower than the mean active area for the sampled sensors from the same sensor lot, the sensitivity assigned to that sensor is the mean sensitivity multiplied by 0.97. The assigned sensitivity can be determined from the mean sensitivity from the sampled sensors, by multiple successive adjustments for each examination or measurement of the sensor. In certain embodiments, examination or measurement of each sensor can additionally include measurement of membrane consistency or texture in addition to the membrane thickness and/or surface are or volume of the active sensing area.

Additional information regarding sensor calibration is provided in U.S. Publication No. 2010/00230285 and U.S. Publication No. 2019/0274598, each of which is incorporated by reference herein in its entirety.

The storage memory 5030 of the sensor 110 can include the software blocks related to communication protocols of the communication module. For example, the storage memory 5030 can include a BLE services software block with functions to provide interfaces to make the BLE module 5041 available to the computing hardware of the sensor 110. These software functions can include a BLE logical interface and interface parser. BLE services offered by the communication module 5040 can include the generic access profile service, the generic attribute service, generic access service, device information service, data transmission services, and security services. The data transmission service can be a primary service used for transmitting data such as sensor control data, sensor status data, analyte measurement data (historical and current), and event log data. The sensor status data can include error data, current time active, and software state. The analyte measurement data can include information such as current and historical raw measurement values, current and historical values after processing using an appropriate algorithm or model, projections and trends of measurement levels, comparisons of other values to patient-specific averages, calls to action as determined by the algorithms or models and other similar types of data.

According to aspects of the disclosed subject matter, and as embodied herein, a sensor 110 can be configured to communicate with multiple devices concurrently by adapting the features of a communication protocol or medium supported by the hardware and radios of the sensor 110. As an example, the BLE module 5041 of the communication module 5040 can be provided with software or firmware to enable multiple concurrent connections between the sensor 110 as a central device and the other devices as peripheral devices, or as a peripheral device where another device is a central device.

Connections, and ensuing communication sessions, between two devices using a communication protocol such as BLE can be characterized by a similar physical channel operated between the two devices (e.g., a sensor 110 and data receiving device 120). The physical channel can include a single channel or a series of channels, including for example and without limitation using an agreed upon series of channels determined by a common clock and channel- or frequency-hopping sequence. Communication sessions can use a similar amount of the available communication spectrum, and multiple such communication sessions can exist in proximity. In certain embodiment, each collection of devices in a communication session uses a different physical channel or series of channels, to manage interference of devices in the same proximity.

For purpose of illustration and not limitation, reference is made to an exemplary embodiment of a procedure for a sensor-receiver connection for use with the disclosed subject matter. First, the sensor 110 repeatedly advertises its connection information to its environment in a search for a data receiving device 120. The sensor 110 can repeat advertising on a regular basis until a connection established. The data receiving device 120 detects the advertising packet and scans and filters for the sensor 120 to connect to through the data provided in the advertising packet. Next, data receiving device 120 sends a scan request command and the sensor 110 responds with a scan response packet providing additional details. Then, the data receiving device 120 sends a connection request using the Bluetooth device address associated with the data receiving device 120. The data receiving device 120 can also continuously request to establish a connection to a sensor 110 with a specific Bluetooth device address. Then, the devices establish an initial connection allowing them to begin to exchange data. The devices begin a process to initialize data exchange services and perform a mutual authentication procedure.

During a first connection between the sensor 110 and data receiving device 120, the data receiving device 120 can initialize a service, characteristic, and attribute discovery procedure. The data receiving device 120 can evaluate these features of the sensor 110 and store them for use during subsequent connections. Next, the devices enable a notification for a customized security service used for mutual authentication of the sensor 110 and data receiving device 120. The mutual authentication procedure can be automated and require no user interaction. Following the successful completion of the mutual authentication procedure, the sensor 110 sends a connection parameter update to request the data receiving device 120 to use connection parameter settings preferred by the sensor 110 and configured to maximum longevity.

The data receiving device 120 then performs sensor control procedures to backfill historical data, current data, event log, and factory data. As an example, for each type of data, the data receiving device 120 sends a request to initiate a backfill process. The request can specify a range of records defined based on, for example, the measurement value, timestamp, or similar, as appropriate. The sensor 110 responds with requested data until all previously unsent data in the memory of the sensor 110 is delivered to the data receiving device 120. The sensor 110 can respond to a backfill request from the data receiving device 120 that all data has already been sent. Once backfill is completed, the data receiving device 120 can notify sensor 110 that it is ready to receive regular measurement readings. The sensor 110 can send readings across multiple notifications result on a repeating basis. As embodied herein, the multiple notifications can be redundant notifications to ensure that data is transmitted correctly. Alternatively, multiple notifications can make up a single payload.

For purpose of illustration and not limitation, reference is made to an exemplary embodiment of a procedure to send a shutdown command to the sensor 110. The shutdown operation is executed if the sensor 110 is in, for example, an error state, insertion failed state, or sensor expired state. If the sensor 110 is not in those states, the sensor 110 can log the command and execute the shutdown when sensor 110 transitions into the error state or sensor expired state. The data receiving device 120 sends a properly formatted shutdown command to the sensor 110. If the sensor 110 is actively processing another command, the sensor 110 will respond with a standard error response indicating that the sensor 110 is busy. Otherwise, the sensor 110 sends a response as the command is received. Additionally, the sensor 110 sends a success notification through the sensor control characteristic to acknowledge the sensor 110 has received the command. The sensor 110 registers the shutdown command. At the next appropriate opportunity (e.g., depending on the current sensor state, as described herein), the sensor 110 will shut down.

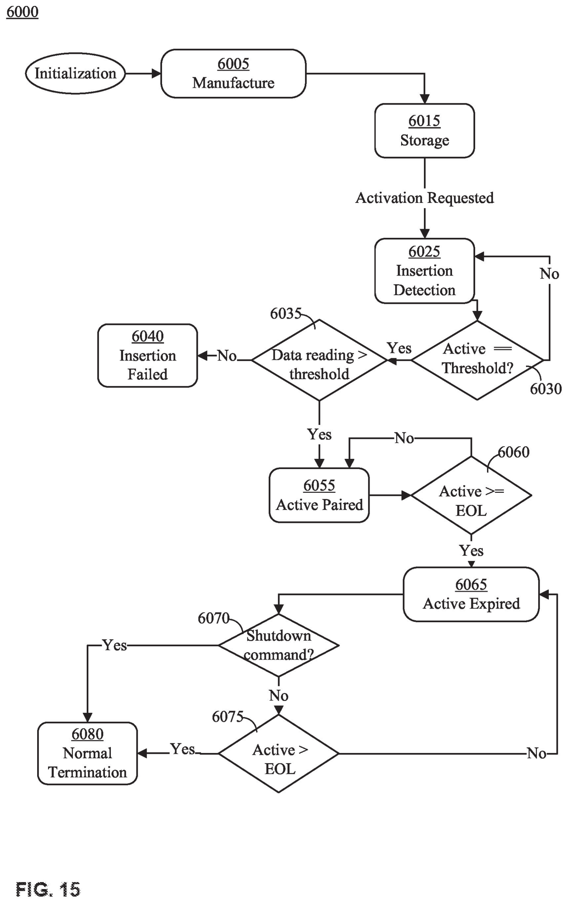

For purpose of illustration and not limitation, reference is made to the exemplary embodiment of a high-level depiction of a state machine representation 6000 of the actions that can be taken by the sensor 110 as shown in FIG. 15. After initialization, the sensor enters state 6005, which relates to the manufacture of the sensor 110. In the manufacture state 6005 the sensor 110 can be configured for operation, for example, the storage memory 5030 can be written. At various times while in state 6005, the sensor 110 checks for a received command to go to the storage state 6015. Upon entry to the storage state 6015, the sensor performs a software integrity check. While in the storage state 6015, the sensor can also receive an activation request command before advancing to the insertion detection state 6025.

Upon entry to state 6025, the sensor 110 can store information relating to devices authenticated to communicate with the sensor as set during activation or initialize algorithms related to conducting and interpreting measurements from the sensing hardware 5060. The sensor 110 can also initialize a lifecycle timer, responsible for maintaining an active count of the time of operation of the sensor 110 and begin communication with authenticated devices to transmit recorded data. While in the insertion detection state 6025, the sensor can enter state 6030, where the sensor 110 checks whether the time of operation is equal to a predetermined threshold. This time of operation threshold can correspond to a timeout function for determining whether an insertion has been successful. If the time of operation has reached the threshold, the sensor 110 advances to state 6035, in which the sensor 110 checks whether the average data reading is greater than a threshold amount corresponding to an expected data reading volume for triggering detection of a successful insertion. If the data reading volume is lower than the threshold while in state 6035, the sensor advances to state 6040, corresponding to a failed insertion. If the data reading volume satisfies the threshold, the sensor advances to the active paired state 6055.

The active paired state 6055 of the sensor 110 reflects the state while the sensor 110 is operating as normal by recording measurements, processing the measurements, and reporting them as appropriate. While in the active paired state 6055, the sensor 110 sends measurement results or attempts to establish a connection with a receiving device 120. The sensor 110 also increments the time of operation. Once the sensor 110 reaches a predetermined threshold time of operation (e.g., once the time of operation reaches a predetermined threshold), the sensor 110 transitions to the active expired state 6065. The active expired state 6065 of the sensor 110 reflects the state while the sensor 110 has operated for its maximum predetermined amount of time.

While in the active expired state 6065, the sensor 110 can generally perform operations relating to winding down operation and ensuring that the collected measurements have been securely transmitted to receiving devices as needed. For example, while in the active expired state 6065, the sensor 110 can transmit collected data and, if no connection is available, can increase efforts to discover authenticated devices nearby and establish and connection therewith. While in the active expired state 6065, the sensor 110 can receive a shutdown command at state 6070. If no shutdown command is received, the sensor 110 can also, at state 6075, check if the time of operation has exceeded a final operation threshold. The final operation threshold can be based on the battery life of the sensor 110. The normal termination state 6080 corresponds to the final operations of the sensor 110 and ultimately shutting down the sensor 110.

Before a sensor is activated, the ASIC 5000 resides in a low power storage mode state. The activation process can begin, for example, when an incoming RF field (e.g., NFC field) drives the voltage of the power supply to the ASIC 5000 above a reset threshold, which causes the sensor 110 to enter a wake-up state. While in the wake-up state, the ASIC 5000 enters an activation sequence state. The ASIC 5000 then wakes the communication module 5040. The communication module 5040 is initialized, triggering a power on self-test. The power on self-test can include the ASIC 5000 communicating with the communication module 5040 using a prescribed sequence of reading and writing data to verify the memory and one-time programmable memory are not corrupted.

When the ASIC 5000 enters the measurement mode for the first time, an insertion detection sequence is performed to verify that the sensor 110 has been properly installed onto the patient's body before a proper measurement can take place. First, the sensor 110 interprets a command to activate the measurement configuration process, causing the ASIC 5000 to enter measurement command mode. The sensor 110 then temporarily enters the measurement lifecycle state to run a number of consecutive measurements to test whether the insertion has been successful. The communication module 5040 or ASIC 5000 evaluates the measurement results to determine insertion success. When insertion is deemed successful, the sensor 110 enters a measurement state, in which the sensor 110 begins taking regular measurements using sensing hardware 5060. If the sensor 110 determines that the insertion was not successful, sensor 110 is triggered into an insertion failure mode, in which the ASIC 5000 is commanded back to storage mode while the communication module 5040 disables itself.

FIG. 1A further illustrates an example operating environment for providing over-the-air ("OTA") updates for use with the techniques described herein. An operator of the analyte monitoring system 100 can bundle updates for the data receiving device 120 or sensor 110 into updates for an application executing on the multi-purpose data receiving device 130. Using available communication channels between the data receiving device 120, the multi-purpose data receiving device 130, and the sensor 110, the multi-purpose data receiving device 130 can receive regular updates for the data receiving device 120 or sensor 110 and initiate installation of the updates on the data receiving device 120 or sensor 110. The multi-purpose data receiving device 130 acts as an installation or update platform for the data receiving device 120 or sensor 110 because the application that enables the multi-purpose data receiving device 130 to communicate with an analyte sensor 110, data receiving device 120 and/or remote application server 150 can update software or firmware on a data receiving device 120 or sensor 110 without wide-area networking capabilities.

As embodied herein, a remote application server 150 operated by the manufacturer of the analyte sensor 110 and/or the operator of the analyte monitoring system 100 can provide software and firmware updates to the devices of the analyte monitoring system 100. In particular embodiments, the remote application server 150 can provides the updated software and firmware to a user device 140 or directly to a multi-purpose data receiving device. As embodied herein, the remote application server 150 can also provide application software updates to an application storefront server 160 using interfaces provided by the application storefront. The multi-purpose data receiving device 130 can contact the application storefront server 160 periodically to download and install the updates.

After the multi-purpose data receiving device 130 downloads an application update including a firmware or software update for a data receiving device 120 or sensor 110, the data receiving device 120 or sensor 110 and multi-purpose data receiving device 130 establish a connection. The multi-purpose data receiving device 130 determines that a firmware or software update is available for the data receiving device 120 or sensor 110. The multi-purpose data receiving device 130 can prepare the software or firmware update for delivery to the data receiving device 120 or sensor 110. As an example, the multi-purpose data receiving device 130 can compress or segment the data associated with the software or firmware update, can encrypt or decrypt the firmware or software update, or can perform an integrity check of the firmware or software update. The multi-purpose data receiving device 130 sends the data for the firmware or software update to the data receiving device 120 or sensor 110. The multi-purpose data receiving device 130 can also send a command to the data receiving device 120 or sensor 110 to initiate the update. Additionally or alternatively, the multi-purpose data receiving device 130 can provide a notification to the user of the multi-purpose data receiving device 130 and include instructions for facilitating the update, such as instructions to keep the data receiving device 120 and the multi-purpose data receiving device 130 connected to a power source and in close proximity until the update is complete.

The data receiving device 120 or sensor 110 receives the data for the update and the command to initiate the update from the multi-purpose data receiving device 130. The data receiving device 120 can then install the firmware or software update. To install the update, the data receiving device 120 or sensor 110 can place or restart itself in a so-called "safe" mode with limited operational capabilities. Once the update is completed, the data receiving device 120 or sensor 110 re-enters or resets into a standard operational mode. The data receiving device 120 or sensor 110 can perform one or more self-tests to determine that the firmware or software update was installed successfully. The multi-purpose data receiving device 130 can receive the notification of the successful update. The multi-purpose data receiving device 130 can then report a confirmation of the successful update to the remote application server 150.

In particular embodiments, the storage memory 5030 of the sensor 110 includes one-time programmable (OTP) memory. The term OTP memory can refer to memory that includes access restrictions and security to facilitate writing to particular addresses or segments in the memory a predetermined number of times. The memory 5030 can be prearranged into multiple pre-allocated memory blocks or containers. The containers are pre-allocated into a fixed size. If storage memory 5030 is one-time programming memory, the containers can be considered to be in a non-programmable state. Additional containers which have not yet been written to can be placed into a programmable or writable state. Containerizing the storage memory 5030 in this fashion can improve the transportability of code and data to be written to the storage memory 5030. Updating the software of a device (e.g., the sensor device described herein) stored in an OTP memory can be performed by superseding only the code in a particular previously-written container or containers with updated code written to a new container or containers, rather than replacing the entire code in the memory. In a second embodiment, the memory is not prearranged. Instead, the space allocated for data is dynamically allocated or determined as needed. Incremental updates can be issued, as containers of varying sizes can be defined where updates are anticipated.

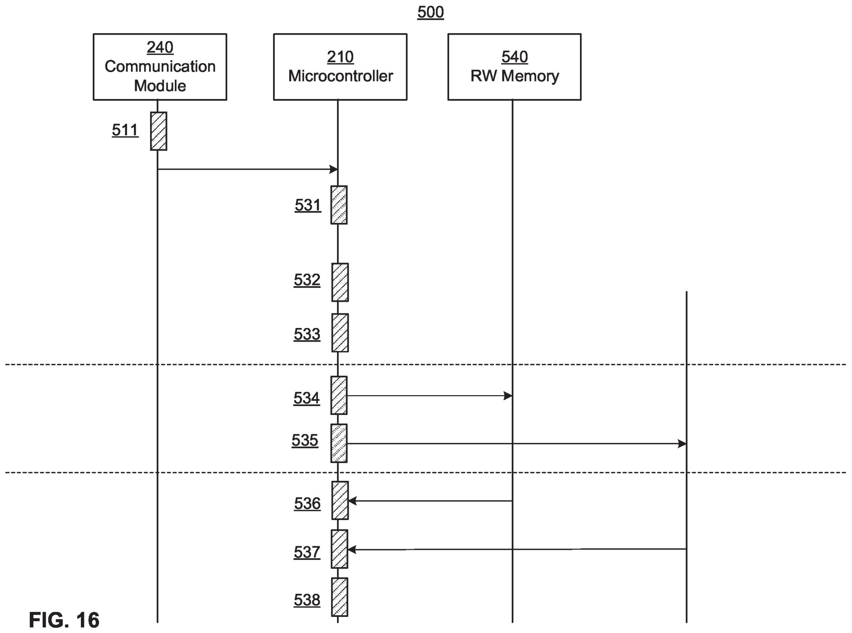

FIG. 16 is a diagram illustrating an example operational and data flow for over-the-air (OTA) programming of a storage memory 5030 in a sensor device 100 as well as use of the memory after the OTA programming in execution of processes by the sensor device 110 according to the disclosed subject matter. In the example OTA programming 500 illustrated in FIG. 5, a request is sent from an external device (e.g., the data receiving device 130) to initiate OTA programming (or re-programming). At 511, a communication module 5040 of a sensor device 110 receives an OTA programming command. The communication module 5040 sends the OTA programming command to the microcontroller 5010 of the sensor device 110.

At 531, after receiving the OTA programming command, the microcontroller 5010 validates the OTA programming command. The microcontroller 5010 can determine, for example, whether the OTA programming command is signed with an appropriate digital signature token. Upon determining that the OTA programming command is valid, the microcontroller 5010 can set the sensor device into an OTA programming mode. At 532, the microcontroller 5010 can validate the OTA programming data. At 533, The microcontroller 5010 can reset the sensor device 110 to re-initialize the sensor device 110 in a programming state. Once the sensor device 110 has transitioned into the OTA programming state, the microcontroller 5010 can begin to write data to the rewriteable memory 540 (e.g., memory 5020) of the sensor device at 534 and write data to the OTP memory 550 of the sensor device at 535 (e.g., storage memory 5030). The data written by the microcontroller 5010 can be based on the validated OTA programming data. The microcontroller 5010 can write data to cause one or more programming blocks or regions of the OTP memory 550 to be marked invalid or inaccessible. The data written to the free or unused portion of the OTP memory can be used to replace invalidated or inaccessible programming blocks of the OTP memory 550. After the microcontroller 5010 writes the data to the respective memories at 534 and 535, the microcontroller 5010 can perform one or more software integrity checks to ensure that errors were not introduced into the programming blocks during the writing process. Once the microcontroller 5010 is able to determine that the data has been written without errors, the microcontroller 5010 can resume standard operations of the sensor device.

In execution mode, at 536, the microcontroller 5010 can retrieve a programming manifest or profile from the rewriteable memory 540. The programming manifest or profile can include a listing of the valid software programming blocks and can include a guide to program execution for the sensor 110. By following the programming manifest or profile, the microcontroller 5010 can determine which memory blocks of the OTP memory 550 are appropriate to execute and avoid execution of out-of-date or invalidated programming blocks or reference to out-of-date data. At 537, the microcontroller 5010 can selectively retrieve memory blocks from the OTP memory 550. At 538, the microcontroller 5010 can use the retrieved memory blocks, by executing programming code stored or using variable stored in the memory.

As embodied herein a first layer of security for communications between the analyte sensor 110 and other devices can be established based on security protocols specified by and integrated in the communication protocols used for the communication. Another layer of security can be based on communication protocols that necessitate close proximity of communicating devices. Furthermore certain packets and/or certain data included within packets can be encrypted while other packets and/or data within packets is otherwise encrypted or not encrypted. Additionally or alternatively, application layer encryption can be used with one or more block ciphers or stream ciphers to establish mutual authentication and communication encryption with other devices in the analyte monitoring system 100.

The ASIC 5000 of the analyte sensor 110 can be configured to dynamically generate authentication and encryption keys using data retained within the storage memory 5030. The storage memory 5030 can also be pre-programmed with a set of valid authentication and encryption keys to use with particular classes of devices. The ASIC 5000 can be further configured to perform authentication procedures with other devices using received data and apply the generated key to sensitive data prior to transmitting the sensitive data. The generated key can be unique to the analyte sensor 110, unique to a pair of devices, unique to a communication session between an analyte sensor 110 and other device, unique to a message sent during a communication session, or unique to a block of data contained within a message.

Both the sensor 110 and a data receiving device 120 can ensure the authorization of the other party in a communication session to, for example, issue a command or receive data. In particular embodiments, identity authentication can be performed through two features. First, the party asserting its identity provides a validated certificate signed by the manufacturer of the device or the operator of the analyte monitoring system 100. Second, authentication can be enforced through the use of public keys and private keys, and shared secrets derived therefrom, established by the devices of the analyte monitoring system 100 or established by the operator of the analyte monitoring system 100. To confirm the identity of the other party, the party can provide proof that the party has control of its private key.

The manufacturer of the analyte sensor 110, data receiving device 120, or provider of the application for multi-purpose data receiving device 130 can provide information and programming necessary for the devices to securely communicate through secured programming and updates. For example, the manufacturer can provide information that can be used to generate encryption keys for each device, including secured root keys for the analyte sensor 110 and optionally for the data receiving device 120 that can be used in combination with device-specific information and operational data (e.g., entropy-based random values) to generate encryption values unique to the device, session, or data transmission as need.

Analyte data associated with a user is sensitive data at least in part because this information can be used for a variety of purposes, including for health monitoring and medication dosing decisions. In addition to user data, the analyte monitoring system 100 can enforce security hardening against efforts by outside parties to reverse-engineering. Communication connections can be encrypted using a device-unique or session-unique encryption key. Encrypted communications or unencrypted communications between any two devices can be verified with transmission integrity checks built into the communications. Analyte sensor 110 operations can be protected from tampering by restricting access to read and write functions to the memory 5020 via a communication interface. The sensor can be configured to grant access only to known or "trusted" devices, provided in a "whitelist" or only to devices that can provide a predetermined code associated with the manufacturer or an otherwise authenticated user. A whitelist can represent an exclusive range, meaning that no connection identifiers besides those included in the whitelist will be used, or a preferred range, in which the whitelist is searched first, but other devices can still be used. The sensor 110 can further deny and shut down connection requests if the requestor cannot complete a login procedure over a communication interface within a predetermined period of time (e.g., within four seconds). These characteristics safeguard against specific denial of service attacks, and in particular against denial of service attacks on a BLE interface.

As embodied herein, the analyte monitoring system 100 can employ periodic key rotation to further reduce the likelihood of key compromise and exploitation. A key rotation strategy employed by the analyte monitoring system 100 can be designed to support backward compatibility of field-deployed or distributed devices. As an example, the analyte monitoring system 100 can employ keys for downstream devices (e.g., devices that are in the field or cannot be feasibly provided updates) that are designed to be compatible with multiple generations of keys used by upstream devices.

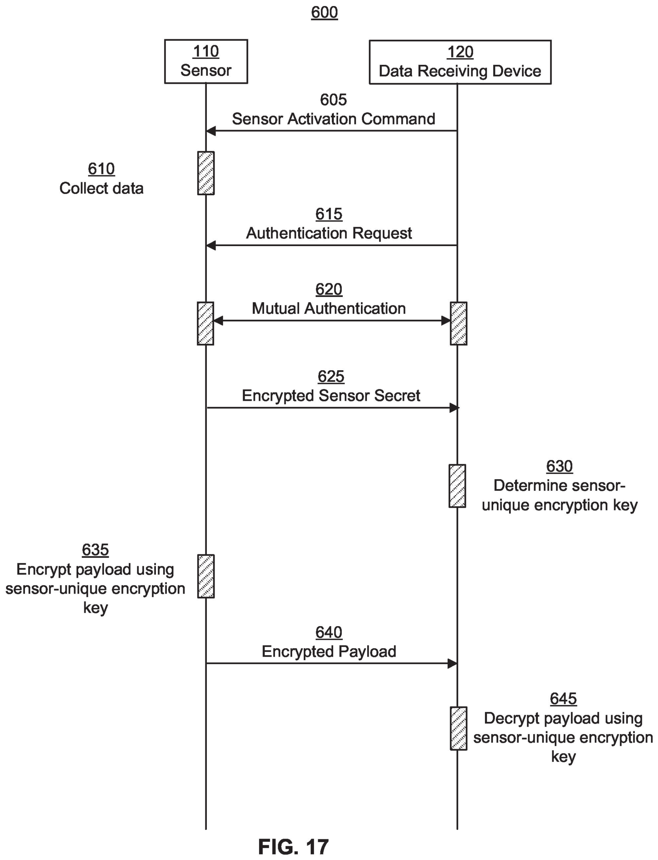

For purpose of illustration and not limitation, reference is made to the exemplary embodiment of a message sequence diagram 600 for use with the disclosed subject matter as shown in FIG. 17 and demonstrating an example exchange of data between a pair of devices, particularly a sensor 110 and a data receiving device 120. The data receiving device 120 can, as embodied herein, be a data receiving device 120 or a multi-purpose data receiving device 130. At step 605, the data receiving device 120 can transmit a sensor activation command 605 to the sensor 110, for example via a short-range communication protocol. The sensor 110 can, prior to step 605 be in a primarily dormant state, preserving its battery until full activation is needed. After activation during step 610, the sensor 110 can collect data or perform other operations as appropriate to the sensing hardware 5060 of the sensor 110. At step 615 the data receiving device 120 can initiate an authentication request command 615. In response to the authentication request command 615, both the sensor 110 and data receiving device 120 can engage in a mutual authentication process 620. The mutual authentication process 620 can involve the transfer of data, including challenge parameters that allow the sensor 110 and data receiving device 120 to ensure that the other device is sufficiently capable of adhering to an agreed-upon security framework described herein. Mutual authentication can be based on mechanisms for authentication of two or more entities to each other with or without on-line trusted third parties to verify establishment of a secret key via challenge-response. Mutual authentication can be performed using two-, three-, four-, or five-pass authentication, or similar versions thereof.

Following a successful mutual authentication process 620, at step 625 the sensor 110 can provide the data receiving device 120 with a sensor secret 625. The sensor secret can contain sensor-unique values and be derived from random values generated during manufacture. The sensor secret can be encrypted prior to or during transmission to prevent third-parties from accessing the secret. The sensor secret 625 can be encrypted via one or more of the keys generated by or in response to the mutual authentication process 620. At step 630, the data receiving device 120 can derive a sensor-unique encryption key from the sensor secret. The sensor-unique encryption key can further be session-unique. As such, the sensor-unique encryption key can be determined by each device without being transmitted between the sensor 110 or data receiving device 120. At step 635, the sensor 110 can encrypt data to be included in payload. At step 640, the sensor 110 can transmit the encrypted payload 640 to the data receiving device 120 using the communication link established between the appropriate communication models of the sensor 110 and data receiving device 120. At step 645, the data receiving device 120 can decrypt the payload using the sensor-unique encryption key derived during step 630. Following step 645, the sensor 110 can deliver additional (including newly collected) data and the data receiving device 120 can process the received data appropriately.

As discussed herein, the sensor 110 can be a device with restricted processing power, battery supply, and storage. The encryption techniques used by the sensor 110 (e.g., the cipher algorithm or the choice of implementation of the algorithm) can be selected based at least in part on these restrictions. The data receiving device 120 can be a more powerful device with fewer restrictions of this nature. Therefore, the data receiving device 120 can employ more sophisticated, computationally intense encryption techniques, such as cipher algorithms and implementations.

The analyte sensor 110 can be configured to alter its discoverability behavior to attempt to increase the probability of the receiving device receiving an appropriate data packet and/or provide an acknowledgement signal or otherwise reduce restrictions that can be causing an inability to receive an acknowledgement signal. Altering the discoverability behavior of the analyte sensor 110 can include, for example and without limitation, altering the frequency at which connection data is included in a data packet, altering how frequently data packets are transmitted generally, lengthening or shortening the broadcast window for data packets, altering the amount of time that the analyte sensor 110 listens for acknowledgement or scan signals after broadcasting, including directed transmissions to one or more devices (e.g., through one or more attempted transmissions) that have previously communicated with the analyte sensor 110 and/or to one or more devices on a whitelist, altering a transmission power associated with the communication module when broadcasting the data packets (e.g., to increase the range of the broadcast or decrease energy consumed and extend the life of the battery of the analyte sensor), altering the rate of preparing and broadcasting data packets, or a combination of one or more other alterations. Additionally, or alternatively, the receiving device can similarly adjust parameters relating to the listening behavior of the device to increase the likelihood of receiving a data packet including connection data.

As embodied herein, the analyte sensor 110 can be configured to broadcast data packets using two types of windows. The first window refers to the rate at which the analyte sensor 110 is configured to operate the communication hardware. The second window refers to the rate at which the analyte sensor 110 is configured to be actively transmitting data packets (e.g., broadcasting). As an example, the first window can indicate that the analyte sensor 110 operates the communication hardware to send and/or receive data packets (including connection data) during the first 2 seconds of each 60 second period. The second window can indicate that, during each 2 second window, the analyte sensor 110 transmits a data packet every 60 milliseconds. The rest of the time during the 2 second window, the analyte sensor 110 is scanning. The analyte sensor 110 can lengthen or shorten either window to modify the discoverability behavior of the analyte sensor 110.

In particular embodiments, the discoverability behavior of the analyte sensor can be stored in a discoverability profile, and alterations can be made based on one or more factors, such as the status of the analyte sensor 110 and/or by applying rules based on the status of the analyte sensor 110. For example, when the battery level of the analyte sensor 110 is below a certain amount, the rules can cause the analyte sensor 110 to decrease the power consumed by the broadcast process. As another example, configuration settings associated with broadcasting or otherwise transmitting packets can be adjusted based on the ambient temperature, the temperature of the analyte sensor 110, or the temperature of certain components of communication hardware of the analyte sensor 110. In addition to modifying the transmission power, other parameters associated with the transmission capabilities or processes of the communication hardware of the analyte sensor 110 can be modified, including, but not limited to, transmission rate, frequency, and timing. As another example, when the analyte data indicates that the subject is, or is about to be, experiencing a negative health event, the rules can cause the analyte sensor 110 to increase its discoverability to alert the receiving device of the negative health event.

As embodied herein, certain calibration features for the sensing hardware 5060 of the analyte sensor 110 can be adjusted based on external or interval environment features as well as to compensate for the decay of the sensing hardware 5060 during expended period of disuse (e.g., a "shelf time" prior to use). The calibration features of the sensing hardware 5060 can be autonomously adjusted by the sensor 110 (e.g., by operation of the ASIC 5000 to modify features in the memory 5020 or storage 5030) or can be adjusted by other devices of the analyte monitoring system 100.

As an example, sensor sensitivity of the sensing hardware 5060 can be adjusted based on external temperature data or the time since manufacture. When external temperatures are monitored during the storage of the sensors, the disclosed subject matter can adaptively change the compensation to sensor sensitivity over time when the device experiences changing storage conditions. For purpose of illustration not limitations, adaptive sensitivity adjustment can be performed in an "active" storage mode where the analyte sensor 110 wakes up periodically to measure temperature. These features can save the battery of the analyte device and extend the lifespan of the analyte sensors. At each temperature measurement, the analyte sensor 110 can calculate a sensitivity adjustment for that time period based on the measured temperature. Then, the temperature-weighted adjustments can be accumulated over the active storage mode period to calculate a total sensor sensitivity adjustment value at the end of the active storage mode (e.g., at insertion). Similarly, at insertion, the sensor 110 can determine the time difference between manufacture of the sensor 110 (which can be written to the storage 5030 of the ASIC 5000) or the sensing hardware 5060 and modify sensor sensitivity or other calibration features according to one or more known decay rates or formulas.

Additionally, for purpose of illustration and not limitation, as embodied herein, sensor sensitivity adjustments can account for other sensor conditions, such as sensor drift. Sensor sensitivity adjustments can be hardcoded into the sensor 110 during manufacture, for example in the case of sensor drift, based on an estimate of how much an average sensor would drift. Sensor 110 can use a calibration function that has time-varying functions for sensor offset and gain, which can account for drift over a wear period of the sensor. Thus, sensor 110 can utilize a function used to transform an interstitial current to interstitial glucose utilizing device-dependent functions describing sensor 110 drift over time, and which can represent sensor sensitivity, and can be device specific, combined with a baseline of the glucose profile. Such functions to account for sensor sensitivity and drift can improve sensor 110 accuracy over a wear period and without involving user calibration.

The sensor 110 detects raw measurement values from sensing hardware 5060. On-sensor processing can be performed, such as by one or more models trained to interpret the raw measurement values. Models can be machine learned models trained off-device to detect, predict, or interpret the raw measurement values to detect, predict, or interpret the levels of one or more analytes. Additional trained models can operate on the output of the machine learning models trained to interact with raw measurement values. As an example, models can be used to detect, predict, or recommend events based on the raw measurements and type of analyte(s) detected by the sensing hardware 5060. Events can include, initiation or completion of physical activity, meals, application of medical treatment or medication, emergent health events, and other events of a similar nature.

Models can be provided to the sensor 110, data receiving device 120, or multi-purpose data receiving device 130 during manufacture or during firmware or software updates. Models can be periodically refined, such as by the manufacturer of the sensor 110 or the operator of the analyte monitoring system 100, based on data received from the sensor 110 and data receiving devices of an individual user or multiple users collectively. In certain embodiments, the sensor 110 includes sufficient computational components to assist with further training or refinement of the machine learned models, such as based on unique features of the user to which the sensor 110 is attached. Machine learning models can include, by way of example and not limitation, models trained using or encompassing decision tree analysis, gradient boosting, ada boosting, artificial neural networks or variants thereof, linear discriminant analysis, nearest neighbor analysis, support vector machines, supervised or unsupervised classification, and others. The models can also include algorithmic or rules-based models in addition to machine learned models. Model-based processing can be performed by other devices, including the data receiving device 120 or multi-purpose data receiving device 130, upon receiving data from the sensor 110 (or other downstream devices).

Data transmitted between the sensor 110 and a data receiving device 120 can include raw or processed measurement values. Data transmitted between the sensor 110 and data receiving device 120 can further include alarms or notification for display to a user. The data receiving device 120 can display or otherwise convey notifications to the user based on the raw or processed measurement values or can display alarms when received from the sensor 110. Alarms that may be triggered for display to the user include alarms based on direct analyte values (e.g., one-time reading exceeding a threshold or failing to satisfy a threshold), analyte value trends (e.g., average reading over a set period of time exceeding a threshold or failing to satisfy a threshold; slope); analyte value predictions (e.g., algorithmic calculation based on analyte values exceeds a threshold or fails to satisfy a threshold), sensor alerts (e.g., suspected malfunction detected), communication alerts (e.g., no communication between sensor 110 and data receiving device 120 for a threshold period of time; unknown device attempting or failing to initiate a communication session with the sensor 110), reminders (e.g., reminder to charge data receiving device 120; reminder to take a medication or perform other activity), and other alerts of a similar nature. For purpose of illustration and not limitation, as embodied herein, the alarm parameters described herein can be configurable by a user or can be fixed during manufacture, or combinations of user-settable and non-user-settable parameters.

According to aspects of the disclosed subject matter, for purpose of illustration and not limitation, a device can include a multiple analyte sensor, a transimpedance amplifier, and a differential amplifier. The device can be embodied as analyte sensing hardware. The multiple analyte sensor can include a first working electrode, a second working electrode, a counter electrode, and a reference electrode. Each of the first working electrode and the second working electrode can be configured to receive a signal indicative of a presence of a respective analyte. The analyte can include one or more of glucose, lactate, oxygen, A1C, alcohol, pH, β-hydroxybutyrate, uric acid, creatinine, ketone, or other analytes, such as those described herein. The counter electrode can be a sum of the first working electrode and the second working electrode. The transimpedance amplifier can be configured to receive a first signal from the first working electrode and a second signal from the second working electrode. The transimpedance amplifier can convert the received first signal and the received second signal to an output. The differential amplifier can be configured to subtract a bias offset from the output to generate a modified output. The bias offset can be determined based on a first analyte measured by the first working electrode or a second analyte measured by the second working electrode. The differential amplifier can be further configured to calibrate out a bias dependent residual and a common-mode op-amp characteristic from the output to generate the modified output. The bias dependent residual can be determined based on a two-point offset calibration for one of the first working electrode or the second working electrode. The common-mode op-amp characteristic can be determined based on a three-point calibration.

In accordance with the disclosed subject matter, for purpose of illustration and not limitation, a device can include a multiple analyte sensor and an application specific integrated circuit (ASIC). The device can be embodied as analyte sensing hardware. The multiple analyte sensor can include a first working electrode, a second working electrode, a counter electrode, and a reference electrode. Each of the first working electrode and the second working electrode can be configured to receive a signal indicative of a presence of a respective analyte. The analyte can include one or more of glucose, lactate, oxygen, A1C, alcohol, pH, β-hydroxybutyrate, uric acid, creatinine, ketone, or other analytes, such as those described herein. The ASIC can be configured to receive a first signal of the received signals from the first working electrode and a second signal of the received signals from the second working electrode. The ASIC can set a first independent bias voltage for the first working electrode and a second independent bias voltage for the second working electrode. The ASIC can generate data indicative of an analyte using the first independent bias voltage and the second independent bias voltage.

In accordance with the disclosed subject matter, for purpose of illustration and not limitation, a device can include a multiple analyte sensor, a first application specific integrated circuit (ASIC), and a second ASIC. The device can be embodied as analyte sensing hardware. The multiple analyte sensor can include a first working electrode, a second working electrode, a counter electrode, and a reference electrode. Each of the first working electrode and the second working electrode can be configured to receive a signal indicative of a presence of a respective analyte. The analyte can include one or more of glucose, lactate, oxygen, A1C, alcohol, pH, β-hydroxybutyrate, uric acid, creatinine, ketone, or other analytes, such as those described herein. The first ASIC can be configured to receive a first signal of the received signals from the first working electrode. The first ASIC can set a first independent bias voltage for the first working electrode. The first ASIC can generate data indicative of a first analyte using the first independent bias voltage. The second ASIC can be configured to receive a second signal of the received signals from the second working electrode. The second ASIC can set a second independent bias voltage for the second working electrode. The second ASIC can generate data indicative of a second analyte using the second independent bias voltage.

According to aspects of the disclosed subject matter, the device can further include an analog to digital converter, a communication module, and a negative poise bias amplifier. The analog to digital converter can be configured to convert the modified output to a digital output. The subtraction of the bias offset from the output can reduce the modified output into a range of the analog to digital converter. The communication module can be configured to process the digital output into measurement results. The communication module can be separate from the device. The communication module can be further configured to provide the measurement results to a receiving device for display via wireless communication. The negative poise bias amplifier can be configured to subtract a predetermined voltage from the reference electrode to generate a first working electrode bias to bias the first working electrode. The predetermined voltage can be determined based on a first analyte measured by the first working electrode or a second analyte measured by the second working electrode. The negative poise bias amplifier can be further configured to receive a reference signal from the reference electrode and a negative poise reference. While this disclosure describes the device as including a negative poise bias amplifier, this disclosure contemplates a device as including a positive poise bias amplifier. The positive poise bias amplifier can be configured to add a predetermined voltage from the reference electrode to generate a first working electrode bias to bias the first working electrode. The positive poise bias amplifier can be further configured to receive a reference signal from the reference electrode and a positive poise reference. The device can be configured to detect blood ketone levels, blood glucose levels, or blood lactate levels, or other analyte levels.

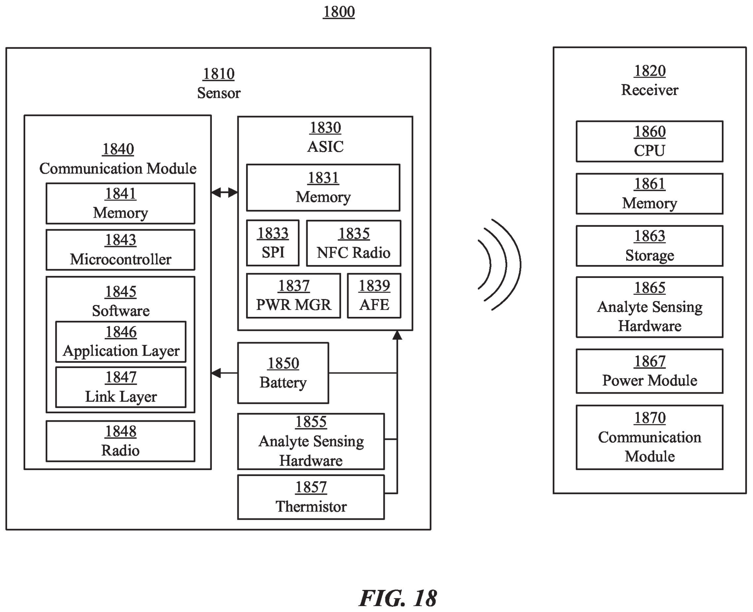

For purpose of illustration and not limitation, reference is made to the exemplary embodiment of a sensor 110 for use with the disclosed subject matter as shown in FIG. 18. FIG. 18 illustrates a block diagram of an example sensor 1810 according to exemplary embodiments compatible with the embedded systems architecture and communication schemes described herein. As embodied herein, the sensor 1810 can include a printed circuit board ("PCB") acting as a backplate for the electronic components of the sensors. Coupled to the PCB is an application-Specific Integrated Circuit ("ASIC") 1830 communicatively coupled with a communication module 1840, analyte sensing hardware 1855 selected for the monitoring function of the sensor 1810, and a thermistor 1857. The ASIC 1830 and communication module 1840 can also be coupled to a battery 1850 supplying power to the ASIC 1830, communication module 1840, and other components of the sensor 1810 not illustrated. The ASIC 1830 and the communication module 1840 can be physically separate modules, as illustrated in FIG. 18. Alternatively, the ASIC 1830 and the communication module 1840 can both be integrated into a single chip.

As embodied herein, as the sensor 1810 is designed to be power-efficient, low-cost, and can be disposable, the ASIC 1830 can include on-board non-volatile memory 1831. The ASIC can include a single reference clock, used for communication and programming, memory management, on-chip timers, etc. The ASIC 1830 can receive power from the battery 1850, regulated by the power manager 1837. Under conditions described herein components of the ASIC 1830 can receive power from nearby fields via coupling with a communication chipset embedded in the ASIC 1830 (e.g., with the NFC radio front end 1835). The NFC radio front end 1835 can be compliant with relevant power-delivery standards. As described herein, the NFC radio front end 1835 can be activated once the battery 1850 is connected. Alternatively, the NFC radio front end 1835 can be used to activate the sensor 1810 and turn on the battery 1850. The ASIC 1830 can further include a serial-peripheral interface (SPI) 1833 and analog front end (AFE) 1839 for communicating with the other components of the sensor 1810. For example, the SPI 1833 can be used for communicating with the communication module 1840 as described herein. The SPI 1833 can also be sued for testing and for programming the components of the sensor 1810 during manufacture. Similarly, the AFE 1839 can be used for communication with the analyte sensing hardware 1855. The sensor 1810 can also include a communication module 1840, as will be described herein.

The non-volatile memory 1831 can be programmed by the manufacturer of the ASIC 1830, by the manufacturer of the sensor 1810 (if the two are manufactured by different entities or at different times) or during other configuration processes. The non-volatile memory 1831 of the ASIC 1830 can be programmed to include information such as an identifier for the ASIC 1830. The identifier can be used to uniquely identify the ASIC 1830 for identification and tracking purposes. The non-volatile memory 1831 of the ASIC 1830 can also be programmed with configuration or calibration parameters for use by the sensor 1810 and its various components, including the NFC radio 1865, power manager 1837, analog front-end 1839, analyte sensing hardware 1855, or by the communication module 1840, as discussed herein.

The non-volatile memory can be further programmed to include a manufacturer code as part of, or separate from, the identifier for the ASIC 1830, communication module 1840, or other components of the sensor 1810. As an example, each manufacturer of components that can be used in the sensor 1810 can be assigned an internationally-recognized code as designated by a standards-setting organization, such as, by way of example only, the International Organization for Standardization. The manufacturer code can be used to identify the manufacturer of the ASIC 1830. As embodied herein, the manufacturer code can be used to verify compatibility with the sensor 1810. For example, the sensor 1810 can be configured to only be used receivers 1820 (or other devices) associated with or produced by a manufacturer from a list of known manufacturers, e.g., a "whitelist", as recognized by the manufacturer code. As another example, the sensor 1810 can be configured to refuse operability with receivers 1820 associated with or produced by manufacturer's on a list of forbidden manufacturers, e.g., a "blacklist". Similarly, the receiver 1820 can be configured to only be compatible with sensors 1810 associated with or produced by select manufacturers.

In one embodiment, the determination of whether a device attempting to communicate or operate with the sensor 1810 will be recognized by the sensor 1810 can be made by the ASIC 1830. As described, the list of valid or invalid manufacturer codes can be programmed into the non-volatile memory 1831 of the ASIC 1830. The ASIC 1830 can then compare its list of manufacturer codes to the manufacturer code of the device attempting to communicate with the sensor 1810. Making this determination at the ASIC 1830 can be advantageous as the non-volatile memory 1831 can be protected from alteration after manufacture of the ASIC 1830 and sensor 1810 is completed. In another embodiment, the determination of whether to allow communication with a device can be made by the communication module 1840. As an example, the communication module 1840 can be updated by the provider of the sensor 1810 to update a list of valid or invalid manufacturer codes (e.g., during a firmware update process). In this way, the list of valid or invalid manufacturer codes is not fixed at time of manufacture but can be adjusted by the provider of the sensor 1810 over time. For example, a new manufacturer can be approved for interoperability or a previously-approved manufacturer can be blocked.

The update process, whether through a firmware update or other process for updating data stored by the sensor 1810 and specifically the communication module can be performed through communication between the communication module 1810 and an update server provided by the provider of the sensor 1810 or an authorized representative thereof. The communication module 1840 can connect to the update server via any of a variety of network protocols. For example, the communication module 1840 can include one or more long-range communication transceivers to allow the communication module 1840 to communicate on a wide area network and with the update server. As an example only, the communication module 1840 can incorporate one or more broadband cellular network radios (and accompanying chipsets) to communicate using, for example third generation (e.g., HSPA, HSPA+), fourth generation (e.g., Mobile WiMAX, LTE, LTE Advanced Pro), fifth generation (e.g., low-band, mid-band, high-band) standards. The communication module 1840 can additionally or alternatively include Wi-Fi radios and chipset to communicate with the wide area network. Additionally or alternatively, the update process can be mediated by a second device, such as a mobile phone or personal computing device. The second device can securely receive an update from the update server and deliver the update the sensor 1810. Relatedly, the sensor 1810 can use the communication capabilities of the second device to connect with the update server.

As an example only and not by way of limitation, example communication modules 1840 of the sensor 1810 can include chipsets compatible with a variety of communication protocols, including Bluetooth Low-Energy ("BLE"), Near-Field Communication ("NFC"), similar short-range communication schemes, wireless personal area networks (WPAN) or piconets, wireless body area networks (WBAN), the Zigbee protocol, WIMAX protocols, or other IEEE 802.15 or IEEE 802.11 protocols, or infrared communication protocols according to the Infrared Data Association standards (IrDA), etc. The communication module 1840 can transmit and receive data and commands via interaction with similarly-capable communication modules 1860 of a receiver 1820. As described herein, the communication module 1840 can include a microcontroller 1843 for executing software operations defined in memory storing software blocks 1845. The software 1845 can be written to the appropriate memory as a one-time programmable memory. The software 1845 can include software directed to an application layer 1846 and a link layer 1847. The application layer 1846 can include application software programmed to the communication module to control functions of the sensor 1810 related to sensing operations, recording measurements, analyzing measurements, data processing, security applications, anti-fraud applications, error handling, and other customized functions. The link layer 1847 can include software programmed to the communication module 1840 used to facilitate communication using the chosen communication protocol. As an example, where the communication module includes a BLE chipset, the link layer 1847 can include software configured to manage communications using the BLE protocol. The link layer 1847 can be preconfigured or updated by the manufacturer of the communication module 1840, whereas the application layer 1846 can be specially configured or updated by the manufacturer of the sensor 1810.

The communication module 1840 can further include a memory 1841 for supporting the operations of the microcontroller 1843. The communication module 1840 can further include interfaces (not illustrated) for communicating with the other components of the sensor 1810. As embodied herein, the microcontroller 1843 of the communication module 1840 can be relatively more capable than the ASIC 1830. As described herein, the microcontroller 1843 of the communication module 1840 can be a general, reprogrammable processor configured to handle more processing-intensive tasks than the ASIC 1830.

As embodied herein, the sensor 1810 can be a disposable device with a predetermined life span, and without wide-area network communication capability. As embodied herein, the communication module 1840 can provide for communication under battery power. Although this disclosure is described with respect to exemplary configurations of the sensor 1810, other suitable configurations are envisioned. As an example, processing hardware of the sensor 1810 can be implemented as another type of special-purpose processor, such as a field programmable gate array (FPGA). As embodied herein, the processing hardware of the sensor 1810 (e.g., of the communication module 1840) can include a general-purpose processing unit (e.g., a CPU) or another programmable processor that is temporarily configured by software to execute the functions of the sensor 1810. More generally, the processing hardware can be implemented using hardware, firmware, software, or a suitable combination of hardware, firmware, and software. For purpose of illustration and not limitation, the processing hardware of the sensor 1810 can be defined by one or more factors including computational capability, power capacity, memory capacity, availability of a network connection, etc.

As embodied herein, the processing responsibilities of the ASIC 1830 and communication module 1840 can be divided based on computational complexity and computational resources. The ASIC 1830 can be configured to focus on interpreting measurements and other data from the analyte sensing hardware 1855. For example, the ASIC 1830 can be specially configured to interpret the analog signals output by the analyte sensing hardware 1855 and received by the ASIC 1830 through the AFE 1839. Through interpretation, the ASIC 1830 can create digital measurements suitable for use in algorithms executed by the communication module 1840. The communication module 1840 can in turn perform advanced processing on the raw measurement signals received from the ASIC 1830. For example, the communication module 1840 can analyze the measurement signals to derive analyte levels and identify trends and actionable responses for the patient, which are then transmitted to the receiver 1820. In certain embodiments, the ASIC 1830 can also be configured to derive analyte levels and identify trends and actionable responses for the patient, before providing the analysis to the communication module 1840 to transmit to the receiver 1820.

To perform its sensing functionalities, the sensor 1800 can further include suitable analyte sensing hardware 1855 appropriate to its function. As embodied herein, the analyte sensing hardware 1855 can include, for example, an autoinjector prescribed to a patient for self-administering a drug or other medicament. Accordingly, the analyte sensing hardware 1855 can include a mechanism that drives a needle or a plunger of a syringe in order to subcutaneously deliver a drug. The syringe can be pre-filled with the drug and can operate in response to a triggering event. For example, the mechanism can drive the needle into the patient and advance the plunger to deliver the drug subcutaneously via the needle.

As embodied herein, the sensor 1810 can be configured as an on-body injector attachable to a patient's body tissue (e.g., skin, organ, muscle, etc.) and capable of automatically delivering a subcutaneous injection of a fixed or patient-selected dose of a drug over a controlled or selected period of time. In such embodiments, the analyte sensing hardware 1855 or analyte sensor can include, for example, an adhesive or other means for temporarily attaching the analyte sensing hardware 1855 to the patient's body tissue, a primary container for storing a drug or medicament, a drive mechanism configured to drive or permit the release of a plunger to discharge the drug from the primary container, a trocar (e.g., a solid core needle), a flexible cannula disposed around the trocar, an insertion mechanism configured to insert the trocar and/or flexible cannula into the patient and optionally retract the trocar leaving the flexible cannula in the patient, a fluid pathway connector configured to establish fluid communication between the primary container and the flexible cannula upon device activation, and an actuator (e.g., a user displaceable button) configured to activate the device. As embodied herein, the on-body injector can be pre-filled and/or pre-loaded.

In addition to mechanical components, the analyte sensing hardware 1855 can include electric and/or electronic components. For example, an electronic switch can be coupled to the mechanism. The sensor 1810 can establish an authenticated communication, receive an encrypted signal, decrypt the signal using the techniques of this disclosure, determine that the signal includes a command to operate the switch, and cause the switch to drive the needle. Thus, the computing device embodied herein can be configured to perform a sensing function using the analyte sensing hardware 1855 in response to a remote command.

As embodied herein, the analyte sensing hardware 1855 can include a travel sensor and an analog-to-digital converter (ADC) to generate a digital signal indicative of the distance travelled by the needle or plunger. Upon delivering the medicament, the sensor 1810 can obtain a reading from the sensor, encrypt the reading using the techniques of this disclosure, and securely report the reading to the peer device. Additionally or alternatively, the sensor 1810 can report other measurements or parameters, such as a time at which the medicament was delivered, volume of medicament delivered, any issues encountered while delivering the medicament, etc. The sensor 1810 can be configured to provide data related to the operation of the analyte sensing hardware 1855 to a remote device.

The analyte sensing hardware 1855 can be configured to implement any suitable combination of one or more sensing functions and can include one or more sensing components. Such sensing components can be configured to detect an operational state of the sensor 1810 (e.g., unpackaged/ready for administration, sterile barrier removal, contact with patient's body tissue, cannula and/or needle insertion, drug delivery initiation, actuator or button displacement, drug delivery completion, plunger position, fluid pathway occlusion, etc.), a condition of the sensor 1810 or drug contained therein (e.g., temperature, shock or vibration exposure, light exposure, drug color, drug turbidity, drug viscosity, geographic location, spatial orientation, temporal information, ambient air pressure, etc.), and/or physiological information about the patient (e.g., body temperature, blood pressure, pulse or heart rate, glucose levels, physical activity or movement, fingerprint detection, etc.).

The analyte sensing hardware 1855 can be connected electrically to the PCB via multiple electrical connections, e.g., a working connection, a reference connection, a counter connection. In embodiment in which the analyte sensing hardware 1855 includes an analyte sensor (and/or a multiple analyte sensor) incorporating multiple electrodes, each electrical connection can correspond to one or more of the electrodes. Voltage from these connections can be delivered to the ASIC 1830 (e.g., through the analog front end 1839). The analyte sensor can generate a current that flows between the electrodes and is dependent on an analyte concentration and temperature. The ambient conditions of the analyte sensor can also change over time which can affect the voltage readings from the analyte sensing hardware provided to the ASIC 1830. The potential of one or more of the electrical connections (e.g., the counter connection) can be adjusted correspondingly to ensure a relatively constant target voltage over other electrical connections (e.g., the reference connection) for interpretation to determine analyte levels.

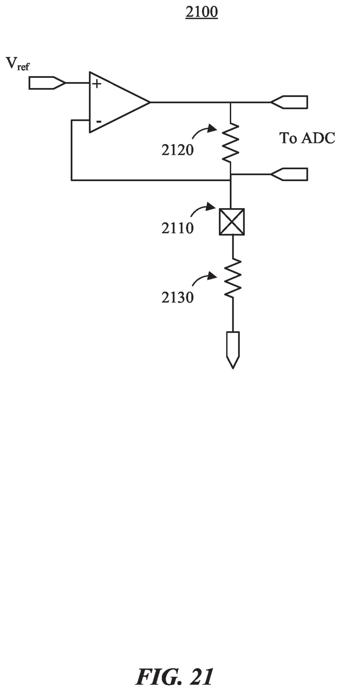

The thermistor 1857 can include one or more thermistors to measure the skin temperature of a patient to which the sensor 1810 is attached or other aspects of the operating environment of the sensor 1810, and especially the analyte sensing hardware 1855. As embodied herein, user skin measurement can be implemented with a single element thermistor and a trimmed resistive elements within the ASIC 1830. The thermistor can be positioned so that its active element is positioned in close proximity to the sensor element (e.g., an analyte sensor sensing element) and in as close thermal contact with the patient's skin as possible. FIG. 21 illustrates a diagram of the thermistor measurement function 2100. To measure the thermistor temperature, ASIC 1830 biases the thermistor element 2110 in series with a trimmed resistive element 2120 and resistive element 2130 included in the ASIC 1830. The ASIC 1830 measures the voltage across the resistive elements 2120 and 2130. The ASIC 1830 can average multiple measurement results depending on a selected sampling frequency. The conversion to temperature can be an iterative calculation, first calculating the temperature based on the trimmed resistive element value and the Steinhart coefficients of the thermistor. Then a second calculation is performed applying the temperature correction for the resistive element. The calculations can further include device specific parameters provided with the ASIC 1830.

FIG. 18 further illustrates an architectural diagram of an exemplary embodiment of a receiver 1820 for use with the disclosed subject matter. As embodied herein, the receiver 1820 can include a small-form factor device. The receiver 1820 can optionally not be as memory- or processing-power constrained as the sensor 1810, and as embodied herein, the receiver 1820 can include sufficient memory for operational software storage and data storage, and sufficient RAM for software execution to communicate with sensor 1810 as described herein. The receiver 1820 can include a CPU 1860, memory 1861, and storage 1863, communicatively coupled with a communication module 1870. Power for the components of the receiver 1820 can be delivered by a power module 1867, which as embodied herein can include a rechargeable battery, allowing for sustained operations and continued use.

The receiver 1820 can be configured to wirelessly couple with, or scan the sensor 1810 and retrieve data, e.g., sensing data or data about the sensor therefrom. As embodied herein, the receiver 1820 can optionally include analyte sensing hardware 1865 similar to, or expanded from, the analyte sensing hardware 1855 of the sensor 1810. As an example only, and not by way of limitation, in an embodiment in which the analyte sensing hardware 1855 of the sensor 1810 is configured for continuous glucose monitoring, the analyte sensing hardware 1867 of the receiver 1820 can be configured with a blood glucose meter, compatible for use with blood glucose test strips, thus expanding on the blood glucose monitoring of the sensor 1810. In additional embodiments, the receiver 1820 does not include the additional analyte sensing hardware 1865.

As embodied herein, the receiver 1820 can be configured to operate, with respect to the sensor 1810 as described herein, as an NFC scanner and a BLE end point via specific modules of the communication module 1870. As embodied herein, the receiver 1820 can be configured for communication with via a Universal Serial Bus (USB) of the communication module 1870. As embodied herein, the on-board storage 1863 of the receiver 1820 can be capable of storing sensing data received from the sensor 1810 over an extended period of time. Further, the receiver 1820 can be configured to communicate with a user computing device or remote cloud server via a wide area network.

Upon successful activation of the sensor 1810 by a receiver 1820, the sensor 1810 can be configured to collect sensing data and makes that data available to the receiver 1820. The receiver 1820 acts as a collector. As an example, the receiver can pair with the sensor 1810 over an NFC interface, providing short-range power to the sensor 1810 and communicatively coupling with the sensor 1810 over said NFC interface. Alternatively, the receiver 1820 can communicatively couple with the sensor 1810 over a medium-range interface, such as a Bluetooth or Bluetooth Low Energy ("BLE") interface or any suitable interfaces compatible with the communication protocols implemented by the sensor 1810, as described herein. The sensor 1810 can transmit sensing data used for monitoring and alarms functions.

As used throughout this disclosure, Bluetooth Low Energy refers to a medium-range communication protocol configured to make paring of Bluetooth devices simple for end users. As described herein, the use of BLE on the sensor 1810 can optionally not rely on standard BLE implementation of Bluetooth for security but can instead use application layer encryption using one or more block ciphers to establish mutual authentication and encryption. The use of a non-standard encryption design implemented in the application layer has several benefits. One benefit of this approach is that the user can complete the pairing of the sensor 1810 and receiver 1820 with only an NFC scan and without involving the user providing additional input, such as entering a security pin or confirming BLE pairing between the data receiving device and the sensor 1810. Another benefit is that this approach mitigates the potential to allow devices that are not in the immediate proximity of the sensor 1810 to inadvertently or intentionally pair, at least in part because the information used to support the pairing process is shared via a back-up short-range communication link (e.g., NFC) over a short range instead of over the longer-range BLE channel. Furthermore, as BLE pairing and bonding schemes are not involved, pairing of the sensor 1810 can avoid implementation issues by chip vendors or vulnerabilities in the BLE specification.

As the data collected by the sensor 1810 and exchanged between the sensor 1810 and data receiving device pertain to sensed information about a user, the data is highly sensitive and can be beneficial to be protected. Sensor data associated with a patient can be considered sensitive data at least in part because this information can be used for a variety of purposes, including for health monitoring and medication dosing decisions. As embodied herein, encryption and authentication can be used as two of the primary technical controls for providing protective features. As embodied herein, the sensor 1810 and receiver 1820 can be configured compliant with a security interface designed to protect the Confidentiality, Integrity and Availability ("CIA") of this communication and associated data. To address these CIA concerns, security functions can be incorporated into the design of the hardware and software.

To facilitate the confidentiality of data, communication connections between the sensor 1810 and receiver 1820 can be mutually authenticated prior to transmitting sensitive data by either device. Communication connections can be encrypted using a device-unique or session-unique encryption key. To guarantee the integrity of data, to ensure that patient data is unmodified, encrypted communications between the sensor 1810 and receiver 1820 can be verified with transmission integrity checks built into the communications. As embodied herein, session key information, which can be used to encrypt the communication, can be exchanged between two devices after the devices have each been authenticated.

As embodied herein, the sensor 1810 and receiver 1820 can each employ a variety of security practices to ensure the confidentiality of data exchanged over communication sessions and facilitate the relevant devices to find and establish connections with trusted endpoints. As an example, the sensor 1810 can configure the communication module 1840 to use preconfigured advertising parameters with a public device address. The communication module can send connectable undirected advertising events and process scans and connection requests from all receivers 1820. The sensor 1810 can request the communication module 1840 to start advertising immediately after it is activated. The communication module 1840 can continue to advertise until it receives and accepts a connection request packet. Once connected to a receiver 1820, the communication module 1840 can be no longer discoverable. No other device can connect to it. The communication module 1840 can also stop advertising if no connection request packet is received within a predefined amount of time (e.g., two second, four seconds). The sensor 1810 can stop advertising by configuring the communication module to non-discoverable mode. Once in the non-discoverable mode, the communication module 1840 stops sending advertising events, and no device can discover or connect to it. To restart advertising, the sensor 1810 requests the communication module 1840 to advertise on every other instance when a measurement is logged. Therefore, if the sensor 1810 is not connected to a receiver 1820, it can restart advertising every two minutes. When a sensor 1810 is both activated and is in a state in which no measurement data is logged, the sensor 1810 can use a timer to continue the same advertising schedule.

These characteristics safeguard against specific denial of service attacks, and in particular against denial of service attacks on a BLE interface. As embodied herein, the identifiers used to connect to the sensor 1810 can be mutable to reduce the ability to track a single sensor 1810 as it connects to one or more data receiving devices. Connection identifiers for the sensor 1810 or data receiving device can include, as an example only, a unique or semi-unique device identifier, a media access control address for the communication module of the device, a device address configured for the particular communication protocol (e.g., a BLUETOOTH address, etc.), internet protocol address, an identifier assigned to the device by the analyte monitoring system, a universally-agreed identifier for the type of device that is broadcasting, etc. The sensor 1810 can change identifiers between sensor 1810 activation and pairing with the first receiver 1820. If the sensor 1810 disconnects from the first receiver 1820 during its active use timeline, the sensor 1810 can change the connection identifier on disconnection or on receiving a request for a new connection with a second receiver 1820.

As embodied herein, the sensor can support establishing long-term connection pairs by storing encryption and authentication keys associated with data receiving devices, or support a data receiving device storing an encryption and authentication key for the sensor 1810 for a prolonged period of time. For example, the sensor 1810 or data receiving device can associate a connection identifier for the other party to the exchange in association with the encryption and authentication keys used by the other party. In so doing, the sensor 1810 can establish connections with a data receiving device more quickly, at least in part because sensor 1810 can avoid establishing a new authentication pairing with that data receiving device and can proceed directly to exchanging information via the encrypted communication protocols described herein. After a connection is successfully established, the two devices can refrain from broadcasting connection identifiers and other information to establish a new connection and can communicate using an agreed channel-hopping scheme to reduce the opportunity for third-parties to listen to the communication. As another example, the sensor 1810 can be configured to scan available connection points and prefer connections with those devices to which it has already connected, such as those devices which the sensor 1810 has previously established an authenticated exchange. Scanning for and connecting to known devices can reduce the opportunity for malicious third-parties to intersect an authentication exchange when other trusted data receiving devices are within communication range.

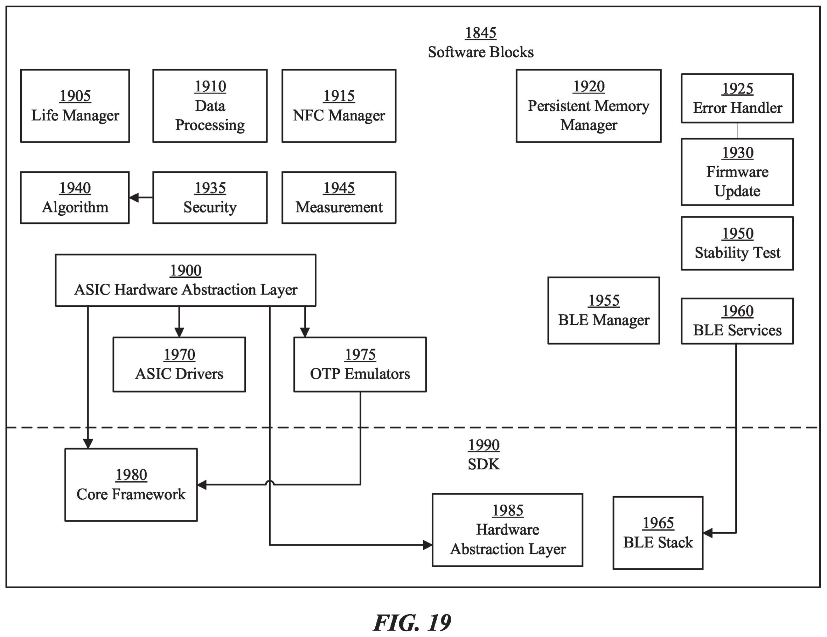

For purpose of illustration and not limitation, reference is made to the exemplary embodiment of the software 1845 configured for operation by the communication module 1840 as shown in FIG. 19. FIG. 19 illustrates an example hierarchical organization of the software blocks 1845 described herein with respect to exemplary embodiments. As embodied herein, all the software blocks can be installed on and run on the communication module 1840 (e.g., through the microcontroller 1843) alone, on the ASIC 1830 alone, and/or can be allocated between the communication module 1840 and ASIC 1830.

As embodied herein, the software blocks are installed on the communication module 1840, and the ASIC 1830 is configured without an embedded processor and without software or reprogrammable logic. As such, by installing and executing the software blocks on the communication module 1840, the sensor 1810 can leverage the processing power of communication module 1840 to reduce the cost of the ASIC 1830. The complexity of a suitable ASIC 1830 can be reduced such that the ASIC 1830 is configured to perform operations designated for it and no more. The footprint of the ASIC 1830 within the sensor 1810 can also be reduced based on the limited complexity and functionality. Additionally, because the ASIC 1830 does not necessarily need to be a general-purpose processor, the efficiency of the ASIC 1830, and the sensor 1810 overall, can be improved.

Additionally or alternatively, one or more software blocks can be installed on or otherwise implemented by the ASIC 1830 (not illustrated), such that the ASIC 1830 can be configured to have an embedded processor and reprogrammable logic and memory. As such, by installing and executing at least some of the software blocks on the ASIC 1830 (e.g., in addition to installing some software blocks on the communication module 1840), the sensor 1810 can leverage the added flexibility of the programmable ASIC 1830 and communication module 1840 to extend the usefulness of the sensor 1810 at least in part because the ASIC 1830 will not need to be replaced to add functionality to the ASIC 1830. For example, the ASIC 1830 can be updated through firmware or software updates (e.g., using a process similar to that described herein for updating the communication module 1840). Additionally, the ASIC 1830 in these embodiments can include a processor configured to perform more complex analyte detection and processing algorithms, and the sensor 1810 can use the combined processing capabilities of the ASIC 1830 and communication module 1840.

The overall architecture of the software blocks 1845 is shown in FIG. 19. The blocks below the dotted line designated SDK 1990, exist in read-only memory and are stereotyped as such. All other software blocks, which can be written by the manufacturer of the sensor 1810, are loaded into one-time programmable (OTP) memory of the communication module 1840.

The life manager software block 1905 can include software functions to manage the overall life operations of the sensor 1810. The software functions can include functions to update counter for the active operation time of the sensor 1810, manage sensor activation, insertion detection, update the sensor state, and update the sensor status. The data processing software block 1910 can include software functions to manage the data processing aspects of the sensor 1810. In particular, the communication module 1840 handles the bulk of the data processing of the sensor, offloading much of the data processing requirements of the sensor. The software functions can include functions to process raw data after posting, store historical data, and send current measurement data to an authenticated receiving device. The NFC manager 1915 can include software functions to hand operations related to sending and receiving NFC communications (e.g., through the ASIC 1830). The software functions can include functions to send data to the ASIC NFC radio 1835, receive data from ASIC NFC radio 1835, and handle received commands.

The persistent memory manager 1920 can include software functions to manage the storage of memory in the memory 1841 of the communication module 1840. For example, the persistent memory 1841 can store diagnostic data relating to the sensor 1810 as well as historical data of measurements recorded by the analyte sensing hardware 1855, interpreted by the ASIC 1830, and sent to the communication module 1840. The software functions can include functions relating to factory configuration of the sensor 1810, historical measurement data, sensor event logging, recording sensor state data, managing RAM used by the dynamic algorithm, manage access to and storage on various memory hardware including static and dynamic persistent RAM, dynamic system RAM, managing access to the one-time programmable memory, and providing for access to the persistent memory through an API. The error handler 1925 can include software functions, described in detail herein, to manage accounting for errors detected in the performance by the sensor 1810 (e.g., while recording measurements). The firmware update software block 1930 can include software functions to enable remote updating and upgrading of the firmware of the sensor 1810.

The security software block 1935 can include software functions to relating to the encryption and decryption of sensitive data stored by the communication module 1840, encryption and decryption of communications to and from the sensor 1810 (e.g., with a receiver 1820), and authentication of devices in communication. The security software block 1935 can also include functions relating to management of secure and public keys used by the sensor 1810. The algorithm software block 1940 can include software functions to process and interpret the raw measurement data reported by the analyte sensing hardware 1855 via the ASIC 1830. The software functions can include functions to process fast data (e.g., instantaneous measurements) and slow data (e.g., multiple measurement trends), data quality accessors, calculate current measurement results (e.g., instantaneous glucose measurements), calculate historical measurement results (e.g., historical glucose trends). The measurement software block 1945 can include software functions to directly managing the analyte sensing hardware 1855. The software functions can include a function to initiate and terminate measurement and configure the analog front end 1839 interfacing with the analyte sensing hardware 1855. The stability test software block 1950 can include software functions used to perform sensor testing during or after manufacture.

The communication module 1840 can include one or more medium-range communication radios 1848 (e.g., with a range longer than the NFC radio 1835 of the ASIC 1830). As an example, the communication module 1840 can include a Bluetooth radio or Bluetooth Low Energy (BLE) radio. The software blocks 1845 can include software to manage the communication radios. As an example, the software blocks 1845 can include a BLE manager 1955. The manager 1955 can include software functions relating to maintenance of the BLE radio, such as BLE configuration, sending to a receiver 1820, receiving data from a receiver 1820, receiving commands from the receiver 1820 (e.g., shutdown) and handling advertisements by which the communication module attempts to identify devices with which the communication module 1840 can attempt to establish connections. In particular, the parameters relevant to BLE connections can include transmitter power, connection interval, slave latency, and supervision timeout. The transmitter power parameter can be controlled. The sensor 1810 can use a BLE connection parameter update procedure to request a receiver 1820 to use a set of preferred connection parameters. After a receiver 1820 completes an authentication process, the sensor 1820 can set its preferred parameters to request for an update to the preferred connection interval, latency, and supervision timeout. This update procedure can reduce power consumption by maximizing the time the radio is in low power mode while maintaining the connection with a receiver 1820.

Similarly, the software blocks 1845 can include a BLE services software block 1960 with software functions to provide interfaces to make the BLE radio available to the computing hardware of the communication module 1840. These software functions can include a BLE logical interface and interface parser. BLE services offered by the communication module can include the generic access profile service, the generic attribute service, generic access service, device information service, data transmission services, and security services. The data transmission service can be a primary service used for transmitting data such as sensor control data, sensor status data, analyte measurement data (historical and current), and event log data. The sensor status data can include error data, current time active, and software state. As embodied herein, the sensor 1810 can be configured to send sensor status data to a connected receiver 1820 on initial authenticated connection to the receiver 1820, when the sensor status changes, or upon request from the receiver 1820. The analyte measurement data can include information such as current and historical raw measurement values (e.g., blood glucose, temperature, etc.), current and historical values after processing using an appropriate algorithm by the communication module 1840, projections and trends of measurement levels (e.g., trends of blood glucose levels or temperature levels, etc.), comparisons of other values to patient-specific averages, calls to action as determined by the algorithms of the communication module (e.g., so that the receiver acts a mere display device, while the communication module 1840 handles secured calculations) and other similar types of data.

The security services can be used to provide authentication to communicate with sensor 1810 operations. Authentication can include challenge and response commands, challenge data commands, security certificate data, and secured keys used therewith. The BLE stack 1965 can include additional software functions used by the BLE components of the communication module 1840.

An ASIC hardware abstraction layer 1900 can include software functions to enable the communication module to communicate with the ASIC 1830 via the serial-peripheral interface 300. As shown by the arrows connecting the ASIC hardware abstraction layer 1900 and other components, the ASIC hardware abstraction layer 1900 includes a variety of functions to enable the communication module 1840 to write to, read from, and control the ASIC 1830. For example, the ASIC hardware abstraction layer 1900 supports ASIC drivers 1970 exposing various functions of the ASIC 1830 to the communication module. The OTP emulators 1975 can include software functions to emulate and enable access of the communication module 1840 to the OTP memory 1831 of the ASIC 1830, for example, the internal RAM and serial memory.

The core framework 1980 can include functions underlying fundamental operation of the ASIC 1830. These functions include the necessary core operations allowing for the ASIC 1830 to execute, such as the processor scheduler, interrupt handler, memory manager, and timer utility. The hardware abstraction layer 1985 can include functions tying into the various low-level hardware components of the ASIC 1830. These functions includes functions relating to the boot sequencer, power manager, voltage monitor, and other hardware drivers.

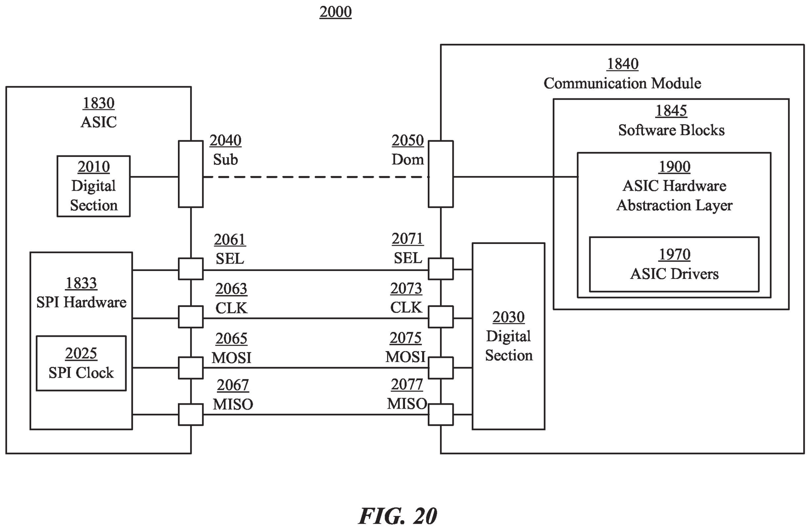

For purpose of illustration and not limitation, reference is made to the exemplary embodiment of a physical and logical serial-peripheral interface 300 between the ASIC 1830 and communication module 1840 for use with the disclosed subject matter as shown in FIG. 20. The SPI logical interface maps to the registers and register settings of the ASIC 1830. Certain registers of the ASIC 1830 are read-only. Additionally or alternatively, certain registers can be written as well as being read. Designated operations allow for the SPI dominant to read and write ASIC registers. Elements of the logical interface (and valid registers of the ASIC 1830) that are read only include interrupt requests. Elements of the logical interface (and valid registers of the ASIC 1830) that are write only include NFC response flags and error codes, NFC response valid payloads, and NFC response data. Elements of the logical interface (and valid registers of the ASIC 1830) that can be read and written include a unique identifier, calculated data, trimmed data, outlier status, temperature measurements, measurement status, and current measurement results.

As illustrated for example in FIG. 20, the ASIC hardware of the serial-peripheral interface 2000 includes an ASIC digital section 2010 and physical serial-peripheral interface hardware 1833. The SPI hardware 1833 can include an SPI clock 2025. The SPI hardware of the communication module includes a digital section 2030. In the illustrated embodiments, one of the ASIC 1830 and communication module 1840 is designated as the role of the SPI dominant and the other is designated as the SPI submissive. Particularly, the ASIC 1830 is the SPI submissive and has a submissive logical interface 2040. The communication module 1840 is the SPI dominant and has a dominant logical interface 2050. The submissive logical interface 2040 and dominant logical interface 2050 are connected via a logical connection. The SPI logical interface 2000 specifies the connection between the ASIC 1830 and the communication module 1840 at the logical register level. The ASIC Hardware Abstraction Layer 200 module of the software blocks 1845 forms messages with their parameters as the SPI dominant, and the ASIC digital section 2010 contains the registers and the logic to read and write them. As embodied herein, the SPI clock frequency is set to the maximum allowable by the ASIC 1830 (e.g., 3 MHz). FIG. 20 further illustrates the four wire physical SPI interface between the SPI hardware 1833 of the ASIC 1830 and the digital section 2030 of the communication module. The physical SPI interface includes a connection between the SEL pin 2061 of the ASIC 1830 and a counterpart 2071 of the communication module 1840, the CLK pin 2063 of the ASIC 1830 and a counterpart 2073 of the communication module 1840, the MOSI pin 2065 of the ASIC 1830 and a counterpart 2075 of the communication module 1840, and the MISO pin 2067 of the ASIC 1830 and a counterpart 2077 of the communication module 1840. For purpose of illustration and not limitation, exemplary sequences of read and write messages suitable to accomplish various functionality and use case scenarios of the system are described herein with reference to the corresponding diagrams.

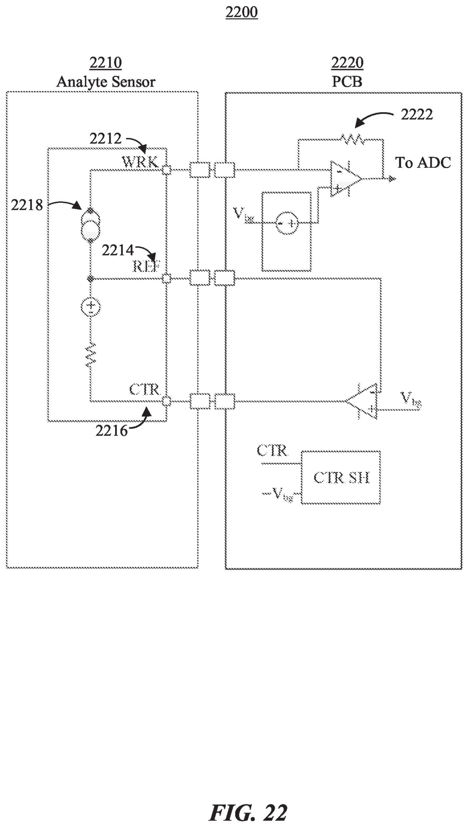

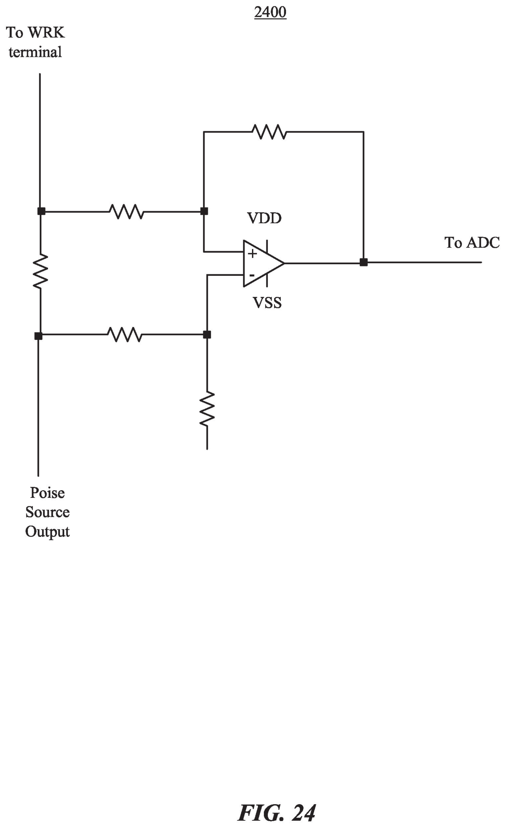

Additionally or alternatively, diagnostics can be derived based on the analog front end 1839 of the ASIC 1830 monitoring the current and voltage read from various electrodes of the analyte sensing hardware 1855 (e.g., an analyte sensor). FIG. 22 illustrates an example diagram 2200 of the analog front end 1839 illustrating functions for the analog front end 1839 between the analyte sensor 2210 and the PCB 2220 of the sensor 1810. As embodied herein, FIG. 22 illustrates the working electrode 2212, reference electrode 2214, and counter electrode 2216, with the analyte sensing element 2218 between the working electrode 2212 and reference electrode 2214. The counter electrode is driven with a bandgap reference voltage V_{bg} by the analog front end 1839 which is then adjusted before being provided to the reference electrode 2214. The analyte sensing element 2218 provides additional voltage to the working electrode 2212. The difference between the voltage at the working electrode 2212 and the voltage at the reference electrode 2214, referred to as the poise voltage, is used to determine the amount of analyte present. The voltage at the reference electrode 2214 is also monitored. Between each of the working electrode 2212, reference electrode 2214, and counter electrode 2216 can be one or more additional sources of resistance (not illustrated) such as the resistance of the trace that connects the sensor electrodes to the connection point with the sensor connector and the resistance from the PCB connector to the electronics. The PCB can include a transimpedance amplifier 2222 which can include a programmable offset from the bandgap reference voltage V_{bg}. The transimpedance amplifier can convert the current signal from the working electrode into voltage at output, however, the common mode voltage (e.g., WRK) can be removed to determine the true measurement. In alternative embodiments, a difference amplifier 2400, such as that illustrated in FIG. 24 can be used. Note that with the difference amplifier 2400 a precise matching between the resistors can be applied for it to function.

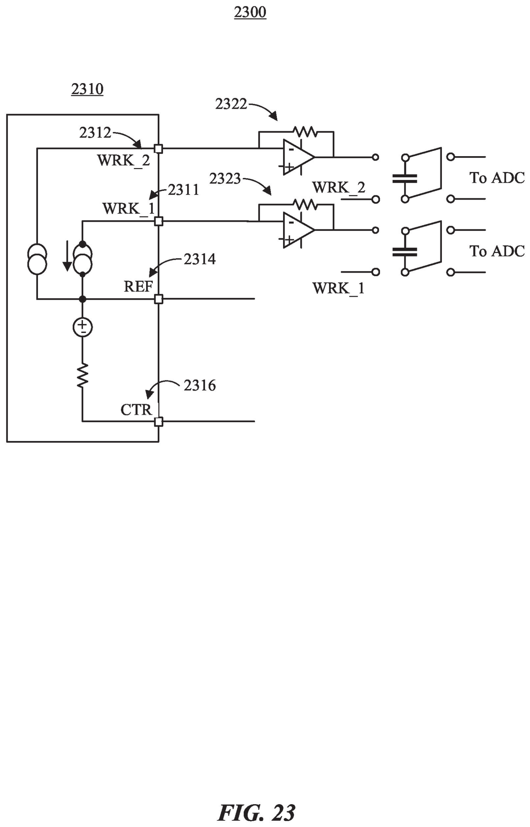

In an alternative embodiment, the sensor 1810 can include multiple working electrodes. FIG. 23 illustrates an example of a configuration of the sensor 1810. As embodied herein, FIG. 23 illustrates an example circuit 2300 including an analyte sensor 2310 with a first working electrode current 2311 (WRK_1) and a second working electrode current (2312 (WRK_2). The analyte sensor 2310 further includes a reference electrode 2314 (REF) and counter electrode 2316 (CTR) similarly situated as in the analyte sensor 2210. The current from each of the working electrodes can be fed to a transimpedance amplifier 2322 and 2323, as illustrated in FIG. 22. Each transimpedance amplifier can include an independently programmable offset from the bandgap reference voltage V_{bg}. The transimpedance amplifiers can convert the current signal from the working electrode into voltage at output. In alternative embodiments, a difference amplifier, such as at illustrated in FIG. 24 can be used, however, the common mode voltage (e.g., WRK) can be removed to determine the true measurement. In alternative embodiments, one or more difference amplifiers, such as the difference amplifier 2400 illustrated in FIG. 24 can be used.

As embodied herein, the analog front end 1839 can be configured to monitor the current and voltage from at least the working electrode (e.g., working electrode 2212) and counter electrode (e.g., counter electrode 2216). The analog front end 1839 can monitor for a low working current. The analog front end 1839 or ASIC 1830 can be configured with a minimum working current threshold for the sensor 1810 to continue to operate after an analyte sensor has been inserted into a patient. Additionally, the analog front end 1839 can particularly monitor for a low working current for a predefined period of time after user insertion of the sensor. In addition to monitoring the working current for the minimum working current threshold, the analog front end 1839 and ASIC 1830 can monitor for working current values within a threshold range of a target working current value. The analog front end 1839 can further monitor for a high working current. The analog front end 1839 or ASIC 1830 can be configured with a maximum working current threshold that corresponds to a value below analyte sensor saturation, the point at which sensor values cannot be taken as accurate. The analog front end 1839 can further monitor for a low counter electrode voltage. In particular, the analyte sensor voltage between the working electrode and counter electrode can be maintained by a servo amplifier that adjusts the voltage at the counter electrode, for example, in response to negative feedback from a reference electrode. As the conditions of the analyte sensor change, the counter electrode voltage automatically adjusts to maintain the reference voltage at a predetermined amount, which can be used to maintain the target poise voltage within a suitable range. The analog front end 1839 or ASIC 1830 can be configured with a minimum counter voltage threshold for the analyte sensor to continue to operate properly. The analog front end 1839 can further monitor for a high counter electrode voltage. In particular, because the analyte sensor works in part by measuring voltage differences between the working electrode and counter electrode, for the analyte sensor to function properly, the voltage from the counter electrode can be no higher than the working electrode. The analog front end 1839 or ASIC 1830 can be configured with an appropriate maximum counter voltage threshold. These analog front end diagnostic checks can be used to assess analyte sensor failure modes, including sensor connection problems.

The poise voltage can be maintained using a hardware-enforced or programmable poise voltage source. The purpose of the programmable poise voltage source is to bias the difference between the voltage at working electrodes and reference electrodes to ensure that the measurement range is within a target range that is suitable for analyte measurement. To change the poise voltage with a hardware-enforced poise voltage source, the resistor networks that set the poise voltage had to be recalculated to the new intended poise voltage and change. As such, to change the poise voltage, the board is disassembled and the resistors swapped out for new values, which can be inconvenient, for example if resistors having suitable values are unavailable. A programmable poise voltage thus allows for the sensitivity analyte sensor to be adjusted according to developing conditions over the lifespan of the analyte sensor. For example, the circuit which generates the poise voltage can be controlled by a microprocessor which can set the poise voltage by outputting the necessary voltage via the DAC pin of the microprocessor. This allows for fast and automated calibration of the poise voltage. DAC settings can be optimized with software routines and stored for later usage.

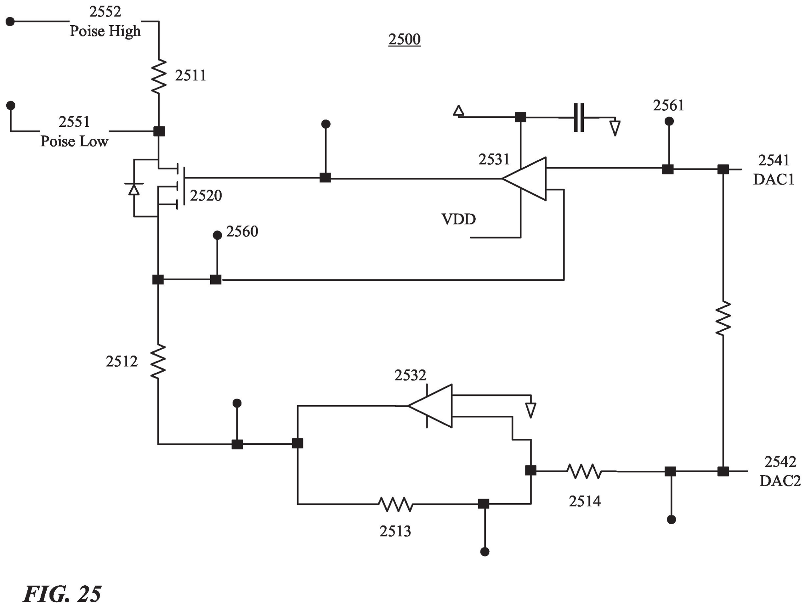

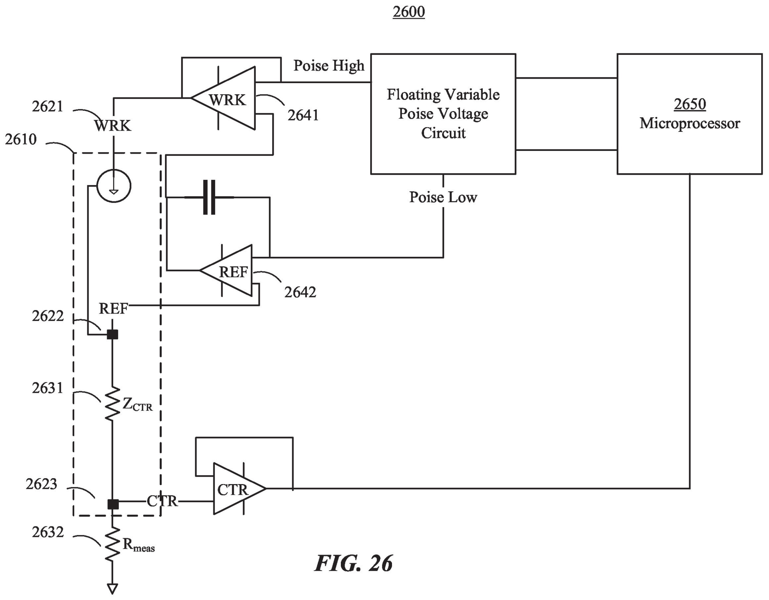

FIG. 25 illustrates an example circuit that can used as a variable floating poise voltage generator. The circuit 2500 as shown in FIG. 25 uses four resistors 2511, 2512, 2513, and 2514, an N-channel MOSFET 2520, and two op-amps 2531 and 2532, one acting as a current source 2531 and the other acting as an inverting amplifier 2532. The poise voltage is set with the DAC1 2541 and DAC2 2542 pins from the microprocessor. Op-amp 2532 will invert the voltage from DAC2 2542 into a voltage equal to the negative of DAC2 2542. Op-amp 2531 is configured as a current source that is programmed with the positive input of the op-amp. The difference between "poise high" 2551 and "poise low" 2552 is the poise voltage and is controlled by the current flowing through resistor 2511 (as illustrated in FIG. 26). The voltage at "Poise High" 2552 is driven by an amplifier shown in FIG. 26 that also controls the voltage at the working electrode of the sensor. The op-amp 2531 and MOSFET 2520 will control the voltage at 2560 so that it is equal to the voltage at 2561. The voltage across resistor 2512 is controlled by the settings for DAC1 2511 and DAC2 2512. The current flowing through resistor 2512 is the same as the current flowing through resistor 2511. In this way the poise voltage can be controlled independent of the voltage at WRK. With this circuit, if the voltage at WRK is adjusted due to change in sensor current, the poise voltage will remain unchanged even as the voltage at WRK changes.

FIG. 26 illustrates an example configuration of a sensor in a measurement configuration with a floating variable poise voltage circuit (e.g., the circuit 2500). The poise voltage is measured from the WRK electrode and the REF electrode and will be equal to the voltage measured from Poise High and Poise Low. The analyte sensor 2510 is a sensor, as described herein, with chemistry to react to the analyte sensor during use.

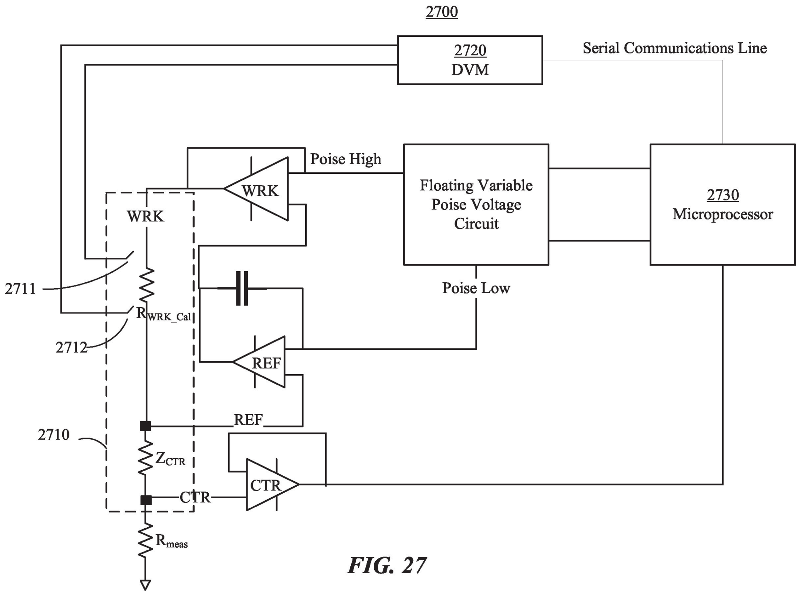

FIG. 27 illustrates an example configuration 2700 of a sensor in a calibration configuration with a floating variable poise voltage circuit (e.g., the circuit 2500). During calibration a simulated analyte sensor 2710 can be used with two resists 2711 and 2712 as shown in FIG. 27. A digital voltmeter (DVM) 2720 will measure the voltage between WRK and REF and feed the measurement back to the microprocessor 2730 to adjust DAC1 and DAC2 and get the poise voltage to the desired value. In the sensor measurement configuration, the poise voltage will have previously been set during calibration and the WRK op-amp will provide the current across resistor 2511 of FIG. 25.

When an analyte sensor 2610 is attached to the circuit 2600 in FIG. 26, the sensor portion connected between the WRK 2621 and REF 2621 will generate a current when it comes in contact with the analyte. The variable poise voltage can allow the sensor to detect a variety of analyte, by adjusting the reactive current in both positive and negative domains. The current will flow down through Z_{CTR} 2631 and Rₘₑₐₛ 2632. The voltage at the CTR electrode 2623 will vary depending on the current generated by the analyte sensor 2610 and therefore the voltage at the REF electrode 2622 will vary according to the current going through Rₘₑₐₛ 2632 and Z_{CTR} 2631. As such, a floating poise voltage is provided. The feedback loop of the REF op-amp 2642 includes the WRK op-amp 2642 and resistor 2511. The voltage output of the REF op-amp 2642 goes to the positive terminal of the WRK op-amp 2641 which will cause the output to adjust the negative terminal of the WRK op-amp 2641 to match the positive terminal, the feedback loop of the REF op-amp 2642 continues through resistor 2511, which will have voltage equal to the digitally set poise voltage. It is due to this feedback loop that the voltage at the REF electrode 2622and WRK electrode 2621 will differ by the voltage across resistor 2511, which is the poise voltage set and calibrated by the microprocessor 2650.

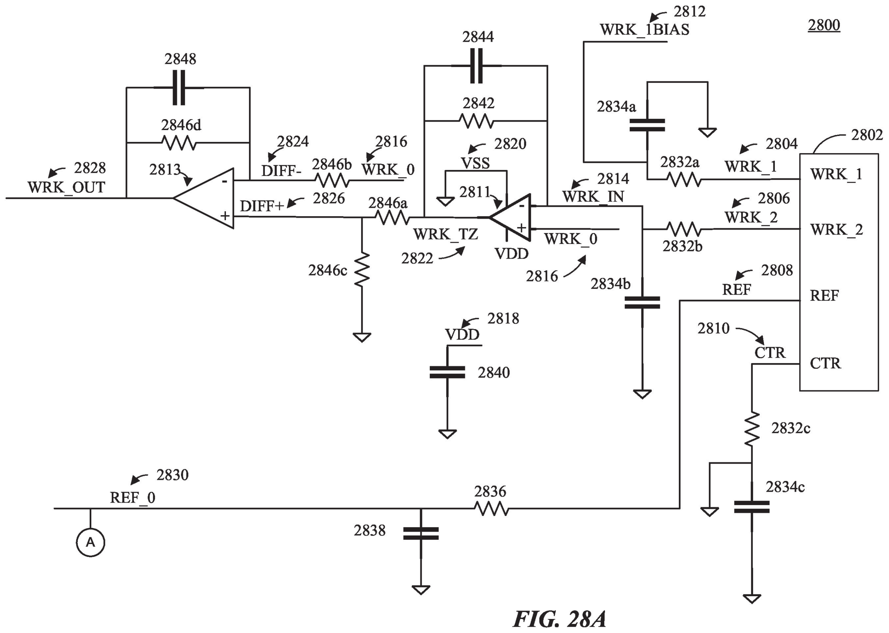

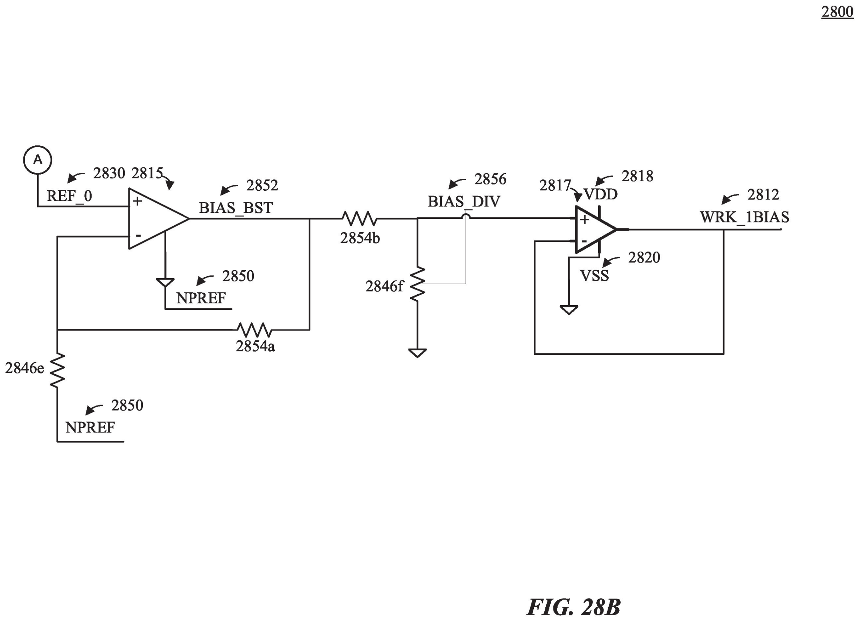

FIGS. 28-30 illustrate exemplary configurations of a circuit configured to sample signals from a multiple analyte sensor. The circuit 2800 illustrated in FIGS. 28A-28B includes a multiple analyte sensor 2802, 13 resistors 2832a-2832c, 2836, 2842, 2846a-2846f, and 2854a-2854b, seven capacitors 2834a-2834c, 2838, 2840, 2844, and 2848, op-amps 2811, 2813, 2815, and 2817. The circuit 2800 can be used to measure glucose levels. The resistance of resistors 2832a-2832c can be R1, resistor 2836 can be R2, resistor 2842 can be R3, resistors 2846a-2846f can be R4, and resistors 2854a-2854b can be R5. Any of the resistors can be different values based on the circuit 2800. The capacitance of the capacitors 2834a-2834c can be C1, capacitor 2838 can be C2, capacitor 2840 can be C3, capacitor 2844 can be C4, and capacitor 2848 can be C5. Any of the capacitors can be different values based on the circuit 2800.

As embodied herein, referring still to FIGS. 28A-28B, the multiple analyte sensor 2802 includes a first working electrode 2804 (WRK_1), a second working electrode 2806 (WRK_2), a reference electrode 2808 (REF), and counter electrode 2810 (CTR). A pair of one of resistors 2832a-2832c and one of capacitors 2834a-2834c are coupled to each of the first working electrode current 2804, the second working electrode current 2806, and the counter electrode 2810 for electrostatic discharge (ESD)/ electromagnetic interference (EMI) rejection. The resistor 2836 and capacitor 2838 are coupled to the reference electrode 2808 for ESD/EMI rejection. The first working electrode bias 2812 can be determined from a negative poise bias amplifier described herein as shown in FIG. 28B. The first working electrode bias 2812 can be the reference electrode 2808 - a predetermined voltage. The predetermined voltage is based on the test platform of the circuit 2800.

With continued reference to FIGS. 28A-28B, op-amp 2811, resistor 2842, and capacitor 2844 are configured as a transimpedance amplifier. The transimpedance amplifier receives a work in signal 2814 from the second working electrode 2806 and a bias offset Vbias (e.g., WRK_0 2816). WRK_0 2816 can be the reference electrode 2808 - a predetermined voltage. The predetermined voltage is based on the test platform of the circuit 2800. The transimpedance amplifier converts the current from the second working electrode 2806 to voltage to get the bias offset + current from the second working electrode 2806 * resistance of the transimpedance amplifier. For instance, the output of the transimpedance amplifier can be Vbias + I_WRK(x) * Rtz, which is work signal of the transimpedance amplifier WRK_TZ 2822. The time constant of the transimpedance amplifier can be R3 * C4 = T1.

As embodied herein, the output of the transimpedance amplifier is input to a differential amplifier configured from resistors 2846a-2846d, capacitor 2848, and op-amp 2813. The differential amplifier subtracts the bias offset (Vbias) from output of the transimpedance amplifier, WRK_TZ 2822, to obtain the current from the second working electrode 2806 * the resistance of the transimpedance amplifier (e.g., I_WRK(x) * Rtz), which is used as the input to the analog to digital converter. The input DIFF+ 2826 is WRK_TZ 2822 divided by 2 and the input DIFF- 2824 is the bias offset, Vbias, through a summing resistor, resistor 2846b. The output of the differential amplifier is work out (WRK_OUT 2828). For instance, WRK_OUT 2828 can be (WRK_TZ 2822 / 2) * (1 + R4 / R4) - (R4 / R4) * Vbias, where Vbias is WRK_0 2816. The high resistance of resistors 2846a-2846f are used to minimize the loading on WRK_0 2816 or Vbias. The time constant of the differential amplifier can be T2.

With reference to FIG. 28A, as embodied herein, REF_0 2830 can be coupled to a negative poise bias amplifier configured from resistors 2846e-2846f, 2854a-2854b, and op-amps 2815 and 2817. Referring to FIG. 28B, a negative poise bias amplifier is shown, where the circuit subtracts a predetermined voltage from REF 2808 to bias the first working electrode 2804 directly. One input into the negative poise bias amplifier is REF_0 2830 connected to the "+" input of op-amp 2815 and the other input is the negative poise reference NPREF 2850, which is summed in to the inverting input of the op-amp 2815. The resistor ratio of resistors 2846e and 2854a, k = R4 / R5 = resistor ratio, which determines the poise subtracted from REF 2830 as NPREF 2850 / (k + 1). For a nominal NPREF 2850 of V1, this gives V2 subtracted from REF 2830. The output of op-amp 2815 is a slightly boosted REF 2830 plus bias signal, BIAS_BST 2852. Op-amp 2817 is a unity gain buffer. The output of op-amp 2817 is first working electrode bias WRK1_BIAS 2812, which is a slightly divided down version of BIAS_BST 2852, called BIAS_DIV 2856. For instance, BIAS_DIV 2856 = WRK1_BIAS 2812 = k * BIAS_BST 2852 / (k + 1) = REF 2830 - k * NPREF 2850 / (k + 1).

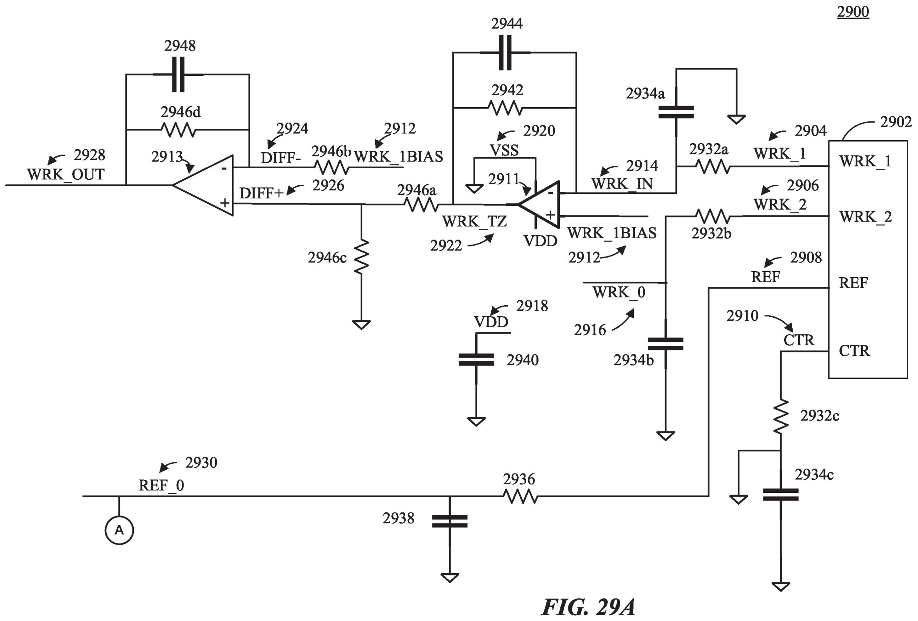

Referring now to FIGS. 29A-29B, as embodied herein, an exemplary circuit 2900 includes a multiple analyte sensor 2902, 13 resistors 2932a-2932c, 2936, 2942, 2946a-2946f, and 2954a-2954b, seven capacitors 2934a-2934c, 2938, 2940, 2944, and 2948, op-amps 2911, 2913, 2915, and 2917. The circuit 2900 can be used to measure ketone levels. The resistance of resistors 2932a-2932c can be R1, resistor 2936 can be R2, resistor 2942 can be R3, resistors 2946a-2946f can be R4, and resistors 2954a-2954b can be R5. Any of the resistors can be different values based on the circuit 2900. The capacitance of the capacitors 2934a-2934c can be C1, capacitor 2938 can be C2, capacitor 2940 can be C3, capacitor 2944 can be C4, and capacitor 2948 can be C5. Any of the capacitors can be different values based on the circuit 2900.

With reference to FIGS. 29A-29B, the multiple analyte sensor 2902 includes a first working electrode 2904 (WRK_1), a second working electrode 2906 (WRK_2), a reference electrode 2908 (REF), and counter electrode 2910 (CTR). A pair of one of resistors 2932a-2932c and one of capacitors 2934a-2934c are coupled to each of the first working electrode current 2904, the second working electrode current 2906, and the counter electrode 2910 for electrostatic discharge (ESD)/ electromagnetic interference (EMI) rejection. The resistor 2936 and capacitor 2938 are coupled to the reference electrode 2908 for ESD/EMI rejection.

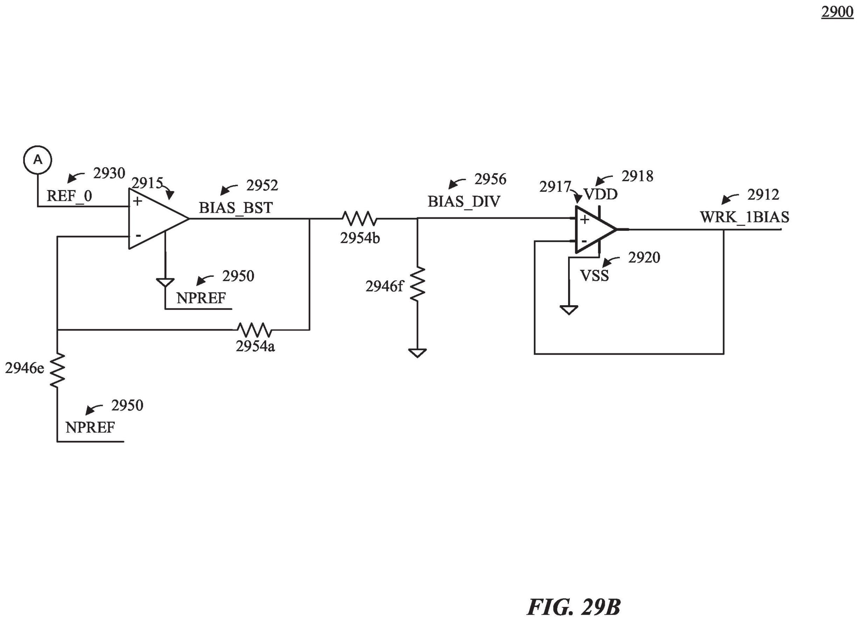

As embodied herein, the first working electrode bias 2912 can be determined from a negative poise bias amplifier described herein as shown for example in FIG. 29B. The first working electrode bias 2912 can be the reference electrode 2908 - a predetermined voltage. The predetermined voltage is based on the test platform of the circuit 2900. Op-amp 2911, resistor 2942, and capacitor 2944 are configured as a transimpedance amplifier. The transimpedance amplifier receives a work in signal 2914 from the first working electrode 2904 and a bias offset Vbias (e.g., WRK_1Bias 2912). The transimpedance amplifier converts the current from the first working electrode 2904 to voltage to get the bias offset + current from the first working electrode 2904 * resistance of the transimpedance amplifier. For instance, the output of the transimpedance amplifier can be Vbias + I_WRK(x) * Rtz, which is the work signal of the transimpedance amplifier, WRK_TZ 2922. The time constant of the transimpedance amplifier can be R3 * C6 = T3.

With continued reference to FIGS. 29A-29B, the output of the transimpedance amplifier, WRK_TZ 2922 is input to a differential amplifier configured from resistors 2946a-2946d, capacitor 2948, and op-amp 2913. The differential amplifier subtracts the bias offset (Vbias) from the output of the transimpedance amplifier, WRK_TZ 2922, to obtain the current from the first working electrode 2904 * the resistance of the transimpedance amplifier (e.g., I_WRK(x) * Rtz), which is used as the input to the analog to digital converter. The input DIFF+ 2926 is WRK_TZ 2922 divided by 2 and the input DIFF- 2924 is the bias offset, Vbias, through a summing resistor, resistor 2946b. The output of the differential amplifier is work out (WRK_OUT 2928). For instance, WRK_OUT 2928 can be (WRK_TZ 2922 / 2) * (1 + R4 / R4) - (R4 / R4) * Vbias, where Vbias is WRK_1Bias 2912. The high resistance of resistors 2946a-2946f are used to reduce or minimize the loading on WRK_1Bias 2912 or Vbias. The time constant of the differential amplifier can be T1. In FIG. 29A, REF_0 2930 can be coupled to a negative poise bias amplifier configured from resistors 2946e-2946f, 2954a-2954b, and op-amps 2915 and 2917.

Referring to FIG. 29B, as embodied herein, a negative poise bias amplifier is shown, where the circuit subtracts a predetermined voltage from REF 2908 to bias the first working electrode 2904 via the transimpedance amplifier. One input into the negative poise bias amplifier is REF_0 2930 connected to the "+" input of op-amp 2915 and the other input is the negative poise reference NPREF 2950, which is summed in to the inverting input of the op-amp 2915. The resistor ratio of resistors 2946e and 2954a, k = R4 / R5 = resistor ratio, which determines the poise subtracted from REF 2930 as NPREF 2950 / (k + 1). For a nominal NPREF 2950 of V1, this gives V2 subtracted from REF 2930. The output of op-amp 2915 is a slightly boosted REF 2930 plus bias signal, BIAS_BST 2952. Op-amp 2917 is a unity gain buffer. The output of op-amp 2917 is first working electrode bias WRK1_BIAS 2912, which is a slightly divided down version of BIAS_BST 2952, called BIAS_DIV 2956. For instance, BIAS_DIV 2956 = WRK1_BIAS 2912 = k * BIAS_BST 2952 / (k + 1) = REF 2930 - k * NPREF 2950 / (k + 1).

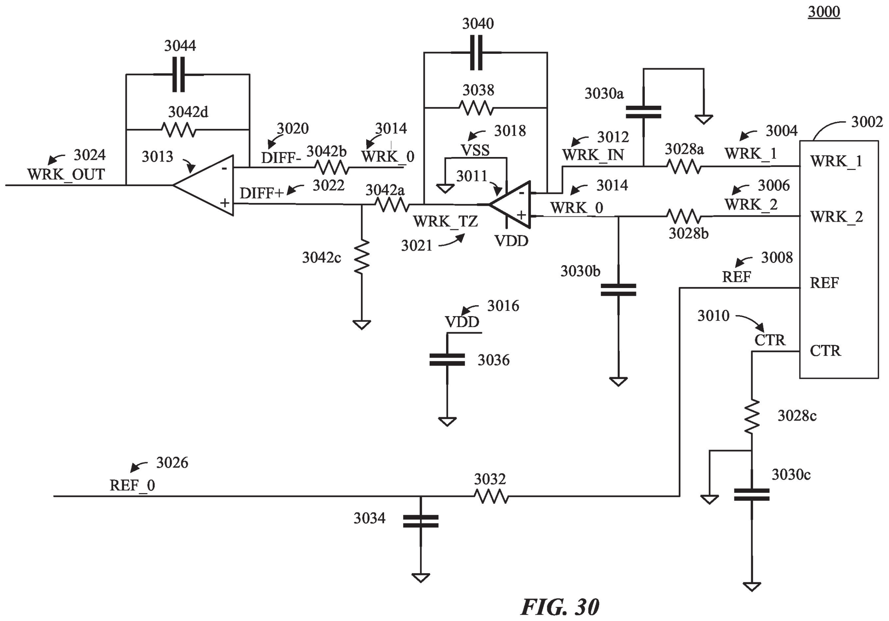

Referring now to FIG. 30, an exemplary circuit 3000 includes a multiple analyte sensor 3002, nine resistors 3028a-3028c, 3032, 3038, and 3042a-3042d, seven capacitors 3030a-3030c, 3034, 3036, 3040, and 3044, op-amps 3011 and 3013. The circuit 3000 can be used to measure lactate levels. The resistance of resistors 3028a-3028c can be R1, resistor 3032 can be R2, resistor 3038 can be R3, and resistors 3042a-3042d can be R4. Any of the resistors can be different values based on the circuit 3000. The capacitance of the capacitors 3030a-3030c can be C1, capacitor 3034 can be C2, capacitor 3036 can be C3, capacitor 3040 can be C4, and capacitor 3044 can be C5. Any of the capacitors can be different values based on the circuit 3000.

As embodied herein. the multiple analyte sensor 3002 includes a first working electrode 3004 (WRK_1), a second working electrode 3006 (WRK_2), a reference electrode 3008 (REF), and counter electrode 3010 (CTR). A pair of one of resistors 3028a-3028c and one of capacitors 3030a-3030c are coupled to each of the first working electrode current 3004, the second working electrode current 3006, and the counter electrode 3010 for electrostatic discharge (ESD)/ electromagnetic interference (EMI) rejection. The resistor 3032 and capacitor 3034 are coupled to the reference electrode 3008 for ESD/EMI rejection. Op-amp 3011, resistor 3038, and capacitor 3040 are configured as a transimpedance amplifier. The transimpedance amplifier receives a work in signal 3012 from the first working electrode 3004 and WRK_0 3014 signal from the second working electrode 3006. The transimpedance amplifier converts the current from the first working electrode 3004 to voltage to get the bias offset + current from the first working electrode 3004 * resistance of the transimpedance amplifier. For instance, the output of the transimpedance amplifier can be Vbias + I_WRK(x) * Rtz, which is the work signal of the transimpedance amplifier, WRK_TZ 3021. The time constant of the transimpedance amplifier can be R3 * C4 = T4.

Referring still to FIG. 30, as embodied herein. the output of the transimpedance amplifier, WRK_TZ 3021 is inputted into a differential amplifier configured from resistors 3042a-3042d, capacitor 3044, and op-amp 3013. The differential amplifier subtracts the bias offset (Vbias or WRK_0 3014) from the output of the transimpedance amplifier, WRK_TZ 3021, to obtain the current from the first working electrode 3004 * the resistance of the transimpedance amplifier (e.g., I_WRK(x) * Rtz), which is used as the input to the analog to digital converter. The input DIFF+ 3022 is WRK_TZ 3021 divided by 2 and the input DIFF- 3020 is the bias offset, Vbias, through a summing resistor, resistor 3042b. The output of the differential amplifier is work out (WRK_OUT 3024). For instance, WRK_OUT 3024 can be (WRK_TZ 3021 / 2) * (1 + R4 / R4) - (R4 / R4) * Vbias, where Vbias is WRK_0 3014. The high resistance of resistors 3042a-3042d are used to minimize the loading on Vbias. The time constant of the differential amplifier can be T5.

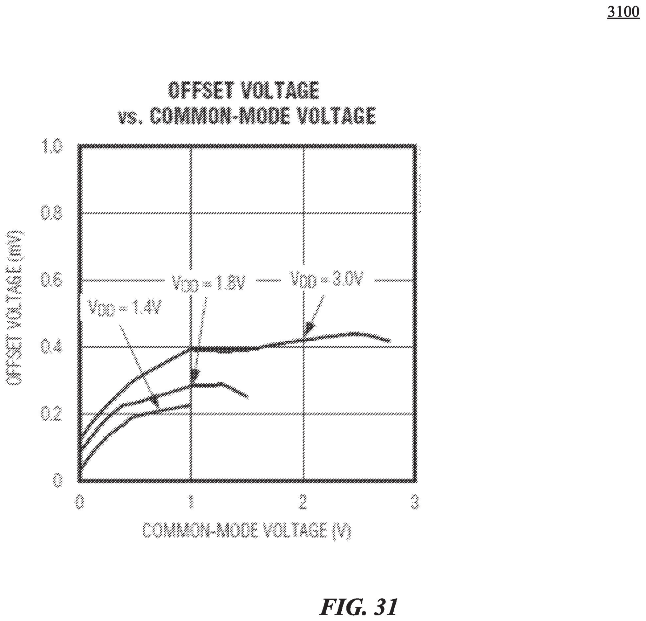

For purpose of illustration and not limitation, as embodied herein, the circuits 2800, 2900, and 3000 can utilize the respective differential amplifiers to subtract the bias offset from the transimpedance amplifier output to reduce the signal into a range of an A/D converter. However, after the subtracting the bias offset from the transimpedance amplifier output, there can be a bias voltage-dependent residual, which can be beneficial to calibrate out. The calibration can involve at least a two-point offset calibration for the WRK(X) channel. A common-mode op-amp characteristic can use a three-point calibration. As illustrated for example in FIG. 31, the three-point calibration can result in a linear fit from .2V to 1V and another linear fit from 1V to 2V as shown in graph 3100.

The calibration and work current equations can include various parameters, including, without limitation, CO (counter offset, zero current input), CS (counter "slope", which is not slope but high cal point with current input), REFR and Thermistor cal parameters, calibration parameters for common mode offsets, and current calibration parameters for slope. The calibration parameters for common mode offsets can include WRKO channel (internal A/D input) Common Mode Voltage (NW0cm1, NW0cm2, NW0cm3) and WRK(X) Channel ("CNTR" A/D input) Offsets (NWxos1, NWxos2, and NWxos3). The current calibration parameters for slope (i.e., A/D counts for slope cal current input) can include WRK(X) Channel ("CNTR" A/D input) (NWxcurr). The three-Point Offset Calibration Equations can include sensor offset cal slope equations, run-time sensor offset equations, sensor current cal slope equation, and run-time sensor current equation. Sensor offset cal slope equations can include MWxos1 = (NWxos2 - Nwxos1) / (NW0cm2 - NW0cm1), where WRK(X) offset slope 1 (delta WRK(X) counts) / (delta WRKO counts). Sensor offset cal slope equations can include MWxos2 = (NWxos3 - Nwxos2) / (NW0cm3 - NW0cm2), where WRK(X) offset slope 2 (delta WRK(X) counts) / (delta WRKO counts). Run time sensor offset equations can include, for NW0cm < NW0cm2 (for WRKO A/D reading < WRKO Cal Pt #2), NWxos = NWxos2 + MWxos1 * (NW0cm - NW0cm2), WRK(X) offset along WRK(X) os line 1 with slope 1. Run time sensor offset equations can include, for NW0cm >= NW0cm2 (for WRKO A/D reading >= WRKO Cal Pt #2), NWxos = NWxos2 + NWxos2 * (NW0cm-NW0cm2), where WRK(X) offset along WRK(X) os line 2 with slope 2. For run time sensor offset equations cal point NWxos2 can be common to both line 1 and line 2. The sensor current cal slope equations can include MWxcurr = IWRK(X)_CAL / (NWXcurr - Nwxos2), where WRK(X) Sensor current slope (pA/count). The sensor current cal slope equations can include IWRK(X)_CAL = 11 for circuit 1200. The sensor current cal slope equations can include IWRK(X)_CAL = I2 for circuits 1100, 3000. The run-time sensor current equation can include IWRK(X) = MWxcurr * (NWRK(X) - Nwxos).

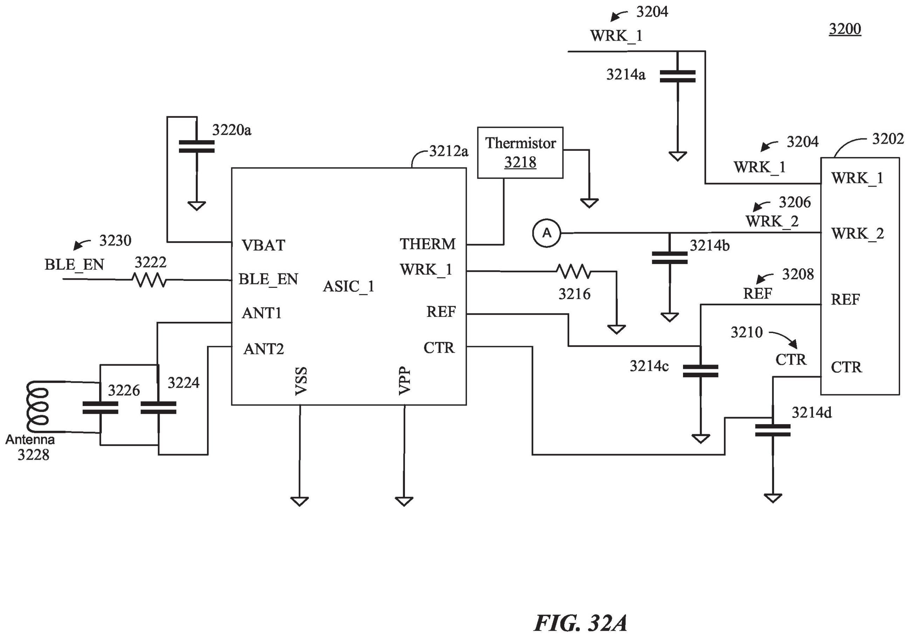

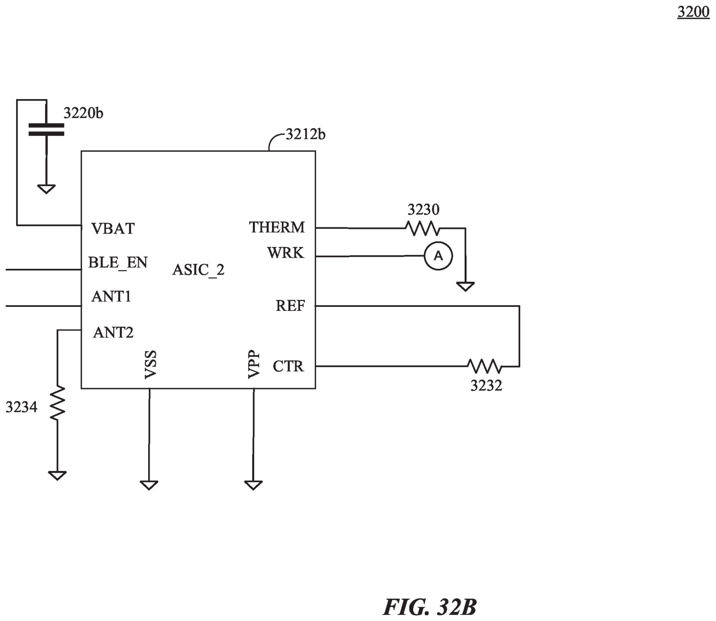

FIGS. 32-33 illustrate exemplary configurations of a circuit configured to sample signals from a multiple analyte sensor using an ASIC. The circuit 3200 illustrated in FIGS. 32A-32B includes a multiple analyte sensor 3202, two ASICs 3212a-3212b, eight capacitors 3214a-3214d, 3220a-3220b, 3224, 3226, five resistors 3216, 3222, 3230, 3232, 3234, thermistor 3218, and antenna 3228. The circuit 3200 can be used to measure ketone levels, lactose levels, and glucose levels. In particular embodiments, the ASIC_1 3212a can be used to measure ketone levels or lactose levels, where resistor 3216 can be loaded to measure for ketone levels. In particular embodiments, the ASIC_2 3212b can be used to measure glucose levels. The capacitance of the capacitors 3214a-3214d can be C6, capacitors 3220a-3220b can be C7, the capacitor 3224 can be C8, and the capacitor 3226 can be C9. Any of the capacitors can be different values based on the circuit 3200. The resistance of the resistor 3216 can be R6, resistor 3222 can be R7, resistor 3230 can be R2, resistor 3232 can be R8, resistor 3234 can be R9. Any of the resistors can be different values based on the circuit 3200.

As embodied herein, referring still to FIGS. 32A-32B, the multiple analyte sensor 3202 includes a first working electrode 3204 (WRK_1), a second working electrode 3206 (WRK_2), a reference electrode 3208 (REF), and counter electrode 3210 (CTR). Capacitors 3214a-3214d can be coupled to each of the first working electrode current 3204, the second working electrode current 3206, the reference electrode 3208, and the counter electrode 3210. ASIC_1 3212a can include a plurality of pins. In particular embodiments, ASIC_1 3212a can include a therm pin, a first working electrode 3204 (WRK_1) pin, a reference pin, a counter pin, a VSS pin, a VPP pin, a VBAT pin, a BLE_EN pin, an ANT1 pin, and an ANT2 pin. In particular embodiments, the ASICs 3212a, 3212b can have ESD protection of +/-2KV HBM for each pin of the ASICs 3212a, 3212b. In low leakage inputs, the ASICs 3212a, 3212b can have +/-500V HBM for each pin of the ASICs 3212a, 3212b. The first working electrode 3204 can be coupled to the working electrode pin of ASIC_1 3212a through resistor 3216. The reference electrode 3208 can be coupled to a reference pin of ASIC_1 3212a. The counter electrode 3210 can be coupled to the counter pin of ASIC_1 3212a. The second working electrode 3206 can be coupled to a working electrode pin of ASIC_2 3212b as shown in FIG. 32B. The therm pin of ASIC_1 3212a can be coupled to thermistor 3218. The VBAT pin can be coupled to capacitor 3220a. The BLE_EN pin can be coupled to resistor 3222 and coupled to BLE_EN 3230. The ANT1 pin and ANT 2 pin can be coupled to two capacitors 3224, 3226 and antenna 3228 in a particular configuration. Although a specific configuration is shown in circuit 3200, other configuration of components of 3200 can be contemplated. ASIC_2 3212b can include similar pins as ASIC_1 3212a. The therm pin of ASIC_2 3212b can be coupled to a resistor 3230. The reference pin of ASIC_2 3212b can be coupled to the counter pin through a resistor 3232. The VBAT pin of ASIC_2 3212b can be coupled to a capacitor 3220b. The ANT2 pin of ASIC_2 3212b can be coupled to a resistor 3234.

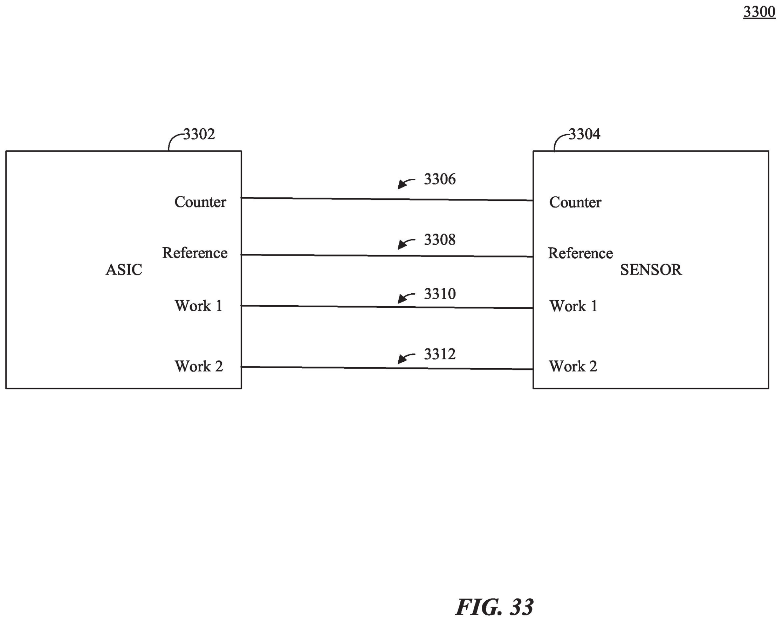

Referring to FIG. 33, a circuit 1600 is shown. The circuit 1600 illustrated in FIG. 33 can include an ASIC 1602 and a sensor 1604. The ASIC 1602 can have a counter pin, a reference pin, a first working electrode pin, and a second working electrode pin. The sensor 1604 can include a counter output 1606 coupled to the counter pin of the ASIC 1602, a reference output 1608 coupled to the reference pin of the ASIC 1602, a first working electrode output 1610 coupled to the first working electrode pin of the ASIC 1602, and a second working electrode output 1612 coupled to the second working electrode pin of the ASIC 1602.

The multiple analyte sensors 3202, 1604 as described in circuits 3200, 1600 can be a four-terminal multiple-analyte electrochemical biosensor. The sensor 3202, 1604 can be exposed to body interstitial fluid and biased correctly to produce two independent current flows from the corresponding first working electrode 3204, 1610 and the corresponding second working electrode 3206, 1612 to its corresponding counter electrode 3210, 1606. The power source of the circuits 3200, 1600 can be a battery. As an example and not by way of limitation, the power source of the circuits 3200, 1600 can be a 1.55V high-drain silver oxide battery. The potassium electrolyte can provide for a low equivalent series resistance (ESR). This can help support the current draw during Bluetooth Low Energy (BLE) transmissions. The ASICs 3212a-3212b, 1602 can perform the analyte, temperature, and diagnostic measurements and incorporate an NFC radio. The NFC radio can be used for the user to activate the patch. In particular embodiments, the NFC radio can be used to scan to obtain historical analyte results from the ASICs 3212a-3212b, 1602. The BLE radio can provide a wireless interface to the reader for the user to obtain results. Result data from the ASICs 3212a-322b, 1602 can be transferred to the radio periodically for storage and transfer. As an example and not by way of limitation, the result data can be transferred to the BLE radio every minute.

In particular embodiments, the circuit 3200 can use the two ASICs 3212a-3212b to measure current produced from the sensor 3202. The analyte combinations that are measured can vary depending on the target product. The first working electrode 3204 and the second working electrode 3206 can have transimpedance amplifiers that can measure the maximum current concurrently. The reference electrode 3208 can provide an electrochemical reference potential for the sensor 3202. The ASICs 3212a-3212b can buffer the voltage on the reference electrode 3208 with a high impedance, low leakage amplifier and set up independent bias voltages on each of the working electrodes 3204, 3206. In particular embodiments, the bias voltages can be programmable bias voltages, which can be set to fixed values by the BLE radio. The circuit 3200 can measure skin temperature using the thermistor 3218. The reference electrode 3208 and the counter electrode 3210 compliance voltage can be measured and used as a diagnostic in the ASIC_1 3212a.

In particular embodiments, the measurement process for the first working electrode 3204 and the second working electrode 3206 can use a predetermined sampling window to filter out unwanted low frequency and body effect interference. As an example and not by way of limitation, a 32 second sampling window can be used. In particular embodiments, the circuit 3200 can have a measurement interval. There can be a measurement interval determined by a time constraint. The two working electrodes 3204, 3206 can be sampled concurrently. There can be an offset drift associated with the measurement using the ASICs 3212a-3212b. The ASICs 3212a-3212b can compensate for the offset in real time. The BLE radio can periodically read out the converted values for the first working electrode 3204, second working electrode 3206, temperature, and the counter 3210 potential.

In particular embodiments, the circuit 3200 can have a signal chain that is responsible for two analyte channels (e.g., first working electrode 3204, second working electrode 3206), a temperature channel, and a channel to measure the counter voltage as a diagnostic indicator. The battery voltage monitoring can be accomplished with the measurement channel. In particular embodiments, the circuit 3200 can be calibrated for offset and gain during PCBA manufacturing test. The measurement ranges can be from I1 to I2 (HI) and I1 to I3 (LO). The first working electrode 3204 and the second working electrode 3206 can each have a selectable HI and LO ranges. The resolution can have a bit minimum. As an example and not by way of limitation, the resolution can be 16 bits minimum. The measurement time can have a maximum time period. The measurement time can be the time to complete both analytes, temperature, and counter electrode measurements. As an example and not by way of limitation, the maximum time period can be 50 seconds. In particular embodiments, the first working electrode 3204 and the second working electrode 3206 can have the ability to sample concurrently for a predetermined time period. As an example and not by way of limitation, the first working electrode 3204 and the second working electrode 3206 can have the ability to sample concurrently for 32 seconds. In particular embodiments, the measurement channels can be calibrated in manufacturing at zero input current and an appropriate reference point within a level of accuracy. The range of the measurement channel can be determined by one or more factors including analyte measurement range, sensor sensitivity variation across all sensor lots, and temperature sensitivity. The sensor sensitivity in nA/(mmol/L) can be determined by the lot sensor sensitivity and the temperature.

In particular embodiments, bandwidth limiting can be used for the circuit 3200. The bandwidth limiting can enable high frequency filtering to ensure that extraneous high frequency noise sources are not aliased into the measurement channel. The bandwidth limiting can be implemented by the ASICs 3212a-3212b.

In particular embodiments, the sensor 3202 can use a bias between the reference electrode 3208 and the working electrodes 3204, 3206. The bias can be used to cause a desired reaction to occur to prevent unwanted reactions to occur. Individual measurement channels can be independently programmed for different bias voltages for different analytes. As an example and not by way of limitation, a measurement channel for a glucose measurement channel can have a different bias voltage from a ketone measurement channel. In particular embodiments, the bias can be static and set up during activation and not changed during the use of the circuit 3200. In particular embodiments, each channel of the circuit 3200 can be independently configurable.

In particular embodiments, the ASICs 3212a-3212b can have a timer that will time the operations of the state machine which runs the measurement process. The timer can have a threshold accuracy to ensure the measurement process is completed within a predetermined time period. The timer can restart after a certain time period. As an example and not by way of limitation, the measurement process can be 50 seconds and the timer can restart each minute.

Referring to FIG. 33, the circuit 3300 can have similar properties and function similarly to circuit 3200, with the ASIC 3302 performing the both of the functions of the ASICs 3212a, 3212b. In particular embodiments, when the sensor 3304 is immersed in an analyte solution, current can flow from the working electrodes 3310, 3312 to be measured by the ASIC 3302. In particular embodiments the current can be measured on the high side to distinguish the two currents from the working electrodes 3310, 3312. The circuit 3300 can use several different measurement topologies to interface the ASIC 3302 to the sensor 3304. As an example and not by way of limitation, a potentiostat can be used. The reference electrode 3308 can provide an electrochemical reference potential for the sensor 3304. The ASIC 3302 can buffer the voltage on the reference electrode 3308 with a high impedance, low leakage amplifier and set up a bias voltage on the working electrodes 3310, 3312 relative to the reference electrode 3308. The bias voltage can be set to a fixed value. The value can be set by a DAC programmed through registers, or by a pair of fixed resistors external to the ASIC 3302. In particular embodiments, the circuit 3300 can use a transimpedance amplifier to measure current that flows out of the working electrodes 3310, 3312. The circuit 3300 can use a high impedance buffer amplifier to measure the potential on the reference electrode 3308. The signal of the reference electrode 3308 can be connected to a servo-amplifier to drive the counter electrode 3306 to a voltage which causes the reference electrode 3308 to be at the desired bias voltage relative to the working electrodes 3310, 3312. In particular embodiments, the sensor 3304 bias can be settable by a register or an external resistor.

In particular embodiments, sensor bias of the ASIC 3302 can be monitored by measuring the voltage on the working electrodes 3310, 3312. The measurement of the working electrode voltages can be performed each minute. In particular embodiments, if either working electrode 3310, 3312 exceeds a certain threshold, the measurement for that minute can be rejected. The determination can be done by software running on a BLE radio of the circuit 3300. The ASIC 3302 can measure the work or counter voltage to provide the diagnostic measurement. In particular embodiments, the measurement channels of the circuit 3300 can be individually enabled with configurable sample rates and configurable number of samples. The configuration can be persistent until it is reconfigured through a SPI interface. Measurement configurations can include a manufacturing stability test, where the glucose channel is measured to collect data every 1 second. Additionally or alternatively, as embodied herein, measurement configurations can include manufacturing calibration, where the glucose channel is measured with injected current to determine the slope and intercept of the glucose measurement. In addition, or as a further alternative, measurement configurations can include a product measurement sequence including diagnostics.

In particular embodiments, the circuit 3300 can have an analog signal chain, which is can be for the glucose channel, one temperature channel, and channels to measure the working voltages as a diagnostic indicator.

In particular embodiments, the bandwidth limiting of the circuit 3300 can be implemented as a combination of external analog filtering and internal digital filtering. External ceramic capacitors can be used. The capacitance of the external ceramic capacitors can be less than 10uF. Voltage comparators can be used to perform battery supervision in real time.

In particular embodiments, the measurement channel in the ASIC 3302 can include of a differential PGA followed by a sigma delta converter. The measurement channel can be calibrated in manufacturing at zero input current and at predetermined current within the above level of accuracy. The dynamic range of the measurement channel can be set up to accommodate the needed measurement resolution in mg/DL of glucose. The range of the measurement channel can be determined by one or more factors including a glucose measurement range, sensor sensitivity variation across sensor lots, and temperature sensitivity. The following table shows cases of the factors based on Li. The sensor sensitivity in nA/mg/DL is determined by the lot sensor sensitivity and the temperature.

In particular embodiments, bandwidth limiting can be used in circuit 3300. The bandwidth limiting can be implemented using an external RC filter. In particular embodiments, averaging of the acquired signals can be used to reduce the effect the body can have to impact the signal produced by the sensor. In particular embodiments, a time period used for averaging the signal can be used. As an example and not by way of limitation, a time period of 16 seconds of averaging the signal can be used to reduce the noise. The sensor 3304 can have similar sensor bias as sensor 3202 as described herein.

In particular embodiments, the ASIC 3302 can have a timer that will time the operations of the state machine which runs the measurement process. The timer can have a threshold accuracy to ensure the measurement process is completed within a predetermined time period. The timer can restart after a certain time period. As an example and not by way of limitation, the measurement process can be 50 seconds and the timer can restart each minute.

In particular embodiments, the ASIC 3302 can implement a measurement sequence that is initiated by a BLE radio over the SPI bus. The sequence can capture the glucose channel measurement, temperature measurement, and counter diagnostics measurements.

In particular embodiments, a reference resistor can be integrated into the circuit 3300 to perform a ratiometric measurement, driving a reference current through the thermistor and the reference resistor, or provide a stable enough current source for the thermistor to require a single measurement.

In particular embodiments, the ASIC 3302 can perform one or more internal diagnostics including in-band RF detection, counter compliance, and measurement outlier. For the in-band RF detection, the diagnostics can provide an indication that an NFC was present during a measurement cycle. Data taken when a field is present can be excluded from the onboard glucose calculation. For the counter compliance, the diagnostics can detect when the counter control circuit no longer is able to regulate the REF-CNTR loop. An internal ADC can be used to monitor this voltage. For the measurement outlier, the diagnostics can provide an indication that there is a fault in the sensor or senor connection path. The measurement outlier can be implemented using software to compare four consecutive 8 second measurements (total of 32 seconds) for deviations greater than a predetermined limit.

In particular embodiments, while the circuit 3300 is discussed in context of two working electrodes 3310, 3312, the circuit can utilize a single working electrode or multiple working electrodes. As embodied herein, an outlier filter can be integrated into a digital signals processing module of the ASIC 1830 and can be used to monitor a pending measurement before the results are processed. The outlier filter can compare a current result to results in the recent past (e.g., an immediately previous result) by comparing the pending result to the result to be reported from the last cycle. If the current result is identified as an outlier (e.g., for deviating from the previous result) the result is removed. As an example, the result to be reported from a cycle with an outlier can be replaced with the previous result, written over by a new measurement, or replaced with a default value that indicates that there was an outlier error. The number of outliers recorded for each measurement cycle can be stored and retrieved during measurement processing. Outliers can be reported for each sample, where a measurement includes a combination of multiple samples. The digital signals processing module of the ASIC 130 can also include an averaging filter that calculates the average of all samples from a single measurement sequence.

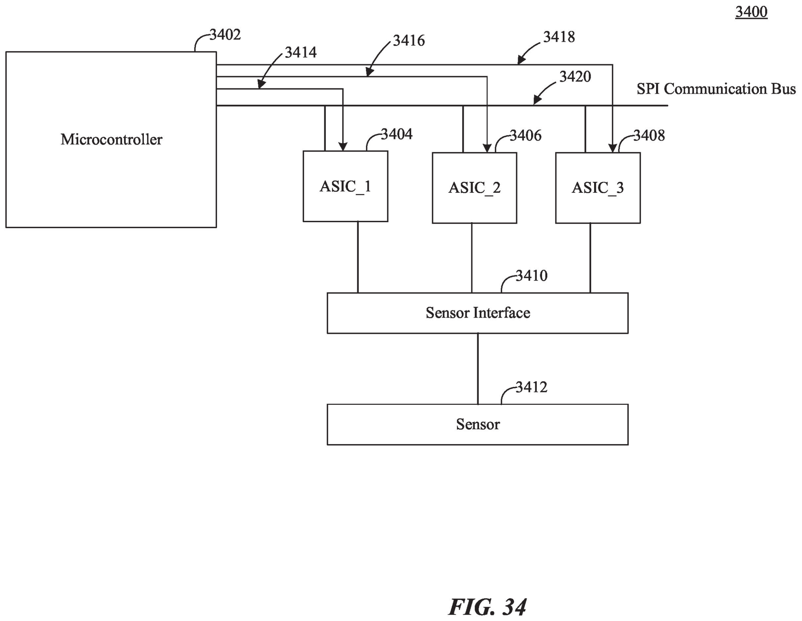

FIG. 34 illustrates an exemplary configuration of a circuit 3400 configured to sample signals from a multiple analyte sensor 3412 using multiple ASICs 3404, 3406, 3408. In particular embodiments, the circuit 3400 may comprise a microcontroller 3402, ASIC_1 3404, ASIC_2 3406, ASIC_3 3408, sensor interface 3410, sensor 3412, SPI select line_1 3414, SPI select line_2 3416, SPI select line_3 3418, and SPI communication bus 3420. In particular embodiments, the microcontroller 3402 may interface the ASICs 3404, 3406, 3408 through the SPI select lines 3414, 3416, 3418, and SPI communication bus 3420. The ASICs may be coupled to the sensor interface 3410. The sensor interface 3410 may be coupled to the sensor 3412 to receive a signal indicative of one or more analyte levels. In particular embodiments, the sensor 3412 may be embodied as a multiple analyte sensor. In particular embodiments, each of the ASICs 3404, 3406, 3408 may be configured to measure a respective analyte level as described herein. The microcontroller 3402 may be configured to control the ASICs 3404, 3406, 3408 to measure a respective analyte level. Although FIG. 34 illustrates a circuit 3400 with a certain number of components in a specific configuration, this disclosure contemplates the circuit 3400 with any number of components in a different configuration. As an example and not by way of limitation, the circuit 3400 may include additional ASICs to measure other analyte signals using the sensor interface 3410 and sensor 3412.

In certain embodiments, the temperature detected by the ASIC 1830 or by a temperature sensor associated with the communication module 1840 can be used to regulate the transmitter power of the communication module. In addition to affecting the ability of signals from the communication module 1840 being detectable by a receiver 1820, the receiver 1820 can interpret transmitter power to determine a distance between the sensor 1810 and the receiver 1820. For example, the receiver 1820 can compare a perceived transmission power of a received signal to a specified or expected transmitter power to determine a level of drop-off or difference and infer the distance between the sensor 1810 and receiver 1820. The receiver 1820 can provide this information to a user to assist the user in locating the sensor 1810 or the patient to whom the sensor 1810 is attached. The receiver 1820 can further warn the user that the sensor 1810 is out of range or nearly out of range.

The communication module 1840 can be configured to disable transmission upon the temperature failing to satisfy a threshold temperature. The communication module 1840 can further be configured to increase or decrease the transmitter power upon the temperature crossing certain interim thresholds. For example, the sensor 1810 can have defined a threshold for full power transmission by the communication module. The sensor 1810 can also have defined a threshold for turning off transmitter functionality. Between the two thresholds, an interim transmitter power can be use. Furthermore, the communication module 1840 can integrate benchmark values to offset potential hysteresis effects where the change in the transmitter power causes the change in temperature, thus mitigating potential race conditions. For example, a hysteresis value of Temp1 degrees Celsius can be used as a benchmark.

In certain embodiments, the background operating current for the multiple analyte sensor can be higher than other analyte sensors because of the addition of two dual op-amps. The addition of the dual op-amps can add as much as a predetermined current at all times. The resulting nominal operating current can be another predetermined current, which would utilize a battery capacity for a target of a predetermined number of days of operation. The nominal clock frequency output can be frequency1.

In addition to the specific embodiments claimed below, the disclosed subject matter is also directed to other embodiments having any other possible combination of the dependent features claimed below and those disclosed above and in the attached figures. As such, the particular features disclosed herein can be combined with each other in other manners within the scope of the disclosed subject matter such that the disclosed subject matter should be recognized as also specifically directed to other embodiments having any other possible combinations. Thus, the foregoing description of specific embodiments of the disclosed subject matter has been presented for purposes of illustration and description. It is not intended to be exhaustive or to limit the disclosed subject matter to those embodiments disclosed.

It will be apparent to those skilled in the art that various modifications and variations can be made in the method and system of the disclosed subject matter without departing from the spirit or scope of the disclosed subject matter. Thus, it is intended that the disclosed subject matter include modifications and variations that are within the scope of the appended claims and their equivalents.

### CLAUSES

1. An analyte monitoring device, comprising:
   a multiple analyte sensor comprising a first working electrode, a second working electrode, a counter electrode, and a reference electrode, wherein each of the first working electrode and the second working electrode is configured to receive a signal indicative of a presence of a respective analyte, and wherein the counter electrode is a sum of the received signal of each of the first working electrode and the second working electrode;
   a transimpedance amplifier configured to receive a first signal of the received signals from the first working electrode and a second signal of the received signals from the second working electrode, wherein the transimpedance amplifier converts the received first signal and the received second signal to an output including a variable bias offset;
   a differential amplifier configured to subtract the variable bias offset from the output to generate a modified output including a variable residual; and
   a processor configured to generate data indicative of an analyte value from the modified output.
2. The device of Clause 1, further comprising:
   an analog to digital converter configured to convert the modified output to a digital output.
3. The device of Clause 2, wherein the subtraction of the variable bias offset from the output reduces the modified output into a range of the analog to digital converter.
4. The device of Clause 2, further comprising:
   a communication module configured to process the digital output into measurement results.
5. The device of Clause 4, wherein the communication module is further configured to provide the measurement results to a receiving device for display via wireless communication.
6. The device of Clause 1, wherein the device is configured to detect blood ketone levels, blood glucose levels, or blood lactate levels.
7. The device of Clause 1, wherein the analyte comprises ketone.
8. The device of Clause 1, wherein the analyte comprises glucose.
9. The device of Clause 1, wherein the analyte comprises lactate.
10. The device of Clause 1, wherein the variable bias offset is determined based on a first analyte measured by the first working electrode or a second analyte measured by the second working electrode.
11. The device of Clause 1, further comprising:
   a negative poise bias amplifier configured to subtract a predetermined voltage from the reference electrode to generate a first working electrode bias to bias the first working electrode.
12. The device of Clause 11, wherein the predetermined voltage is determined based on a first analyte measured by the first working electrode or a second analyte measured by the second working electrode.
13. The device of Clause 11, wherein the negative poise bias amplifier is configured to receive a reference signal from the reference electrode and a negative poise reference.
14. The device of Clause 1, wherein the differential amplifier is further configured to calibrate out a bias dependent residual and a common-mode op-amp characteristic from the output to generate the modified output.
15. The device of Clause 14, wherein the bias dependent residual is determined based on a two-point offset calibration for one of the first working electrode or the second working electrode.
16. The device of Clause 14, wherein the common-mode op-amp characteristic is determined based on a three-point calibration.
17. The device of Clause 1, wherein the first signal of the received signal is a first current signal, and wherein the second signal of the received signals is a second current signal.
18. The device of Clause 17, wherein the transimpedance amplifier is further configured to convert the first current signal and the second current signal into an output voltage.
19. The device of Clause 1, wherein the processor is further configured to generate the data indicative of the analyte value using a calibration function configured to adjust the generated data based on the variable residual.
20. The device of Clause 1, wherein the processor is further configured to generate the data indicative of the analyte value using a calibration function configured to adjust the generated data to remove the variable residual.
21. An analyte monitoring device comprising:
   a multiple analyte sensor comprising a first working electrode, a second working electrode, a counter electrode, and a reference electrode, wherein each of the first working electrode and the second working electrode is configured to receive a signal indicative of a presence of a respective analyte; and
   an application specific integrated circuit configured to receive a first signal of the received signals from the first working electrode and a second signal of the received signals from the second working electrode, wherein the application specific integrated circuit sets a first independent bias voltage for the first working electrode and a second independent bias voltage for the second working electrode, and wherein the application specific integrated circuit is further configured to generate data indicative of an analyte using the first independent bias voltage and the second independent bias voltage.
22. The device of Clause 21, wherein the application specific integrated circuit is configured to sense one or more signals below a threshold current specific to a particular analyte.
23. An analyte monitoring device comprising:
   a multiple analyte sensor comprising a first working electrode, a second working electrode, a counter electrode, and a reference electrode, wherein each of the first working electrode and the second working electrode is configured to receive a signal indicative of a presence of a respective analyte;
   a first application specific integrated circuit configured to receive a first signal of the received signals from the first working electrode, wherein the first application specific integrated circuit sets a first independent bias voltage for the first working electrode, and wherein the first application specific integrated circuit is further configured to generate data indicative of a first analyte using the first independent bias voltage; and
   a second application specific integrated circuit configured to receive a second signal of the received signals from the second working electrode, wherein the second application specific integrated circuit sets a second independent bias voltage for the second working electrode, and wherein the second application specific integrated circuit is further configured to generate data indicative of a second analyte using the second independent bias voltage.
24. The device of Clause 23, further comprising:
   a serial-peripheral interface (SPI) interface configured to individually receive communication from either of the first application specific integrated circuit or the second application specific integrated circuit, wherein the SPI interface comprises one or more communication lines that are shared between the first application specific integrated circuit and the second application specific integrated circuit.

## Claims

1. An analyte monitoring device comprising:
a multiple analyte sensor comprising a first working electrode, a second working electrode, a counter electrode, and a reference electrode, wherein each of the first working electrode and the second working electrode is configured to receive a signal indicative of a presence of a respective analyte sensed in an interstitial fluid of a subject, and wherein the counter electrode is configured to receive a current flow the first working electrode and a current flow from the second working electrode; and
an application specific integrated circuit configured to receive a first signal of the received signals from the first working electrode and a second signal of the received signals from the second working electrode, wherein the application specific integrated circuit sets a first independent bias voltage for the first working electrode and a second independent bias voltage for the second working electrode, and wherein the application specific integrated circuit is further configured to generate data indicative of an analyte using the first independent bias voltage and the second independent bias voltage.

2. The analyte monitoring device of Claim 1, wherein the application specific integrated circuit is configured to sense one or more signals below a threshold current specific to a particular analyte.

3. The analyte monitoring device of Claim 1 or 2, further comprising:
a second application specific integrated circuit, wherein the second application specific integrated circuit is configured to receive the second signal of the received signals from the second working electrode, wherein the second application specific integrated circuit sets the second independent bias voltage for the second working electrode, and wherein the second application specific integrated circuit is further configured to generate data indicative of a second analyte using the second independent bias voltage.

4. The analyte monitoring device of Claim 3, further comprising:
a serial-peripheral interface (SPI) interface configured to individually receive communication from either of the first application specific integrated circuit or the second application specific integrated circuit, wherein the SPI interface comprises one or more communication lines that are shared between the first application specific integrated circuit and the second application specific integrated circuit.

5. The analyte monitoring device of any of Claims 1-4, wherein a first analyte corresponding to the first working electrode is different from a second analyte corresponding to the second working electrode.

6. The analyte monitoring device of Claim 5, wherein the first independent bias voltage is different than the second independent bias voltage.

7. The analyte monitoring device of any of Claims 1-6, further comprising a Bluetooth Low Energy (BLE) radio.

8. The analyte monitoring device of Claim 7, wherein the application specific integrated circuit is configured to independently program the first independent bias voltage and the second independent bias voltage based on input received by the BLE radio.

9. The analyte monitoring device of any of Claims 1-8, wherein the application specific integrated circuit sets the first independent bias voltage and the second independent bias voltage to fixed values.
